# EUROPEAN PATENT APPLICATION

(11) **EP 1 279 734 A1**
(43) Date of publication of application: **29.01.2003**
(21) Application number: 02003554.9
(22) Date of filing: 02.02.1990
(51) Int. Cl.: C12N 15/29, C12N 15/82

(54) **Molecular methods of hybrid seed production**

(30) Priority: 02.02.1989 NZ 22783589; 03.02.1989 US 306438; 03.02.1989 EP 89301053
(62) Divisional of application: 90902754.2
(71) Applicant: PIONEER HI-BRED INTERNATIONAL, INC., Des Moines Iowa 50390 (US)
(72) Inventor: Fabijanski, Steven F., Ottawa, Ontario (CA); Albani, Diego, 07100 Sassari (IT); Robert, Laurian Sylvio, Gatineau, Quebec (CA); Arnison, Paul G., Georgetown, Ontario (CA)
(74) Representative: Olgemöller, Luitgard, Dr.

(57) **Abstract**

The present application is directed to a DNA sequence comprising a promoter sequence selected from sequences of one or more pollen specific genes from Brassica napus and from one or more of the promoter regions of the pollen targeted genes in clones L4 as shown in Fig. 2a or 3a, L10 as shown in Fig. 2b or 3b, L16 as shown in Fig. 2c or 3c or L19 as shown in Fig. 2d or 3d or of portions thereof which function as a pollen specific promoter, or a DNA sequence which hybridizes with the said DNA sequence and has the function of a pollen specific promoter. Specifically, the invention is directed to pollen targeted promoters or such promoter constructs as illustrated in the description and the figures of this application.

## Description

### TECHNICAL FIELD

The present invention relates to a method for producing male sterile plants and hybrid seed, to genetic material used to impart the male sterility trait and to new products produced by said method, namely, genetically transformed plants carrying the male sterile trait, male sterile plants and hybrid seed produced by pollinating said plants with pollen from male fertile plants.

### BACKGROUND ART

Production of hybrid seed for commercial sale is a large industry. Hybrid plants grown from hybrid seed benefit from the heterotic effects of crossing two genetically distinct breeding lines. The agronomic performance of this offspring is superior to both parents, typically in vigour, yield, and uniformity. The better performance of hybrid seed varieties compared to open-pollinated varieties makes the hybrid seed more attractive for farmers to plant and thereby commands a premium price in the market place.

In order to produce hybrid seed uncontaminated with selfed seed pollination control methods must be implemented to ensure cross-pollination and not self-pollination. Pollination control mechanisms can be mechanical, chemical, or genetic.

A simple mechanical method for hybrid seed production can be used if the plant species in question has spatially separate male and female flowers or separate male and female plants. The corn plant, for example, has pollen producing male flowers in an inflorescence at the apex of the plant and female flowers in the axils of leaves along the stem. Outcrossing is assured by mechanically de-tasselling female plants to prevent selfing.

Most major crop plants of interest, however, have both functional male and female organs within the same flower so emasculation is not a simple procedure. It is possible to remove by hand the pollen forming organs before pollen shed, however this form of hybrid seed production is extremely labour intensive and hence expensive. Seed is produced in this manner if the value and amount of seed recovered warrants the effort.

A second general method of producing hybrid seed is to use chemicals that kill or block viable pollen formation. These chemicals, termed gametocides, are used to impart a transitory male-sterility. Commercial production of hybrid seed by use of gametocides is limited by the expense and availability of the chemicals and the reliability and length of action of the applications. These chemicals are not effective for crops with an extended flowering period because new flowers will be produced that will not be affected. Repeated application of chemicals is impractical because of costs.

Many current commercial hybrid seed production systems for field crops rely on a genetic method of pollination control. Plants that are used as females either fail to make pollen, fail to shed pollen or produce pollen that is biochemically unable to effect self-fertilization. Plants that are unable (by several different means) to self pollinate biochemically are termed self-incompatible. Difficulties associated with the use of self-incompatibilities are: availability and propagation of the self-incompatible female line and stability of the self-incompatibility. In some instances self-incompatibility can be overcome chemically or immature buds can be pollinated by hand before the biochemical mechanism that blocks pollen is activated. Self-incompatible systems that can be deactivated are often very vulnerable to stressful climatic conditions that break or reduce the effectiveness of the biochemical block to self-pollination.

Of more widespread interest for commercial seed production are systems of pollen control based on genetic mechanisms causing male sterility. These systems are of two general types: (a) genic male sterility, which is the failure of pollen formation because of one or more nuclear genes or (b) cytoplasmic-genetic male sterility (commonly called cytoplasmic male sterility or CMS) in which pollen formation is blocked or aborted because of a defect in a cytoplasmic organelle (mitochondrion).

Nuclear (genic) sterility can be either dominant or recessive. A dominant sterility can only be used for hybrid seed production if fertility of the hybrid plants is not critical and if propagation of the female line is feasible (eg. by clonal propagation or by the use of a selectible marker closely linked to the sterility gene).

Many successful hybridization schemes involve the use of CMS. In these systems, a specific mutation in the cytoplasmically located mitochondrion can, when in the proper nuclear background, lead to the failure of mature pollen formation. In some other instances, the nuclear background can compensate for the cytoplasmic mutation and normal pollen formation occurs. The nuclear trait that allows pollen formation in plants with CMS mitochondria is called restoration and is the property of specific "restorer genes". Generally the use of CMS for commercial seed production involves the use of three breeding lines, the male-sterile line (female parent), a maintainer line which is isogenic to the male-sterile line but contains fully functional mitochondria and the male parent line.

The male parent line may carry the specific restorer genes (usually designated a restorer line) which then imparts fertility to the hybrid seed. For crops (eg. vegetables) for which seed recovery from the hybrid is unimportant, a CMS system could be used without restoration. For crops for which the fruit or seed of the hybrid is the commercial product then the fertility of the hybrid seed must be restored by specific restorer genes in the male parent or the male-sterile hybrid must be pollinated. Pollination of non-restored hybrids can be achieved by including with hybrids a small percentage of male fertile plants to effect pollination. In most species, the CMS trait is inherited maternally (because all cytoplasmic organelles are inherited from the egg cell only), which can restrict the use of the system.

In a crop of particular interest herein, the oilseed crop of the species Brassica napus or Brassica campestris, commonly referred to as Canola, no commercial hybrid system has been perfected to date. Mechanical emasculation of flowers is not practical for hybrid seed production on any scale. The use of currently available gametocides is impractical because of the indeterminate nature of flower production. Repeated application of chemicals is expensive and the method is prone to contamination with selfed seed.

Genes that result in self-incompatibility are quite widespread in Brassica species and self-incompatible hybrid systems have been used for hybrid seed production in vegetables. Major difficulties are associated with the propagation of the female lines and the breakdown of self-incompatibilities under stressful conditions. Adaptation of these systems to Brassica oilseeds is restricted by the expense of increasing the female lines and the availability of appropriate self-incompatible genes in the dominant Canola species, Brassica napus.

A variety of sources of male sterility are available in Brassica species. Both recessive and dominant genic systems have been reported, however their use is restricted because large scale in vitro propagation or roguing of female lines is in most cases impractical for large scale seed production.

Additionally, a number of CMS systems have been reported in Brassica species. Four of these systems have been explored as possible vehicles for hybrid seed production: pol, nap, anand and ogu. The Polima system (pol) has been widely studied and is probably the closest to commercial use. Good restoration and maintenance of pol CMS has been achieved, however the system suffers from potential instability of the CMS with high temperature, a reduction in the heterotic effect of crossing different lines (because of the defective mitochondria) and a reduction in hybrid seed oil content. The use of other CMS systems is also restricted by heat sensitivity (nap), difficulty in restoration of fertility (ogu, anand), difficulty in the maintenance of the sterility (nap) and low temperature chlorosis associated with the sterile cytoplasm (ogu). Improvement of these systems is the object of considerable research, however all of the systems have some inherent weaknesses that limit their utility.

It is recognized that an ideal system for hybrid seed production in any crop would be a form of genic male sterility that could be regulated or overcome to allow male fertility for the propagation or increase of the female lines or to allow fertility in hybrids. This recognition has stimulated research on the use of molecular systems to effect genic male sterility that could be used for hybrid seed formation. In addition, the advent and widespread application of recombinant DNA methodologies provides a mechanism of introduction of novel DNA sequences into a wide variety of different crop species that is not possible by the limited sexual methods of genetic exchange between different species. The molecular approach has the advantage that the hybridization system can be imposed on all breeding lines or cultivars of any given crop without the need for extensive backcrossing and disruption of established inbred lines leading to the rapid production of male sterile lines with well characterized and superior agronomic performance.

The development of the molecular approach is based on the disruption of any of a number of developmental steps specifically required for the production of functional pollen within the microspore, or for development of any somatic tissues supporting the microspore.

### DISCLOSURE OF INVENTION

It is an object of the invention as claimed herein to provide a method for producing genic male sterility in plants. It is also an object of the invention as claimed herein to provide a method of producing genic male sterility which can be readily adapted to the production of hybrid seed, including hybrid seed that is male fertile.

For certain crops of interest, such as vegetables, it is only the leaves, stems or roots of the plant that are sold commercially. Therefore, even though the male sterility trait may be inherited and expressed in the hybrid plant it is not necessary to overcome or restore male fertility in the seed of the hybrid plant. However, for other crops, the commodity of commerce is the seed or fruit produced by the hybrid plant. Thus for optimal commercial utility of the hybrid it is desirable that the hybrid is self-fertile. For this purpose in outcrossing species, it is sufficient that the hybrid seed comprise a mixture of male sterile and male fertile seeds.

In addition, the use of genic male sterile plants for commercial seed production requires the increase of seed of the genic male-sterile line (frequently called maintenance). The invention contemplates this requirement.

We provide a method of producing a male sterile plant or plant carrying the male sterile trait by integrating into the genome of said plant a pollen targeted recombinant DNA molecule that selectively blocks the production of functional pollen grains or renders developing pollen grains susceptible to a chemical agent or physiological stress. A pollen targeted recombinant molecule as used herein is a recombinant DNA molecule that is selectively expressed in tissues that are critical to pollen formation and function, including pollen itself.

Tissues that are critical to pollen formation or function may also include but are not necessarily limited to tissues which are associated with microspore growth and development or microspore function such as the filament, tapetum and the anther wall.

According to various aspects of the invention discussed below, a pollen targeted recombinant DNA molecule can be used to selectively block the production of functional pollen grains or render developing pollen grains susceptible to a chemical agent or physiological stress in a variety of ways, including:
(i) interfering with a gene that is critical to pollen formation or function using an anti-sense gene, namely a gene that selectively interferes with the growth, development or function of tissues that are critical to pollen formation or function.
(ii) using a pollen targeted promoter to regulate the expression of an anti-sense gene to a constitutive gene to cellular integrity, namely a promoter that selectively regulates gene expression in tissues that are critical to pollen formation and function, including pollen itself.
(iii) using a pollen targeted promoter to regulate the expression of a gene that codes for a molecule that is cytotoxic or a molecule that renders a non-toxic molecule cytotoxic.

We provide methods to produce hybrid seed by pollinating said male sterile plant with a suitable male fertile plant.

We also provide methods to produce hybrid seed by pollinating said plant carrying the male sterile trait at a time when the male sterile trait is expressed by pollinating said plant with a suitable male fertile plant.

We also provide methods to produce fertile hybrid seed by compensating for the gene function that has been compromised as discussed above or by negating the disruption caused to tisues critical to pollen formation or function.

We also elaborate methods of hybrid seed production wherein specific restoration methods are not required.

Specifically, it is an object of the present invention to provide a method of producing hybrid seed by crossing a genetically transformed female parent plant with a suitable male fertile male parent plant, said genetically transformed female parent plant containing a recombinant DNA sequence comprising an sense or anti-sense gene, which when expressed, blocks the production of tissues critical to pollen formation or function or renders said tissues susceptible to a chemical agent or physiological stress that blocks function of said tissues, said suitable male fertile male parent plant, when required, containing a recombinant DNA sequence which compensates for the gene function that has been compromised or negates the disruption caused to tisues critical to pollen formation or function in said genetically transformed female parent plant.

According to one aspect of the present invention genic male sterility may be produced by transforming plant cells that are capable of regeneration into a differentiated whole plant, with a recombinant DNA molecule ("anti-sense gene") that codes for RNA that is complementary to and capable of hybridizing with the RNA encoded by a sense gene, thereby inhibiting the expression of the sense gene.

The sense gene is a gene that is expressed in tissues critical to pollen formation or function or is a gene that is required for the proper development or function of a variety of tissues, including tissues critical to pollen formation or function. If the sense gene is expressed only in tissues critical to pollen formation or function then a promoter that functions in all, many, or a variety of cell types including tissues critical to pollen formation or function can be used for the construction of an anti-sense gene. An example of such a constitutive promoter is CaMV 35S or preferably HP 101 which we have isolated and identified from Brassica napus as described below. In this type of construct, the promoter that is used may be constitutively active in all or many cell types but only interferes with pollen development or function as tissues critical to pollen formation or function are the only cells producing the sense gene RNA in question. As discussed below, it is also possible to use an inducible promoter, as well as a pollen specific promoter.

Alternatively, if the sense gene is a gene required for the development or function of all cells or a variety of cells including tissues critical for pollen formation or function, then the promoter that is used to express the anti-sense gene must be a promoter that is active only in tissues critical to pollen formation or function.

The anti-sense gene is a DNA sequence produced when the sense gene is inverted relative to its normal presentation for transcription and inserted downstream from a promoter. The anti-sense gene may be constructed in a number of different ways, provided that it is capable of being transcribed into RNA which is complementary to and capable of blocking translation of the RNA produced by the sense gene. It is to be understood that to regulate the expression of a sense gene it is sufficient to produce an anti-sense gene that is complementary in whole or in substantial part to the sense gene sought to be regulated.

The anti-sense gene is preferably constructed by inverting the coding region of the sense gene relative to its normal presentation for transcription to allow for transcription of its complement, hence the complementariness of the respective RNA's encoded by these DNA's. In order to block the production of mRNA produced by the sense gene, the timing of anti-sense gene expression must be appropriate in that the anti-sense RNA must be present at the same time as the mRNA from the sense gene in question. The co-incident expression of sense and anti-sense genes can be achieved in a variety of ways using combinations of constitutive, inducible and organ specific promoters, however, it is easily achieved by regulating the expression of the anti-sense gene with the same promoter that controls the sense gene, thereby causing both to be transcribed in the same time frame. The concept of regulating gene expression using anti-sense genes is described in Weintraub, H. et al., Antisense RNA as a molecular tool for genetic analysis, Reviews - Trends in Genetics, Vol. 1(1) 1986.

Accordingly, various types of sense and anti-sense genes and promoters can be used to carry out specific embodiments of this invention. In this regard the gene targeted for inactivation will determine the promoter required to carry out the invention.

Thus, according to one aspect of the invention as claimed herein, we provide a method of producing a male sterile plant from a plant selected from those species of pollen producing plants that are capable of being genetically transformed, which method comprises:
(a) identifying and isolating, preferably from said pollen producing plant, a sense gene that is critical to pollen formation or function in said plant;
(b) inserting into the genome of a plant cell of said plant that is capable of being regenerated into a differentiated whole plant, a gene comprising:
   (i) a DNA sequence that codes for RNA that is complementary to the RNA sequence encoded by said sense gene;
   (ii) a promoter which functions in said plant to cause transcription of said DNA sequence into RNA at or about the time of transcription of said sense gene; and preferably
   (iii) a terminator sequence which defines a termination signal during transcription of said DNA sequence.
(c) obtaining a transformed plant cell of said plant; and
(d) regenerating from said transformed plant cell a genetically transformed plant which is male sterile.

Since the sense gene in question would initially be isolated in its native form, it is to be understood that the term "sense gene" as used herein may refer to one or more parts of the gene including 5' untranslated leader sequences, coding sequences, promoter sequences, intron sequences, and untranscribed 3' sequences, or any substantial fragments of these sequences. It may be desirable to isolate the promoter in the case where the same promoter is used to drive the anti-sense gene.

It is to be further understood herein that it is not essential to identify and isolate the sense gene de novo. Rather the sense gene may have been obtained by any means (eg: described in literature or obtained commercially).

It is possible that the RNA transcribed from the anti-sense gene will be effective in blocking translation of the RNA encoded by the sense gene even though a terminator sequence is not encoded by said recombinant DNA molecule comprising the anti-sense gene.

It is to be understood that it is preferable that the recombinant DNA molecule comprise a gene which confers on plant cells of said plant resistance to a chemical agent or physiological stress, such that said transformed plant cells may be easily selected.

In accordance with the preceding method the invention is also directed to a plant cell which has been transformed with a recombinant DNA molecule as defined in step (b) and which is capable of being regenerated into a male sterile plant.

The invention is also directed to the use of said male sterile plant in a hybrid cross.

In order to produce hybrid seed on a commercial scale a variety of methods for increasing male sterile paints mat be employed, as discussed below. According to one scheme the anti-sense gene may be linked to a gene that confers resistance to a selective agent. According to this scheme, it is possible to produce a male sterile line by crossing the genetically transformed plant (male sterile) with a suitable non-transformed male fertile plant and using said selective agent to select for plants containing the anti-sense gene among plants grown from seed produced from such a cross. Theoretically, any effective selective agent for which a resistance gene has been identified could be used within the scope of this aspect of the invention, including but not limited to genes coding for resistance to herbicides, antibiotics, toxic elements and plant diseases. Such a selective agent could be said to fall within two broad non-mutually exclusive categories, a chemical agent and a physiological stress. A chemical agent, such as a herbicide, could be used to produce male sterile plants on a commercial scale. Examples of herbicides for which a resistance gene has been identified are glyphosate (described in Comai, L., Facciotti, D., Hiatt, W.R., Thompson, G., Rose, R. E., Stalker, D. M., 1985, Nature, Vol. 317, Pages 741-744), chlorsulfuron (described in Haughn, G. W., and Somerville, C. R., 1986, Mol. Gen. Genet., Vol. 210, Pages 430-434) and phosphinotricin (Murakami T, et al, Mol. Gen. Genet. 205: 42-50, 1986).

According to another scheme the number of male sterile plants may be increased by clonal propagation using tissue explants thereof, or other in vitro propagation techniques.

Other schemes may be apparent to those skilled in the art. Therefore it is to be understood that the embodiments of various aspects of the invention defined herein may be carried out using any one of a variety of such methods.

Thus, according to one embodiment of another aspect of the invention as claimed herein, we provide a method of producing hybrid seed from plants selected from those species of pollen producing plants which are capable of being genetically transformed comprising the steps of:
(a) producing a genetically transformed plant which is male sterile by:
   i) identifying and isolating, preferably from said pollen producing plant, a sense gene that is critical to pollen formation or function in said plant;
   ii) inserting into the genome of a plant cell of said plant that is capable of regeneration into a differentiated whole plant, a gene which confers on said plant resistance to a chemical agent or physiological stress and, linked to said gene, a gene comprising:
      (A) a DNA sequence that codes for RNA that is complementary to the RNA sequence encoded by said sense gene;
      (B) a promoter which functions in said plant to cause transcription of said DNA sequence into RNA at about the time of transcription of the RNA encoded by said sense gene; and preferably
      (C) a terminator sequence which defines a termination signal during transcription of said DNA sequence;
   iii) obtaining a transformed plant cell of the said plant; and
   iv) regenerating from said transformed plant cell a plant which is genetically transformed with the genes described in step (a) ii) above; and
(b) increasing the number of genetically transformed plants by:
   i) clonal propagation using tissue explants thereof, or other in vitro propagation techniques; or
   ii)
      A) crossing the genetically transformed plant described in step (a) iv) above with suitable male fertile plant;
      B) using the same chemical agent or physiological stress to eliminate plants which do not contain the genes described in step (a) ii) above among plants grown from seed produced by such cross; and
      C) repeating such a cross over several generations with the plants obtained as in step (b) ii) above in the presence of said chemical agent or physiological stress to increase the numbers of male sterile plants;
(c) effecting a hybrid cross by pollinating said male sterile plants with pollen from suitable male fertile plants.

Hybrid seed produced by the above method may contain a mixture of sterile hybrid seed and fertile hybrid seed because of the segregation of the recombinant DNA causing male sterility. In outcrossing species, the fertile plants that are present will allow full pollination of the male sterile hybrids.

It is to be understood that suitability for the purpose of producing hybrid seed is determined by standard crossing of different lines with subsequent analysis of the progeny and selection of a line with superior combining ability and desired agronomic traits. Suitability of a male fertile plant for the purpose of crossing with a male sterile plant to increase the number of male sterile means use of, but is not necessarily limited to use of, a plant of the same inbred line from which the male sterile plant is derived. In some instances referred to below, maintenance of the male sterile line can be produced simply by selfing in isolation.

In accordance with the preceding method the invention is also directed to a plant cell which has been transformed with genes as defined in step (a) ii) above and which is capable of being regenerated into a male sterile plant, as well as plants and hybrid seed containing the genes defined in (a) ii) above.

In a hybrid seed production scheme where there are alternating rows of male sterile plants and male fertile plants, it may be simpler but not essential to carry out the final selection of male steriles in the field alongside the male fertile donors. Therefore it is desirable if the suitable male fertile donors are previously transformed to resistance to the selective agent to avoid having to selectively apply said agent to the rows of male sterile plants. Therefore step (c) would be accomplished by growing seed produced from a cross between the selected genetically transformed plants and suitable male fertile donors, alongside seed of suitable male fertile donors which have previously been made resistant to said chemical agent or physiological stress (the selective agent).

Where the sense gene that is identified and isolated has sequences that are transcribed but not translated, it is possible to produce hybrid seed with restored fertility by inactivating the sense gene using an anti-sense gene to said sequences to produce male sterile plants and restoring the function of the sense gene in a restorer line. The restorer line would comprise plants that have been genetically transformed with a modified form of the sense gene that does not contain the regions complementary to the anti-sense gene and thus is not subject to the anti-sense regulation.

Said transcribed but untranslated sequence may include an untranslated 5' leader sequence, intervening sequences and an untranslated 3' sequence, or any substantial fragments of these sequences. It is to be understood that said sequences or fragments thereof may be naturally occurring or may be artificially introduced.

Thus according to one aspect of the invention we provide a method of producing a male sterile plant from plants selected from those species of pollen producing plants which are capable of being genetically transformed comprising the steps of:
(a) producing a male sterile plant by:
   (i) identifying and isolating, preferably from said pollen producing plant, a sense gene which comprises a coding sequence that is critical to pollen formation or function and a transcribed but untranslated sequence;
   (ii) inserting into the genome of a plant cell of said plant that is capable of regeneration into a differentiated whole plant, a gene which confers on said plant resistance to a chemical agent or a naturally occurring or artificially induced physiological stress and linked to this a recombinant DNA molecule comprising:
      (A) a DNA sequence that is complementary to said transcribed but untranslated sequence;
      (B) a promoter which functions in said plant to cause transcription of said DNA sequence into RNA at or about the time of the transcription of the sense gene in developing pollen; and preferably
      (C) a terminator sequence which defines a termination signal during transcription of said DNA sequence;
   (iii) obtaining a transformed plant cell of said plant; and
   (iv) regenerating from said plant cell a plant which is genetically transformed with said DNA sequences described in (a)(ii) above and is male sterile.

Thus according to one aspect of the invention we provide a method of producing hybrid seed with restored male fertility from plants selected from those species of pollen producing plants which are capable of being genetically transformed comprising the steps of:
(a) producing a male sterile plant by:
   (i) identifying and isolating, preferably from said pollen producing plant, a sense gene which comprises a coding sequence that is critical to pollen formation or function and a transcribed but untranslated sequence;
   (ii) inserting into the genome of a plant cell of said plant that is capable of regeneration into a differentiated whole plant, a gene which confers on said plant resistance to a chemical agent or a naturally occurring or artificially induced physiological stress and linked to this a recombinant DNA molecule comprising:
      (A) a DNA sequence that is complementary to said transcribed but untranslated sequence;
      (B) a promoter which functions in said plant to cause transcription of said DNA sequence into RNA at or about the time of the transcription of the sense gene in developing pollen; and preferably
      (C) a terminator sequence which defines a termination signal during transcription of said DNA sequence;
   (iii) obtaining a transformed plant cell of said plant; and
   (iv) regenerating from said plant cell a plant which is genetically transformed with said DNA sequences described in (a)(ii) above and is male sterile, and
(b) increasing the number of genetically transformed male sterile plants by:
   (i) clonal propagation of said genetically transformed male sterile plant described in step (a) using tissue explants thereof, or other in vitro propagation techniques; or
   (ii) crossing said genetically transformed male sterile plant with a suitable male fertile plant;
   (iii) using said chemical agent or physiological stress to eliminate plants which do not contain the DNA sequence defined in (a)(ii) amongst plants grown from seed produced by such cross; and
   (iv) repeating such cross over several generations with plants obtained in step (b)(iii) above in the presence of said chemical agent or physiological stress to increase the numbers of male sterile plants.
(c) producing a male fertile restorer plant by:
   (i) inserting into the genome of a plant cell of a suitable male parent plant that is capable of regeneration into a differentiated whole plant a gene that confers resistance to a chemical agent or a naturally occurring or artificially induced physiological stress, linked to a recombinant DNA sequence comprising:
      (A) a recombinant DNA molecule that comprises a modified form of said sense gene that does not contain the regions complementary to said anti-sense gene;
      (B) a promoter that functions in said plant to cause transcription of said modified DNA sequence at a time which restores the function of the sense gene, preferably at or about the time of the action of the antisense gene; and preferably
      (C) a terminator sequence which defines a termination signal during transcription of said DNA sequence.
(d) increasing the number of genetically transformed plants by selecting a plant homozygous for said restorer trait and selfing in isolation.
(e) effecting a hybrid cross by pollinating said male sterile plants with pollen from said male fertile restorer plants.

Selection for a plant that is homozygous for said restorer trait may be carried out by conducting anther or isolated microspore culture of the genetically transformed plant carrying said restorer trait, or preferably by selfing said plant in isolation prior to selection.

It is particularly desirable to restore fertility as discussed above where the target plant species does not outcross. Where the target plant species does outcross it is preferable but not necessary to produce a restorer line as described in steps (c) and (d) above. Crossing with a suitable male fertile plant will yield hybrid seed comprising a mixture of sterile hybrid seed and fertile hybrid seed because of the segregation of the recombinant DNA causing male sterility. The fertile plants that are present will allow full pollination of the male sterile hybrids.

The expression of the restorer gene may be driven by any promoter that allows transcription during the time that pollen development would otherwise be disrupted. It is preferred that the restorer gene be driven by the same promoter used to drive the anti-sense gene or any promoter that is highly active in tissues that are critical to pollen formation or function.

In accordance with the preceding method the invention is also directed to:
(i) a plant cell which has been transformed with a recombinant DNA molecule described in steps (a) (ii) or (c) (i) above and which is capable of being regenerated into a plant which carries the male sterile trait;
(ii) a plant which has been transformed with a recombinant DNA molecule described in steps (a) (ii) or (c) (i) above; and
(iii) hybrid seed which has been transformed with a recombinant DNA molecule described in steps (a) (ii) and (c) (i) above.

According to one embodiment of another aspect of the invention as claimed herein, we regulate the expression of the coding region of the anti-sense gene by using an inducible promoter. In this scheme, the promoter can be left in an induced state throughout pollen formation or at least for a period which spans the period of transcription of the sense gene. A promoter that is inducible by a simple chemical is particularly useful since the male sterile plant can easily be maintained by self-pollination when grown in the absence of said chemical. Restoration is inherent in growing plants produced from hybrid seed in the absence of said inducer. Thus according to one embodiment of another aspect of the invention as claimed herein we provide a method of producing hybrid seed with restored fertility from plants selected from those species of pollen producing plants which are capable of being genetically transformed comprising the steps of:
(a) producing a genetically transformed plant which carries the male sterile trait by:
   i) identifying and isolating, preferably from said pollen producing plant, a sense gene that is critical to pollen formation or function in said plant;
   ii) inserting into the genome of a plant cell of said plant that is capable of regeneration into a differentiated whole plant, a recombinant DNA molecule comprising:
      (A) an anti-sense gene that codes for RNA that is complementary in whole or in part to the RNA sequence encoded by said sense gene;
      (B) an inducible promoter which can function in said plant cell to cause transcription of said DNA sequence into RNA during the time of transcription of said sense gene or coding sequence; and preferably
      (C) a terminator sequence which defines a termination signal during transcription of said DNA sequence
   iii) obtaining a transformed plant cell of said plant; and
   iv) regenerating from said transformed plant cell a plant which is genetically transformed with said DNA molecule described in (ii) above and can be rendered male sterile by said inducer;
(b) increasing the number of genetically transformed plants by:
   (i) clonal propagation of said genetically transformed plant described in step (a) using tissue explants thereof, or other in vitro propagation techniques; or
   (ii) selfing the genetically transformed plant carrying the inducible male sterile trait described in (a), selecting a plant homozygous for the inducible male sterile trait and increasing said plant by selfing in isolation over a number of generations in the absence of the inducer to increase the number of genetically transformed plants;
(c) effecting a hybrid cross between said genetically transformed plants grown in the presence of said inducer and suitable male fertile plants.

In accordance with step (a) of the preceding method we provide a method to produce a plant which carries the male sterile trait.

In accordance with the preceding method the invention is also directed to a plant cell which has been transformed with a recombinant DNA molecule which is defined in step (a) (ii) above and which is capable of being regenerated into a plant which carries the male sterile trait.

In accordance with the preceding method, the invention is also directed to a plant which contains a recombinant DNA molecule comprising:
(a) a DNA sequence that codes for RNA that is complimentary to the RNA sequence encoded by a sense gene which is critical to pollen formation or function in said plant;
(b) an inducible promoter which functions in said plant to cause transcription of said DNA sequence into RNA during the time of transcription of said gene; and
(c) a terminator sequence which defines a termination signal during transcription of said DNA sequence.
In accordance with the preceding methods the invention is also directed to hybrid seed containing a recombinant DNA molecule which comprises:
(a) a DNA sequence that codes for RNA that is complimentary to the RNA sequence encoded by a sense gene which is critical to pollen formation or function in a plant grown from said seed;
(b) an inducible promoter which functions in said plant to cause transcription of said DNA sequence into RNA during the time of transcription of said sense gene; and
(c) a terminator sequence which defines a termination signal during transcription of said DNA sequence.

According to one embodiment of another aspect of the invention as claimed herein, we provide DNA coding sequences isolated from a plant of the species Brassica napus ssp oleifera w Westar which are expressed only in microspores and whose expression is critical to microspore development. A schematic representation of the restriction maps and coding regions of microspore specific genes identified as L4, L10, L16 and L19 are presented herein. The complete nucleotide sequence of clones L4, and relevant sequences of L10, L16 and L19 are also presented herein. The nucleotide sequences of isolated cDNA clones that correspond to the genes or related gene family members within clones L4, L10 and L19 are also presented herein.

It is believed that identical or homologous DNA sequences to those described herein will be found and expressed exclusively in pollen of other species of pollen-bearing plants, particularly species of plants within the genus Brassica and the family Cruciferae (also known as Brassicaceae), and more particularly other cultivars of Brassica napus.

The occurrence of this sequence in other species of pollen-bearing plants may be routinely ascertained by known hybridization techniques. It is believed that the similarity of plant genes from species to species will allow for the preceding aspects of the present invention to be carried out using said DNA fragment in any number of pollen bearing plant species that are capable of being genetically transformed. The universality of plant genes has been widely documented in the literature and homologous plant genes have been described for plant actins (Shah, D.M., et al, J. Mol. Appl. Genet. 2:111-126, 1983), phytochrome (Hershey, H.P., et al., Proc. Natl. Acad. Sci. USA 81:2332-2337, 1984) storage proteins (Singh, N.K., et al., Plant Mol. Biol. 11:633-639, 1988) enzymes such as glutamine synthase (Lightfoot, D.A., et al, Plant Mol. Biol. 11:191-202, 1988, and references within) and nitrate reductase (Cheng, C., et al, EMBO Jour. 7:3309-3314). These and other examples in the literature clearly demonstrate that many plant genes are highly conserved. It is also clear that this conservation applies not only to structural proteins but to enzymatic proteins important to cellular physiology. Therefore, it is believed that said DNA fragment, when found in another plant species, will be critical to microspore development and will be able to be employed to carry out the present invention in such species.

It has also been demonstrated that anti-sense RNA sequences derived from one plant species can effectively inhibit the expression of homologous DNA sequences in a different species (Van der Krol, et al, 1988, Nature 333: 866-869). Therefore, it is expected that anti-sense RNA derived all or in part from Brassica pollen specific DNA sequences will be functional in other plants.

Furthermore, it is to be understood that any number of different genes that are crucial to microspore development or tissues uniquely involved in and critical to microspore development could be isolated and used in accordance with the preceding methods.

According to one embodiment of another aspect of the invention, we provide pollen specific promoter sequences and transformation vectors containing these sequences as presented herein. These sequences can be used to limit the expression of any given DNA sequence to pollen tissue and to a period during pollen formation. The pollen specific promoter can be used to limit the expression of DNA sequences adjacent to it, to pollen tissue in both the Solanacae and Cruciferae families as provided for in the specific examples and it is fully believed that these DNA fragments or functional fragments thereof will function as pollen specific promoters in numerous other if not all pollen bearing plant species that are capable of being genetically transformed.

These pollen specific promoters can be used in conjunction with naturally occurring flanking coding or transcribed sequences described herein or with any other coding sequence that is critical to pollen formation or function to carry out preceding aspects and embodiments of this invention.

It is noted that there is not a high degree of DNA sequence homology between the native pollen specific promoters from the L4, L10 and L19 clones. Test data reveal that the timing and level of expression of these genes is not identical in pollen, but that all overlap in activity at some time. This illustrates that there are divergent gene sequences in Brassica napus that still function as pollen specific promoters.

It is expected that one may use any number of different pollen specific promoters to carry out preceding aspects of this invention and further aspects of the invention to be discussed below. It is often difficult to determine a priori what pollen specific promoter could be used to inhibit a gene that is critical to pollen development or cellular function and development, but certain conditions must be met.

The pollen specific promoter used should be a promoter, or a modification of the promoter that is active at the appropriate time to produce sufficient levels of transcribed RNA to carry out the invention. Pollen specific promoters derived from pollen specific clones are disclosed herein that are active early in the development of microspores such that gene expression takes place both during and after the meiotic and mitotic division of pollen mother cells. Thus the activity of these promoters are not limited by segregation.

It should be noted that the identification of a promoter region is usually defined by function rather than a set DNA sequence. As little as 200 bases or less may be sufficient DNA sequence to maintain promoter function. It should also be recognized that some upstream DNA sequences can be arranged in opposite orientations and still retain or demonstrate enhanced promoter function. In addition, "enhancer-like" DNA sequences, which are usually small conserved DNA sequences ranging in size from less than 10 nucleotides to considerably larger numbers of nucleotides may also be inserted into promoter regions to enhance expression.

It may also be desirable to include some intron sequences in the promoter constructs as it has been shown that the inclusion of intron sequences in the coding region may result in enhanced expression and specificity. Thus it may be advantageous to join the DNA fragments to be expressed to a promoter fragment that contains the first intron and exon sequences of a pollen specific gene.

Additionally regions of one promoter may be joined to regions from a different promoter in order to obtain the desired promoter activity. Specific examples of chimeric promoter constructs are disclosed that can be used to carry out aspects of this invention.

The pollen specific promoter should also function as to produce sufficient levels of anti-sense RNA such that the levels of the sense gene product are reduced. Investigations of the mechanism of anti-sense RNA inhibition of gene expression in model systems have suggested that equal or greater than equal levels of anti-sense RNA may be required in order to observe a significant reduction of sense gene activity. However, in some cases it is noted that low levels of anti-sense RNA can have a specific reduction in sense gene activity. Therefore, in some instances if the gene that is targeted for inactivation by anti-sense RNA is a so-called housekeeping gene, or a gene that is expressed in all cell types at a low level, an excess of anti-sense RNA may not be required for inhibition. Additionally less than total reduction of the gene activity may be more than sufficient to disrupt pollen development which is known to be very sensitive to many stressful conditions. Therefore it is suggested that the pollen specific promoter that is used to carry out certain aspects of this invention be chosen based on the observation that the pollen specific promoter functions to cause the expression of any sequences adjacent to it to be transcribed at a time that parallels or overlaps the period of time that the sense gene sought to be inactivated is expressed and that the levels of anti-sense RNA expressed from the anti-sense gene be of levels sufficient to inhibit the sense gene expression, usually to mean greater than or equal to the levels of sense RNA.

It is also understood that the successful transformation and recovery of a plant that contains these recombinant sequences may not always result in appropriate pollen specific expression. The transformation procedure results in the random insertion of foreign DNA such that so-called position effects may override and suppress the activity of any introduced DNA. It is thus advisable to generate a number of individual transformed plants with any recombinant construct in order to recover individuals free from any limiting position effects. It may also be preferable to select plants that contain more than one copy of the introduced anti-sense gene construct such that high levels of expression are obtained.

The determination of the most likely developmental stage in which the sense gene is targeted for inactivation can be accomplished by choosing a time in the developmental pattern of pollen formation at which the sense gene is maximally expressed and using a pollen specific promoter that displays a similar developmental pattern.

We now discuss further aspects of this invention calling for a pollen specific promoter.

By using a pollen specific promoter to regulate the expression of the anti-sense gene, it is possible to interfere with normal microspore development in any given plant, without having to first isolate from the genomic DNA of said plant, a gene which codes for a developmentally regulated protein that is critical to microspore development. We will describe a method to produce a male sterile plant where the sense gene targetted for inactivation is a gene that is critical to cellular function or development and is expressed in metabolically competent cell types. To produce a male sterile plant, such a gene is specifically inactivated in pollen by using a pollen specific promoter to limit the transcription of its anti-sense gene complement to pollen tissue.

We will also describe a method to produce a male-sterile plant, wherein the sense gene targeted for inactivation is a native or foreign gene which confers on the plant resistance to a chemical agent or a naturally occurring or artificially induced physiological stress. If this gene is a foreign gene, it can be inserted into the genome of the plant, prior to, after or concurrently with the anti-sense gene. A male sterile plant can be produced by growing a plant in the presence of such a stress during pollen formation and using an anti-sense gene comprising said pollen specific promoter to specifically inactivate, in pollen, during pollen formation, a gene conferring on the remainder of the plant resistance to said stress. Any stress which can adequately be controlled on a large scale and for which a resistance gene has been identified may theoretically be employed in this scheme, including possibly but not limited to herbicides, pathogenic organisms, certain antibiotics and toxic drugs. In this scheme, a male sterile plant line can be maintained by self-pollination when grown in the absence of the biochemical or physiological stress. Restoration is inherent in growing plants produced from hybrid seed in the absence of said stress.

Thus according to one embodiment of another aspect of our invention, we provide a method of producing a male sterile plant from a plant selected from those species of pollen bearing plants that are capable of being genetically transformed which method comprises the steps of:
(a) identifying and isolating, preferably from said pollen producing plant, a sense gene or coding sequence that is critical to cellular function or development and expressed in metabolically competent cell types;
(b) inserting into the genome of a plant cell a recombinant DNA molecule comprising:
   i) a DNA sequence that codes for RNA that is complementary to the RNA encoded by said sense gene or coding sequence;
   ii) a pollen specific promoter which functions in said plant cell to cause transcription of said DNA sequence into RNA in a time frame which enables said RNA to block the function of the RNA encoded by said sense gene or coding sequence; and preferably
   iii) a terminator sequence which defines a termination signal during transcription of said DNA sequence.
(c) obtaining a plant cell that has been genetically transformed with said DNA sequence;
(d) regenerating from said transformed plant cell a genetically transformed plant which is male sterile.

In accordance with the preceding method the invention is also directed to:
(i) a plant cell which has been transformed with the recombinant DNA molecule as defined in step (b) above and which is capable of being regenerated into a male sterile plant; and
(ii) a plant which has been transformed with the recombinant DNA molecule as defined in step (b) above and which is male sterile.

Similarly, in accordance with another aspect of this invention we provide a method of producing hybrid seed from plants selected from those species of pollen producing plants which are capable of being genetically transformed comprising the steps of:
(a) producing a male sterile plant by:
   i) identifying and isolating, preferably from said pollen producing plant, a sense gene that is critical to cellular function or development and expressed in metabolically competent cell types.
   ii) inserting into the genome of a plant cell of said plant that is capable of regeneration into a differentiated whole plant, a gene which confers on said plant resistance to a chemical agent or physiological stress and linked to said gene, a recombinant DNA sequence comprising:
      (A) a DNA sequence that codes for RNA that is complementary to the RNA sequence encoded by said sense gene;
      (B) a pollen targeted promoter which functions in said plant to cause transcription of said DNA sequence into RNA at or about the time of transcription of the sense gene in tissues critical to pollen function or formation; and preferably
      (C) a terminator sequence which defines a termination signal during transcription of said DNA sequence
   iii) obtaining a transformed plant cell of said plant; and
   iv) regenerating from said transformed plant cell a plant which is genetically transformed with the genes described in step (a)(ii) above and which is male sterile; and
(b) increasing the number of genetically transformed plants by:
   i) clonal propagation using tissue explants thereof, or other in vitro propagation techniques; or
   ii)
      A) crossing the genetically transformed plant described in step (a) iv) above with suitable male fertile plant;
      B) using the same chemical agent or physiological stress to eliminate plants which do not contain the genes described in step (a) ii) above among plants grown from seed produced by such cross; and
      C) repeating such a cross over several generations with the plants obtained as in step (b) ii) above in the presence of said chemical agent or physiological stress to increase the numbers of male sterile plants;
(c) effecting a hybrid cross between said genetically transformed plants and suitable male fertile plants. As discussed above, hybrid seed produced by the above method may contain a mixture of sterile hybrid seed and fertile hybrid seed because of the segregation of the recombinant DNA causing male sterility. In outcrossing species, the fertile plants that are present will allow full pollination of the male sterile hybrids.

Again it is to be understood that where there are alternating rows of male sterile plants and male fertile plants, it may be simpler but not essential to carry out the final selection of male steriles in the field alongside the male fertile donors. Therefore it is desirable if the suitable male fertile donors are previously transformed to be resistant to the selective agent to avoid having to selectively apply said agent to the rows of male sterile plants.

In accordance with the preceding method the invention is also directed to a plant cell which has been transformed with a gene and a recombinant DNA sequence as defined in step (a)(ii) above and which is capable of being regenerated into a male sterile plant.

In accordance with the preceding aspect of this invention, the invention is also directed to a plant and hybrid seed containing DNA comprising a recombinant DNA molecule which comprises:
(a) a DNA sequence that codes for RNA that is complementary to mRNA encoded by a gene that is essential to cellular function or development in metabolically competent cell types;
(b) a pollen targeted promoter which functions in said plant or plant grown from said hybrid seed to cause transcription of said DNA sequence into RNA in a time frame which enables said RNA to block the function of the RNA encoded by said gene; and preferably
(c) a terminator sequence which defines a termination signal during transcription of said DNA sequence.

It is to be understood that a sense gene that codes for a protein that is critical to cellular function or development may be identified in the literature and isolated in a simplified fashion according to the methods described below.

Examples of such proteins are proteins such as actin, tublin or ubiquitin, three proteins which are essential to cellular growth and development.

Sequences for actin genes isolated from plants have been published (for example; Baird W.V., and Meagher, R.B., EMBO J. 6:3223-3231, 1987, or Shah, D.M., Hightower, R.C. and Meagher, R.B., Proc Natl Acad Sci USA 79: 1022-1026, 1982) and actin is known to play a critical role in normal cellular function especially during mitosis and meiosis where actin forms part of the cellular apparatus for cellular division.

The sequence for plant tubulin has also been described (Raha, D., Sen, K. and Biswas, B.B. Plant Mol Biol 9:565-571, 1987). Tubulin, like actin, is known to be important in the cellular life cycle particularly in regards to cell shape, transport and spindle formation during mitosis and meiosis.

The DNA sequence for plant ubiquitin has also been published (Gausing, K. and Barkardottir, R. Eur J. Biochem 158:57-62, 1986). Ubiquitin is a protein involved in the turnover of cellular proteins and as such has a critical role in the regulation of specific cellular protein levels. In addition, ubiquitin is one of the most highly conserved proteins in eukaryotic cells. Interference with ubiquitin expression can cause abnormalities in the turnover of cellular proteins.

If any of the aforementioned proteins are not present in the cell, proper cellular function is interfered with and the cell fails to develop properly.

It is believed that a gene that is found to be essential to cellular growth or development in one plant species will have a similar counterpart in other plant species, since it is generally understood that within the plant kingdom there are genes that are nearly identical or very homologous involved in the basic processes that control or are a result of cellular development. It is further believed that a gene that interferes with the expression of said gene (ie. an anti-sense gene) in one plant species will have the ability to do so in other plant species.

The similarity and universality of these genes have been exemplified in the literature. The tissue-specific and developmentally regulated expression of a wheat endosperm protein synthesized in tobacco plants genetically transformed with this wheat gene has been reported (Flavell, R.B., et al, Second International Congress for Plant Molecular Biology, Abstract #97). In that example, the wheat gene functioned in the tobacco plant in an identical fashion to the way in which it functions in a wheat plant. Other literature clearly shows that the regulation of a specific gene, which can be in many cases complex, is maintained in transgenic plants. One example of this is the phytochrome mediated regulation of a wheat Chlorophyll a/b-binding protein in transgenic tobacco (Nagy, F. et al, EMBO Jour. 5:1119-1124, 1986). In this example the light responsive specific regulation of the wheat gene was maintained in the foreign genetic environment. Not only do cereal genes function in a conserved manner, but genes from other plant species that are more closely related maintain functionality in heterologous genetic systems. Pea seed proteins are expressed properly in tobacco plants (Higgins, T.J.V., et al Plant Mol. Biol. 11:683-696, 1988), as are soybean seed proteins, (Barker, S.J., et al, Proc. Natl. Acad. Sci.USA 85:458-462, 1988) and pea rbcS genes (Nagy, F. et al., EMBO Jour. 4:3063-3068, 1985). The scientific literature has numerous other examples of genes that have been used to genetically transform plants and those genes maintain their ability to function properly in this new genetic environment. Therefore the conserved nature of these genes, not only in the DNA sequences which control the expression of these genes, but the actual protein structure coded for by these genes, is similar among the plant species.

It follows that one should be able to specifically inhibit the production of these proteins by using an anti-sense gene which is specific to the mRNAs encoded by the published sequences and a pollen specific promoter according to the methods described above.

According to one embodiment of another aspect of the invention as claimed herein, we provide a method of producing a plant which carries a male sterile trait from a plant selected from those species of pollen producing plants which are capable of being genetically transformed, which method comprises the steps of:
(a) transforming a plant cell of said plant which is capable of being regenerated into a differentiated whole plant with a sense gene which confers on said plant resistance to a chemical agent or a naturally occurring or artificially induced physiological or chemical stress;
(b) regenerating from said transformed plant cell a genetically transformed plant which is resistant to the same stress;
(c) inserting into the genome of a plant cell of the stress resistant plant which is capable of being regenerated into a differentiated whole plant a recombinant DNA molecule comprising:
   i) a DNA sequence that codes for RNA that is complementary to the RNA sequence encoded by the said sense gene;
   ii) a pollen specific promoter which functions in said plant cell to cause transcription of said DNA sequence into RNA; and preferably
   iii) a terminator sequence which defines a termination signal during transcription of said DNA sequence.
(d) obtaining a plant cell of said stress resistant plant which has been transformed with the gene described in step (c) above; and
(e) regenerating from said transformed plant cell a plant which has been genetically transformed with the genes described in step (a) and step (c) above and can be rendered male sterile by said chemical agent or stress.

In accordance with the preceding method the invention is also directed to:
(i) a plant cell containing the gene defined in steps (a) and (c) above and which is capable of being regenerated into a plant which carries the male sterile trait.
(ii) a plant containing the gene defined in steps (a) and (c) above and which carries the male sterile trait.

It is to be understood that the plant sought to be rendered male sterile need not be transformed with a sense gene that confers resistance to a chemical agent or a naturally occurring or artificially induced physiological or chemical stress if this sense gene is native to the plant.

It must be understood that the preceding aspect of our invention may also be accomplished by transforming a plant cell with both of the genes referred to in (a) and (c) above simultaneously, and therefore without an intermediate regeneration step (b).

According to one embodiment of another aspect of the invention as claimed herein, we provide a method of producing hybrid seed with restored fertility from plants selected from those species of pollen producing plants which are capable of being genetically transformed comprising the steps of:
(a) producing a plant which carries a male sterile trait by:
   i) inserting concomitantly or independently into the genome of a plant cell of said pollen producing plant which is capable of being regenerated into a differentiated whole plant, a sense gene which confers on said plant resistance to a chemical agent or a naturally occurring or artificially induced physiological stress and a recombinant DNA molecule comprising:
      (A) a DNA sequence that codes for RNA that is complementary to the RNA sequence encoded by said sense gene;
      (B) a pollen targeted promoter which functions in said plant cell to cause transcription of said DNA sequence into RNA; and preferably
      (C) a terminator sequence whch defines a termination signal during transcription of said DNA sequence;
   ii) obtaining a plant cell of a plant which has been transformed with the genes described in step (i) above;
   iii) regenerating from said transformed plant cell a plant which is genetically transformed with the genes described in step (i) above and can be rendered male sterile by said chemical agent or stress;
(b) increasing the number of genetically transformed plants by:
   i) clonal propagation using tissue explants thereof, or other in vitro propagation techniques;
   ii)
      A) crossing the genetically transformed plant described in step (a) iv) above with suitable male fertile plant;
      B) using the same chemical agent or physiological stress to eliminate plants which do not contain the genes described in step (a) ii) above among plants grown from seed produced by such cross; and
      C) repeating such a cross over several generations with the plants obtained as in step (b) ii) above in the presence of said chemical agent or physiological stress to increase the numbers of male sterile plants; or preferably
   iii)
      A) selfing the genetically transformed plant described in (a) that can be rendered male sterile by said chemical agent or stress, and selecting from that selfing progeny, a plant homozygous for the male sterile trait;
      B) growing the genetically transformed plant described in step (a)(iii) above in isolation from the same stress or chemical agent to produce a self-fertile plant;
      C) permitting self-fertilization; and
      D) growing seed of such self-fertile plant, over a number of generations in isolation from the same stress or chemical agent to increase the number of genetically transformed plants;
(c) effecting a hybrid cross between said genetically transformed plants grown in the presence of the same stress or chemical agent present during pollen formation and suitable male fertile plants.

Again it is to be understood that the plant sought to be rendered male sterile need not be transformed with a sense gene that confers resistance to a chemical agent or a naturally occurring or artificially induced physiological or chemical stress if this sense gene is native to the plant.

In accordance with the preceding method the invention is also directed to hybrid seed containing DNA comprising:
(a) a sense gene which confers on a plant grown from said seed resistance to a chemical agent or a naturally occurring or artificially induced physiological stress; and
(b) a recombinant DNA molecule comprising:
   i) a DNA sequence that codes for RNA that is complementary to the RNA sequence encoded by said sense gene;
   ii) a pollen specific promoter which functions in said plant to cause transcription of said DNA sequence into RNA; and
   iii) a terminator sequence which defines a termination signal during transcription of said DNA sequence.

According to one embodiment of another aspect of the invention as provided in the claims, we describe a method of producing a male sterile plant by transforming a plant with a recombinant DNA molecule comprising a pollen specific promoter and a DNA sequence coding for a cytotoxic molecule. In theory, any toxic molecule which is known to be encoded by one or more identifiable DNA sequences may be employed within the scope of this aspect of the invention, including but not limited to ricin and diphtheria toxin. We provide a method to produce hybrid seed with restored male fertility by crossing said male sterile plant with a suitable male fertile plant that has been transformed with a recombinant DNA molecule comprising a DNA sequence which is in the anti-sense orientation to that of the DNA sequence coding for the cytotoxic molecule and a promoter controlling said anti-sense sequence which causes transcription thereof at about the time of transcription of said DNA sequence coding for said cytotoxic molecule, thereby inhibiting the expression in the hybrid plant of said DNA sequence coding for the cytotoxic molecule. The promoter controlling the anti-sense sequence is preferably the same pollen specific promoter that controls production of the cytotoxic molecule. Inducible and constitutive promoters may also be advantageously used to control the timing of expression of said anti-sense sequence.

Thus according to one embodiment of another aspect of the invention as claimed herein we provide a method of producing a male sterile plant from a plant selected from those species of pollen producing plants which are capable of being genetically transformed, which method comprises the steps of:
(a) inserting into the genome of a plant cell of said plant a recombinant DNA molecule comprising:
   (i) a pollen specific promoter;
   (ii) a DNA sequence that codes for a cytotoxic molecule; and
   (iii) a terminator sequence which defines a termination signal during transcription of said DNA sequence.
(b) obtaining a transformed plant cell; and
(c) regenerating from said transformed plant cell a genetically transformed plant which is male sterile.

In accordance with the preceding method the invention is also directed to:
(i) a plant cell which has been transformed with a recombinant DNA molecule as defined in step (a) above and which is capable of being regenerated into a male sterile plant; and
(ii) a plant which has been transformed with a recombinant DNA molecule as defined in step (a) above and which is male sterile.

According to yet another aspect of the invention as claimed herein, we provide a method to produce hybrid seed with restored male fertility from plants selected from those species of pollen producing plants which are capable of being genetically transformed comprising the steps of:
(a) inserting into the genome of a plant cell of said pollen producing plant that is capable of being regenerated into a differentiated whole plant, a gene which confers on said plant resistance to a chemical agent or physiological stress and linked to said gene a recombinant DNA molecule comprising:
   (i) a DNA sequence which codes for a cytotoxic molecule;
   (ii) a pollen targeted promoter which functions in said plant cell to cause transcription of said DNA sequence; and
   (iii) a terminator sequence which defines a termination signal during transcription of such DNA sequence.
(b) obtaining a transformed plant cell;
(c) regenerating from said plant cell a genetically transformed plant which is male sterile;
(d) increasing the number genetically transformed plants by:
   i) crossing the genetically transformed plant described in step (c) above with a suitable male fertile plant;
   ii) using a chemical agent or physiological stress to eliminate plants which do not contain the genes described in step (a) above among plants grown from seed produced by such cross; and
   iii) repeating sucn a cross over several generations with the plants obtained as in step (d) ii) above in the presence of said chemical agent or physiological stress to increase the numbers of male sterile plants;
(e) inserting into a plant cell of suitable male fertile plant selected from the same species a gene which confers on said plant resistance to a chemical agent or physiological stress and linked to said gene a recombinant DNA molecule comprising:
   (i) a DNA sequence which codes for RNA that is complementary to the RNA sequence coding for said cytotoxic molecule;
   (ii) a promoter which causes transcription of the DNA sequence defined in step (e)(i) above at or about the time of transcription of the DNA sequence defined in step (a)(i);
   (iii) a terminator sequence which defines a termination signal during transcription of the DNA sequence described in step (e)(i) above;
(f) obtaining a transformed plant cell from step (d);
(g) regenerating from said transformed plant cell described in step (d) above a genetically transformed male fertile plant.
(h) producing a restorer line by:
   i) selfing the genetically transformed plant described in (g) and selecting from that selfing progeny, a plant homozygous for the male restorer trait;
   ii) permitting self-fertilization of said plant homozygous for the male restorer trait; and
   iii) growing seed of said plant, over a number of generations to increase the number of genetically transformed plants;

(i) effecting a hybrid cross by pollinating said male sterile plants with pollen from said genetically transformed male fertile plants.

Selection for a plant that is homozygous for said restorer trait may be carried out by conducting anther or isolated microspore culture of the genetically transformed plant carrying said restorer trait, or preferably by selfing said plant in isolation prior to selection.

It is particularly desirable to restore fertility as discussed above where the target plant species does not outcross. Where the target plant species does outcross it is preferable but not necessary to produce a restorer line as described in steps (e) through (h) above. Crossing with a suitable male fertile plant will yield hybrid seed comprising a mixture of sterile hybrid seed and fertile hybrid seed because of the segregation of the recombinant DNA causing male sterility. The fertile plants that are present will allow full pollination of the male sterile hybrids.

As discussed above, in a hybrid seed production scheme where there are alternating rows of male sterile plants and male fertile plants, it may be simpler but not essential to carry out the final selection of male sterile plants (step (d) of the preceding method) in the field alongside suitable male fertile plants. Therefore it is desirable if the suitable male fertile plants are previously transformed to resistance to the selective agent to avoid having to selectively apply said agent to the rows of male sterile plants.

In accordance with the preceding method the invention is also directed to:
(i) a plant cell which has been transformed with a recombinant DNA molecule as defined in step (e) above and which is capable of being regenerated into a male fertile plant carrying the restoration trait;
(ii) a plant which has been transformed with a recombinant DNA molecule as defined in step (e) above and which carries the restoration trait.
(iii) hybrid seed containing the genes described in steps (a) and (e) above.

Improvements and further embodiments of methods relating to pollen specific cytotoxicity will now be discussed.

One improvement relates to the nature of the molecules that are harmful or toxic to the pollen grain. In addition to cellular poisons such as ricin and diptheria toxin, of which abrin is another example, these substances include, but are not limited to, degrading or destructive enzymes such as ribonuclease, DNAse, lipase, or protease, molecules that disrupt or de-stabilize cytoplasmic integrity such as poly-lysine or poly-proline, or cryptocytotoxins which which break down into cytotoxic molecules. Examples of cryptocytotoxins include cleavable conjugates of molecules which when cleaved are cytotoxic, for example a cleavable conjugate of glucuronate and a herbicide (eg: glyphosate), napthalene-acetamide and indole-acetamide.

Thus it is possible to carry out the preceding methods by regulating the expression of a gene coding for any of the improved cytotoxins using a pollen targeted promoter to produce male sterile plants and crossing said male sterile plants with any suitable fertile male plant to yield hybrid seed that comprises a mixture of male sterile and male fertile seed or by crossing said male sterile plants with a restorer line to yield fertile hybrid seed.

Variations on the type of restorer line that may be used will now be discussed.

First, with respect to the cellular poisons, an appropriate restoration line may comprise restorer plants containing DNA sequences coding for the production of a detoxifying molecule. Second, with respect to the destructive enzymes, an appropriate restoration line may comprise restorer plants containing DNA sequences coding for the production of a specific enzyme inhibitor. Third, with respect to the cytoplasmic disrupting molecules, an appropriate restoration line may comprise restorer plants containing DNA sequences coding for the production of a specific peptidase.

It follows that cytotoxic methods of producing male sterile plants may involve the pollen specific synthesis of molecules capable of disruption of pollen function or destruction of pollen integrity and that corresponding restoration methods involve the pollen specific synthesis of molecules that specifically block, neutralize or destroy said molecules capable of disruption of pollen function or destruction of pollen integrity.

With respect to the cryptocytotoxic method, a restorer line is not necessarily required since restoration may be accomplished by growing plants in the absence of the cryptocytotoxic molecule. As discussed above with respect to any of the cytotoxic methods, including the cryptocytotoxic method, it is not essential to use a restorer line where the male sterile line is crossed with a suitable male fertile line to yield hybrid seed that comprises a mixture of fertile seed and sterile seed.

In summary, we provide a method of producing hybrid seed with restored male fertility from those species of pollen producing plants capable of being transformed and regenerated into a differentiated whole plant which method comprises:
(a)
   (i) inserting into the genome of a plant cell of said plant that is capable of regeneration into a differentiated whole plant, a gene which confers on said plant resistance to a chemical agent or a naturally occurring or artificially induced physiological stress and linked to this a recombinant DNA molecule comprising:
      (A) a DNA sequence that when transcribed and translated codes for a cytotoxic molecule or a molecule which renders a substance cytotoxic;
      (B) a pollen targeted promoter which functions in said plant to cause transcription of said DNA sequence into RNA at or about the time of the transcription of the sense gene in developing pollen; and preferably
      (C) a terminator sequence which defines a termination signal during transcription of said DNA sequence:
   (iii) obtaining a transformed plant cell of said plant; and
   (iv) regenerating from said plant cell a plant which is genetically transformed with said DNA sequences described in (a)(ii) above and is male sterile, and
(b) increasing the number of genetically transformed male sterile plants by:
   (i) clonal propagation of said genetically transformed male sterile plant described in step (a) using tissue explants thereof, or other in vitro propagation techniques; or
   (ii)
      A) crossing the genetically transformed male sterile plant described in (a) with a isogeneic male fertile plant;
      B) using the chemical agent or physiological stress defined in (a)(ii) above to eliminate plants which do not contain the DNA sequence defined in (a)(ii) amongst plants grown from seed produced by such cross; and
      C) repeating such cross over several generations with plants obtained in step (b)(iii) above in the presence of said chemical agent or physiological stress to increase the numbers of male sterile plants.
(c) producing a male fertile restorer plant by:
   (i) inserting into the genome of a plant cell of a suitable male parent plant that is capable of regeneration into a differentiated whole plant a gene that confers resistance to a chemical agent or a naturally occurring or artificially induced physiological stress, linked to a recombinant DNA sequence comprising:
      (A) a gene that codes for a molecule that negates the disruption caused to tissues critical to pollen formation or function in said genetically transformed female parent plant;
      (B) a promoter that functions in said tissues critical to pollen formation or function to cause transcription of said gene into RNA at or about the time that the sense gene described in (a)(i) is active, preferably a pollen targeted promoter; and preferably
      (C) a terminator sequence which defines a termination signal during transcription of said DNA sequence.
(d) increasing the number of genetically transformed male fertile restorer plants by:
   (i) selfing the genetically transformed plant carrying the restorer trait described in (c), and selecting a plant homozygous for the restorer trait and increasing said plant by selfing in isolation; or
   (ii) when applicable, conducting anther or isolated microspore culture of the genetically transformed plant carrying the restorer trait described in (C) and selecting a plant homozygous for the restorer trait and increasing said plant by selfing in isolation.
(e) effecting a hybrid cross by pollinating said male sterile plants described in (a) and increased in (b) in the presence of the chemical agent or physiological stress defined in (a)(ii),(if required), with pollen from male fertile restorer plants as described in (c) and increased in (d).

It is desirable to restore fertility as discussed above where the target plant species does not outcross. Where the target plant species does outcross it is preferable but not necessary to produce a restorer line as described in steps (c) through (e) above. Crossing with a suitable male fertile plant will yield hybrid seed comprising a mixture of sterile hybrid seed and fertile hybrid seed because of the segregation of the recombinant DNA causing male sterility. The fertile plants that are present will allow full pollination of the male sterile hybrids. As discussed above, restoration may also not be required for the cryptocytotoxic method.

In accordance with the above described method a cytotoxic molecule can be any molecule that when active would disturb normal cell function leading to cell death. The action of such cytotoxic molecules can be negated by using an antisense gene to regulate the expression of the cytotoxic molecule. Alternatively, the action of such cytotoxic molecules can be negated by a gene that codes for a negator molecule that blocks, inhibits or destroys said cytotoxic molecules. Examples of such cytotoxic molecules and negator molecules are the proteinase enzyme trypsin and soybean or cowpea trypsin inhibitor; ribonuclease and a ribonuclease inhibitor; or a starch degrading enzyme such as alpha-amylase and an alpha-amylase inhibitor.

Specific embodiments of the cryptocytotoxic method will now be discussed.

In theory any activity that is known to be encoded by one or more identifiable DNA sequences that can convert a non-toxic to a toxic substance can be employed within the scope of the invention. This includes methods of rendering the developing pollen suceptible to the cyto-toxic effects of IAA (indole acetic acid) or NAA (alpha-naphthalene acetic acid) by transformation of plants with a pollen specific recombinant DNA molecule encoding the enzyme indole acetamide hydrolase (IaMH). One source of the enzyme IaMH is the bacterium Agrobacterium tumefaciens (Inze, D., et al, 1984, Mol. Gen. Genet. 194:265-74). This enzyme will convert non-toxic indoleacetamide to IAA or non-toxic naphthalene acetamide to toxic NAA.

Alternatively, the present invention contemplates rendering the developing pollen susceptible to the cytotoxic effects of methoxyvinyl glycine by transformation of plants with a pollen specific recombinant DNA molecule encoding the enzyme methoxinine dehydrogenase (MDH). One source of MDH is the bacterium Pseudomonas aeruginosa (Margraff, R., et al., 1980, Experimentia 36: 486). MDH converts non-toxic 2-amino-4-methoxy-butanoic acid (methoxinine) to toxic . methoxyvinyl glycine.

Alternatively, the present invention also contemplates rendering the developing pollen suceptible to the cytotoxic effects of rhizobitoxine (2-amino-4-[2-amino-3-hydroxypropyl]-trans-3-butanoic acid) by transformation of plants with a pollen specific recombinant DNA molecule encoding the enzyme rhizobitoxine synthase, one source of which is the bacterium Rhizobium japonicum (Owens, L.D., et al., 1973, Weed Science 21: 63-66). Rhizobitoxine synthase converts 2-amino-4-methoxy-butanoic acid to rhizobitoxine.

Alternatively, the present invention also contemplates rendering the developing pollen susceptible to the toxic effects of glyphosate (N-[phosphomethyl]glycine) by transformation of plants with a pollen specific recombinant DNA molecule that codes for the enzyme beta-glucuronidase which releases toxic glyphosate from non-toxic gylphosate-glucuronic acid conjugates.

In accordance with the preceding methods the invention is also directed to a cell which has been transformed with recombinant DNA as defined in steps (a) and/or (c) above, plants that contain the recombinant DNA defined in (a) and/or (c) above and hybrid seed that contains the recombinant DNA defined in (a) and (c) above.

### BRIEF DESCRIPTION OF THE DRAWINGS

Figure 1 is a representation of the construction of an anti-sense gene vector that was used for anti-sense RNA inhibition of Beta-glucuronidase gene activity in transgenic plants.

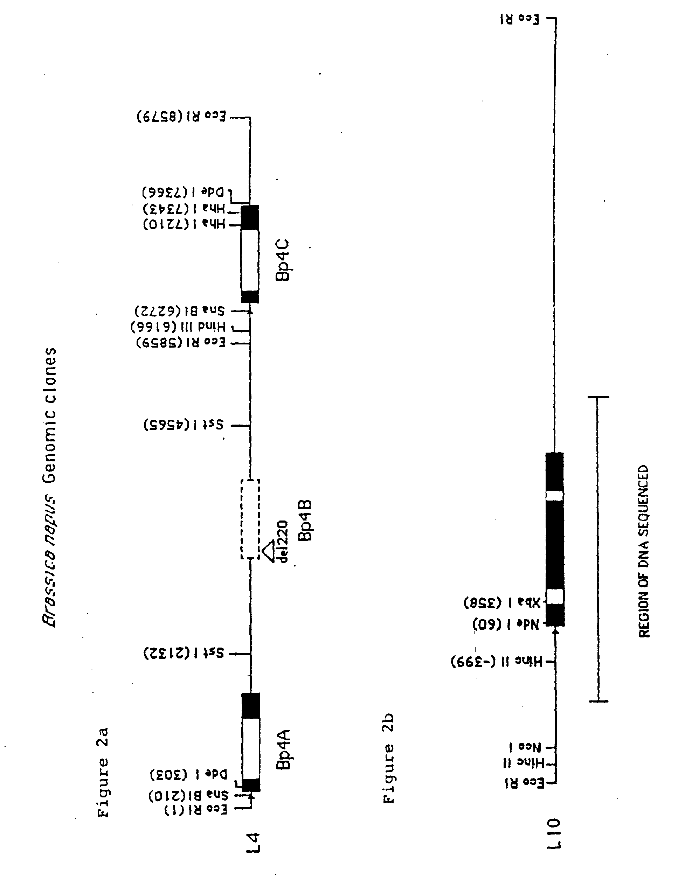

Figure 2a is a schematic representation of a restriction map and coding region of clone number L 4, a microspore specific clone isolated from a Brassica napus genomic library. The clone contains three similar copies of a single gene. These genes are identified as Bp4A, Bp4B and Bp4C. The first (Bp4A) and third (Bp4C) genes are functional, the second gene has modifications that most likely render it non-functional. The restriction map is made diagramatically in that the non-transcribed regions are shown as a single line, while the transcribed regions are shown as a boxed area. The second gene (Bp4B) is identified on the basis of sequence homology and is shown as a boxed area with a dotted line. The notation "del 220" refers to . an approximately 220 base pair deletion/rearrangement that has presumably inactivated the second gene (Bp4B) in this clone. Start of transcription is located at the leftmost side of each boxed area (except in the case of gene Bp4B) and exon and intron positions are noted by the exons being filled in with black and the intron positions being left unfilled. A small arrowhead is shown on the non-transcribed 5' region of each gene, this arrowhead serves to indicate the promoter region of each gene. Restriction sites are identified such that the number of the first nucleotide of the restriction enzyme recognition site is shown. Not all restriction sites are shown, only those relative to the costructs detailed within are indicated. The genes are presented with the 5' region on the left side and the 3' region on the right side. The numeration of the DNA sequence is from left to right, 5' to 3' in all cases.
Figure 2b is a schematic representation of a restriction map and coding region of clone number L 10, a microspore specific clone isolated from a Brassica napus genomic library. The clone contains a single gene. Start of transcription, exon, intron and promoter positions are noted as in figure 2a. Restriction sites are identified such that the number of the first nucleotide of the restriction enzyme recognition site is shown. The genes are presented with the 5' region on the left side and the 3' region on the right side. The numeration of the DNA sequence is from left to right, 5' to 3' in all cases.

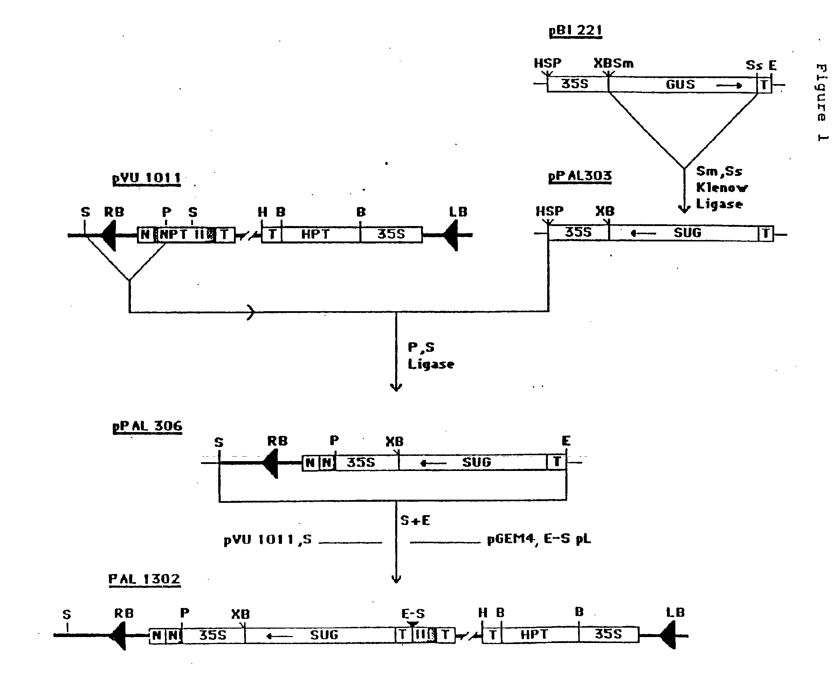

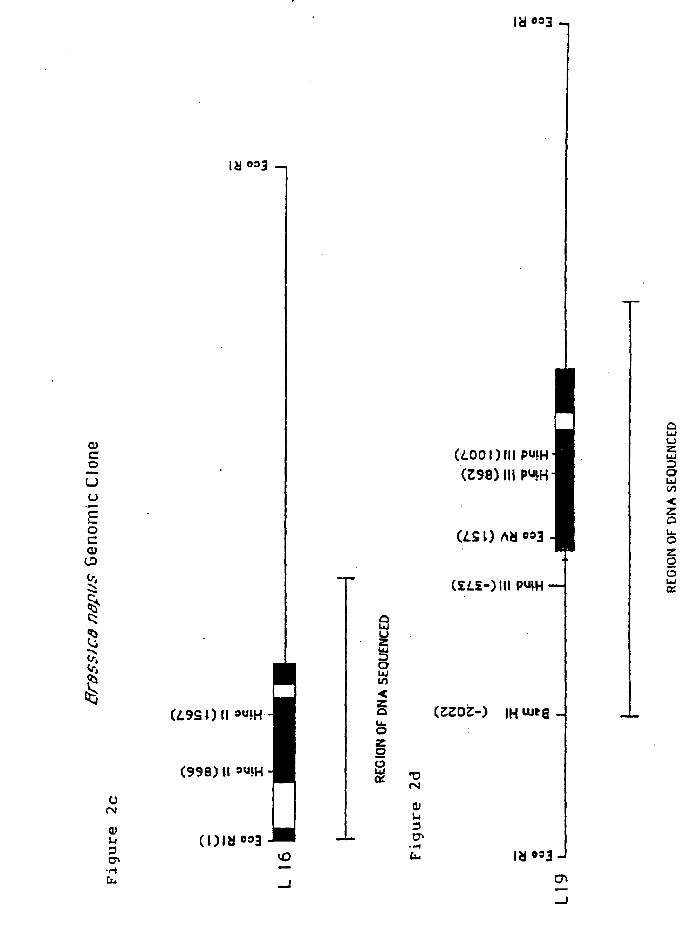

Figure 2c is a schematic representation of the restriction map and coding region of clone number L 16, a microspore specific clone isolated from a Brassica napus genomic library. The clone contains a single gene that shows similarity to clone L 10. Intron and exon positions are noted as in figure 1. Restriction sites are identified such that the number of the first nucleotide of the restriction enzyme recognition site is shown. The genes are presented with the 5' region on the left side and the 3' region on the right side. The numeration of the DNA sequence is from left to right, 5' to 3' in all cases.
Figure 2d is a schematic representation of the restriction map and coding region of clone number L 19, a microspore specific clone isolated from a Brassica napus genomic library. The clone contains a single gene. Start of transcription, exon, intron and promoter positions are noted as in figure 1. Restriction sites are identified such that the number of the first nucleotide of the restriction enzyme recognition site is shown. The genes are presented with the 5' region on the left side and the 3' region on the right side. The numeration of the DNA sequence is from left to right, 5' to 3' in all cases.
Figure 3a is the complete nucleotide sequence of the clone L 4 represented in Figure 2a. Only the coding strand is shown for clarity.
Figure 3b is the nucleotide sequence of the portion of the clone L 10 shown in figure as being underlined in figure 2b. Only the coding strand is shown for clarity.
Figure 3c is the nucleotide sequence of the portion of the clone L 16 shown in figure as being underlined in figure 2c. Only the coding strand is shown for clarity.
Figure 3d is the nucleotide sequence of the portion of the clone L 19 shown in figure as being underlined in figure 2d. Only the coding strand is shown for clarity.

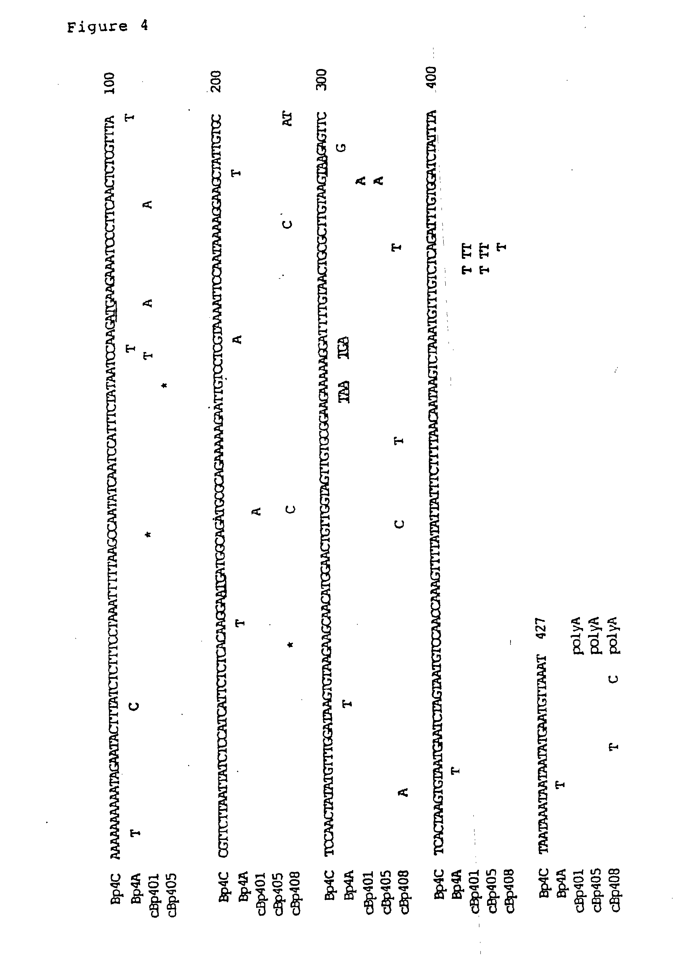

Figure 4 is the nucleotide sequence of 3 cDNA clones isolated from a microspore derived cDNA library of Brassica napus. These clones are named cBp401, cBp405, and cBp408. These three cDNA clones are extremely homologous to members of the L4 Brassica napus microspore specific gene family (Bp4A, Bp4B, Bp4C). The nucleotide sequence of two of these 3 members of the L4 Brassica napus microspore specific gene family are shown in this figure (Bp4A, Bp4C). The gene Bp4C was chosen as a master sequence for comparison. The deduced nucleotide coding sequence for the genes Bp4A and Bp4C is shown as a sequence from which the two exons of the genes have been spliced together at the positions normally spliced in vivo. This gives rise to the coding sequence in the mature mRNA. The cDNA clones are aligned with the sequence of Bp4C such that only nucleotide changes are shown. The sequences are therefore represented as varients of a single master sequence of gene Bp4C which is shown on line 1. The ATG start codon as well as the TGA or TAA stop codons are underlined. These three cDNA clones correspond to related members of the Brassica napus microspore specific gene family a portion of which is contained in the clone L4.
Figure 5 is a partial nucleotide sequence of a cDNA clone that is closely homologous to the gene contained in the clone L10, the restriction map of which is shown in figure 2b.
Figure 6 is the nucleotide sequence of the cDNA clone that is the gene product of clone L19, the restriction map of which is shown in figure 2d.
Figure 7 (7A, 7B, 7C, 7D) are schematic representations of describing production of vectors containing the promoter and promoter regions from clone L4. The specific examples are discussed in greater detail below.
Figure 8 is a schematic representation of producing vectors containing the promoter regions of clone L10, the details of which are discussed below.
Figure 9 is a schematic representation of producing vectors conaining the promoter regions of clone L19, the details of which are discussed below.
Figure 10 is a schematic representation of a restriction map of a Brassica napus genomic clone that contains a gene that is constitutively expressed at high levels in all cells including developing pollen cells. The portion of the clone that was used to provide promoter regions for the production of anti-sense RNA is shown, this construct gives rise to an anti-sense RNA that contains a region of transcribed RNA from this gene.
Figure 11 is a schematic representation of producing a gene coding for a polylysine protein .
Figure 12 is a schematic representation of producing an anti-sense gene specific to the intron region of clone L19 and a restorer that lacks the intron region targetted for anti-sense RNA inhibition.
Figure 13 is a representation of the procedure used to isolate the T-DNA gene 2 (the IamH gene) of Agrobacterium tumefaciens Ti plasmid and the production of a promoterless version of this gene.
Figure 14 is a schematic representation of producing clones containig versions of a ricin A chain coding region.
Figure 15 is a histogram showing GUS activity in plants transformed with sense and anti-sense GUS genes.

### DETAILED DESCRIPTION OF THE FIGURES

In Figure 1, a schematic representation of the production of the anti-sense vector PAL 1302 is shown. A plasmid containing the GUS gene (Beta-glucuronidase, described in Jefferson, R.A., Plant Molecular Biology Reporter, 1987, 5: 387-405) in the anti-sense orientation flanked by the CaMV 35S promoter and the nos ter termination signal was obtained from the vetor pBI 221.1 (available from Clonetech Labotatories, Palo Alto, CA, USA). The GUS coding sequence found between the CaMV 35S promoter and the nos ter of the vetor pBI 221.1 was excised and digested with the restriction enzymes Sma I and Sst I. The Sst I site was made blunt ended using Klenow fragment of DNA polymerase I and the blunt ended vector and GUS coding sequence were religated. A plasmid (pPAL 303) containing the Gus coding sequence inverted with respect to the direction of transcription of the CaMV 35S promoter was identified.

The binary vector PAL 1302 containing the anti-sense GUS gene was constructed using the vector pVU 1011 (obtained from The Plant Breeding Institute, Cambridge, UK). pVU 1011. contains the hygromycin phosphotransferase coding sequence flanked by the CaMV 35S promoter and the nos ter inserted into the polylinker of the Agrobacterium binary vector Bin 19 described by Bevan, M., Nucl. Acids Res. 1984, 12: 8711-8721. The vector pVU 1011 can confer both hygromycin and kanamycin resistance to transformed plant cells. The insetion of the CaMV 35S promoter anti-sense GUS nos ter fragment into pVU 1011 was accomplished in such a way as to inactivate the NPT II gene of this vector and was performed a follows. A small Sph I - Pst I restriction fragment containing the right border (RB), the NOS promoter and the beginning of the NPT II coding sequence of pVU 1011 was first subcloned into the Sph I and Pst I sites proceeding the CaMV 35S promoter of pPAL 303 to form pPAL 306. Digestion of pPAL 306 with Sph I and Eco RI released a fragment consisting of the RB, the NOS promoter, the beginning of the NPT II coding sequence and the CaMV 35S promoter - anti-sense GUS noster construct. This fragment was then ligated into the Sph I sites of pVU 1011 by adding to the ligation pGEM-4Z (Promega Biotech, Madison, WI, USA) cut with Eco RI and Sph I to provide a small fragment of polylinker as a bridge between the Sph I site of pVU 1011 and the Eco RI site of the isert from pPAL 306 respectively. The orientation of the insert was verified and a binary vector (PAL 1302) possessing a reconstructed RB fragnment and a NPT II gene inactivated by the CaMV 35S promoter - anti-sense GUS gene-noster insertion was identified. This vector can confer only hygromycin resistance to plants and carries the anti-sense GUS gene.

In Figure 2a-d the orientation of the genes contained within the four microspore specific clones from Brassica napus are from 5' to 3'. As shown, the 5' region corresponds to the promoter region and is identified with a small arrowhead. The 3' region delineates the end point of transcription of the gene. Clones L4, L10 and L19 were used for the isolation of microspore specific promoter fragments and for the isolation of microspore specific coding regions. The non-transcribed regions are identified as a single thin line, while the regions of the clones that are transcribed are demarcated by a boxed area. Within this boxed area the portion of the transcribed DNA that represents the exon regions is demarcated by being filled in black while the intron sequences are left unfilled. The approximate regions of DNA sequenced for clones L10, L16 and L19 are shown by underlining. Restriction sites identified are those that are relevant to the constructs detailed below. The right and left arms of the lambda cloning vectors are not shown.

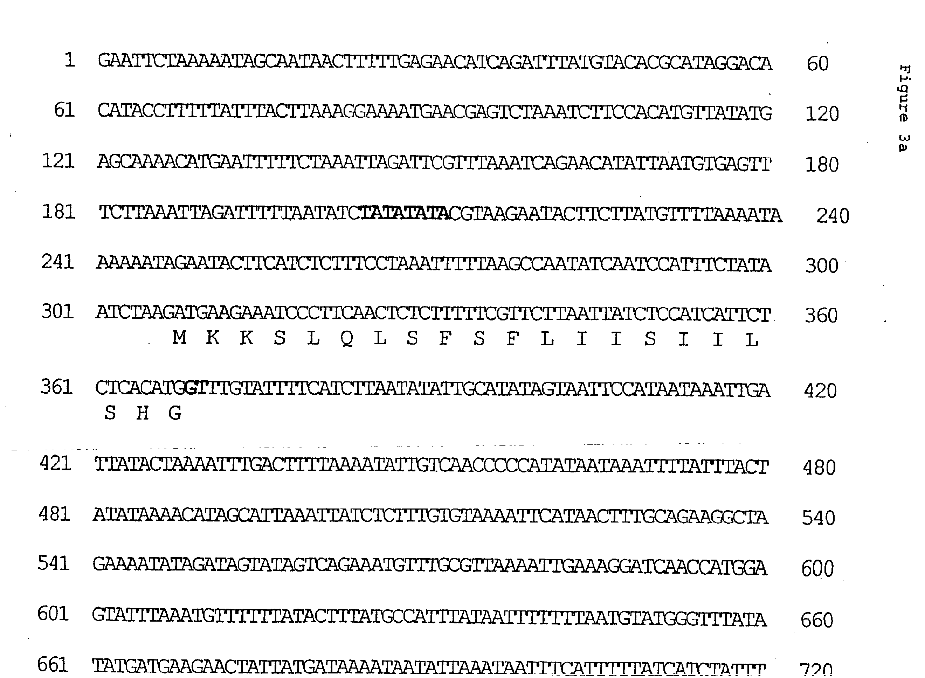

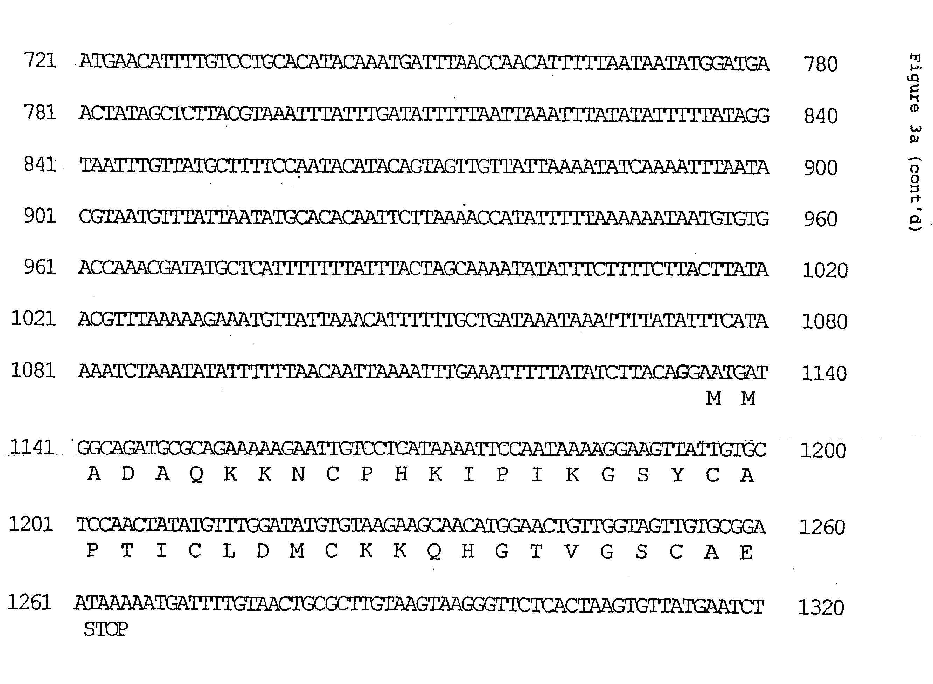

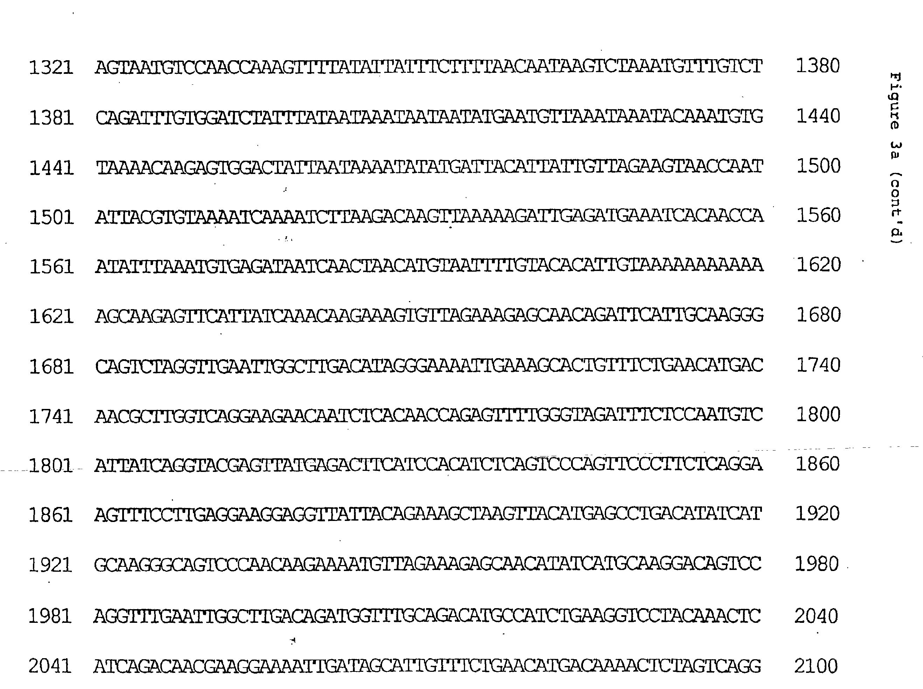

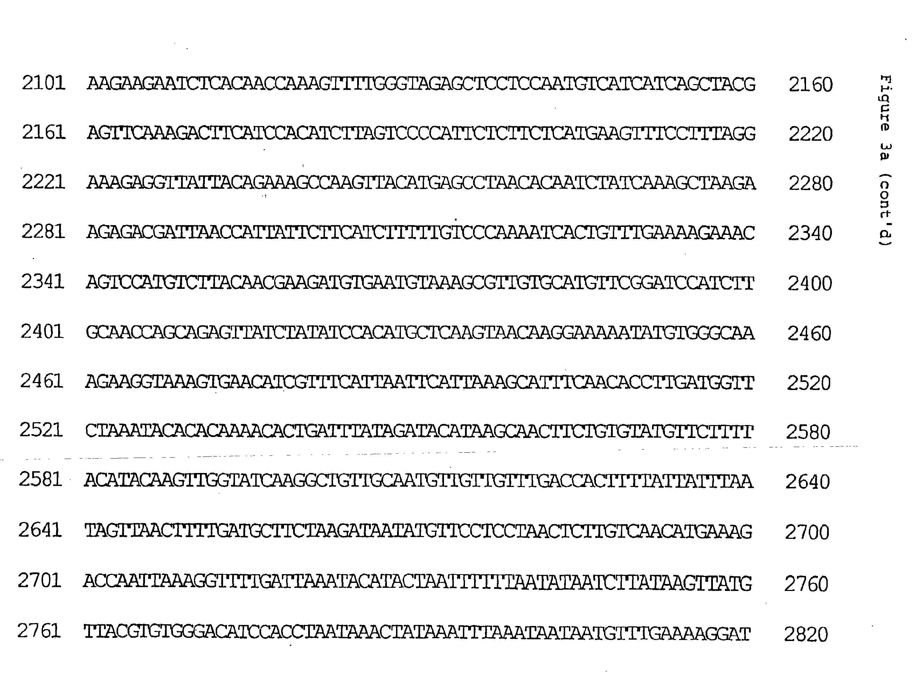

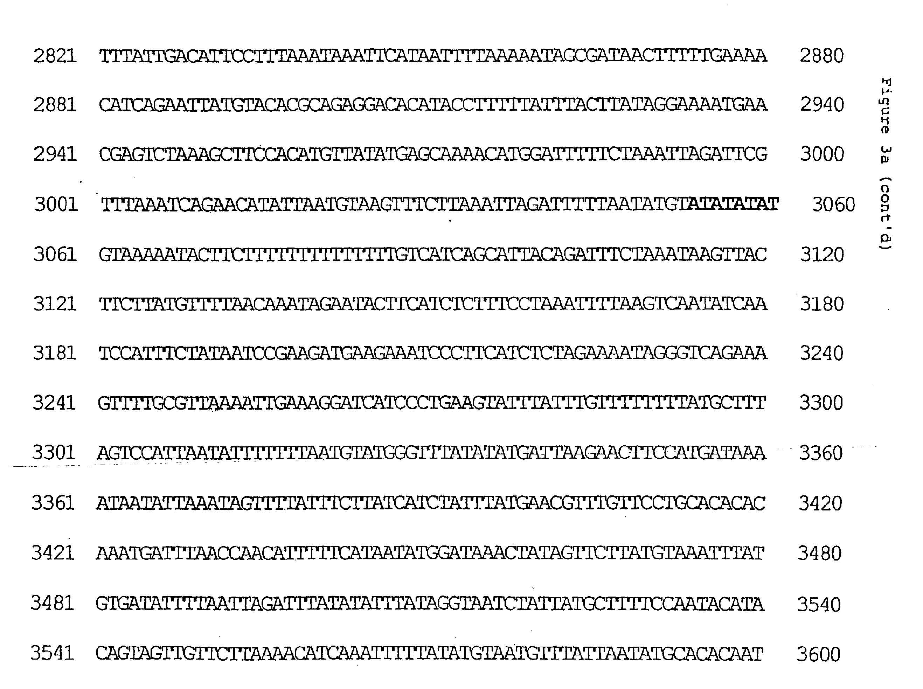

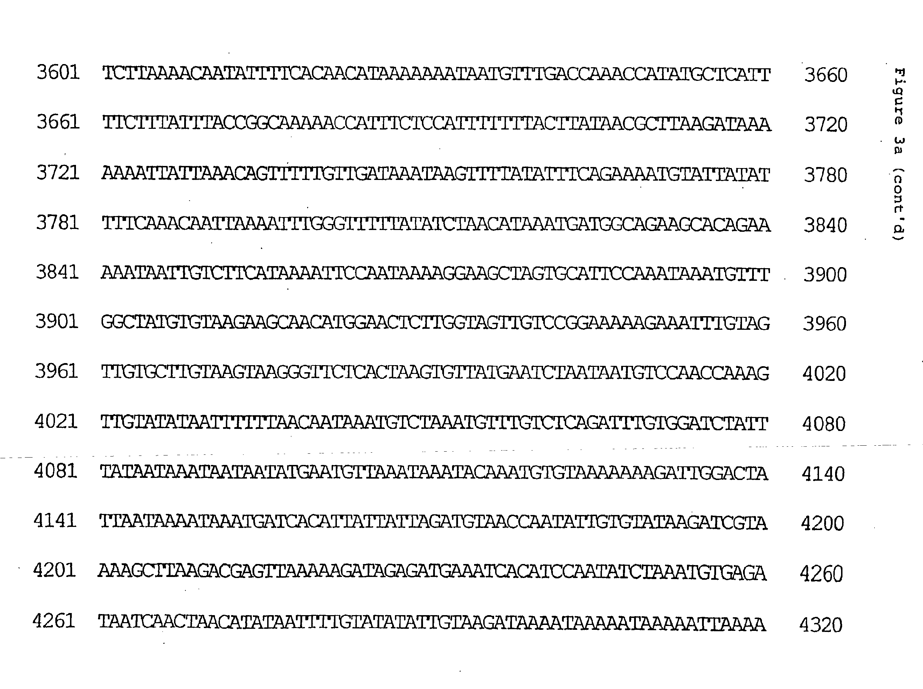

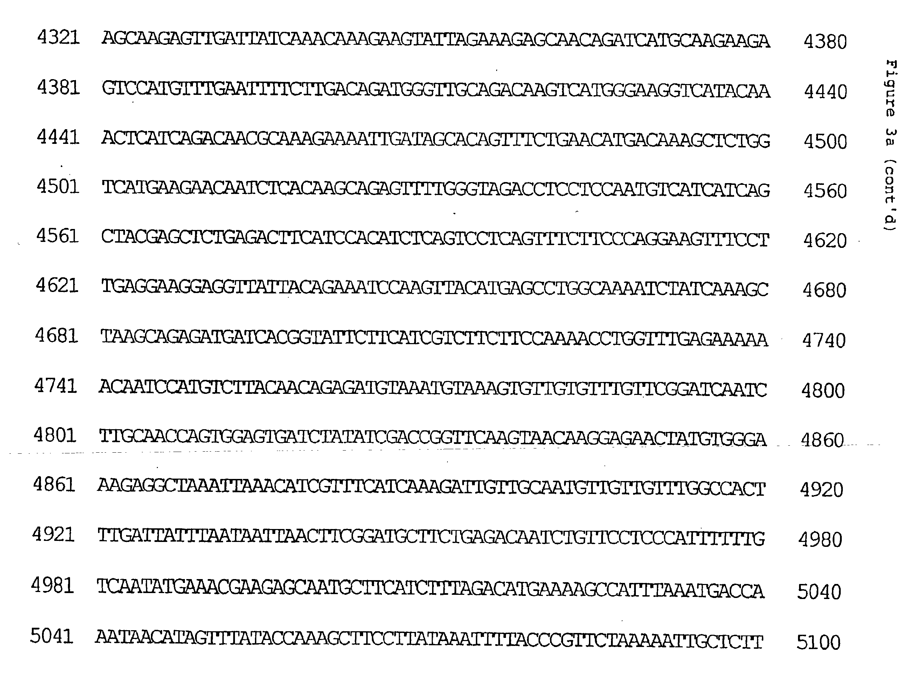

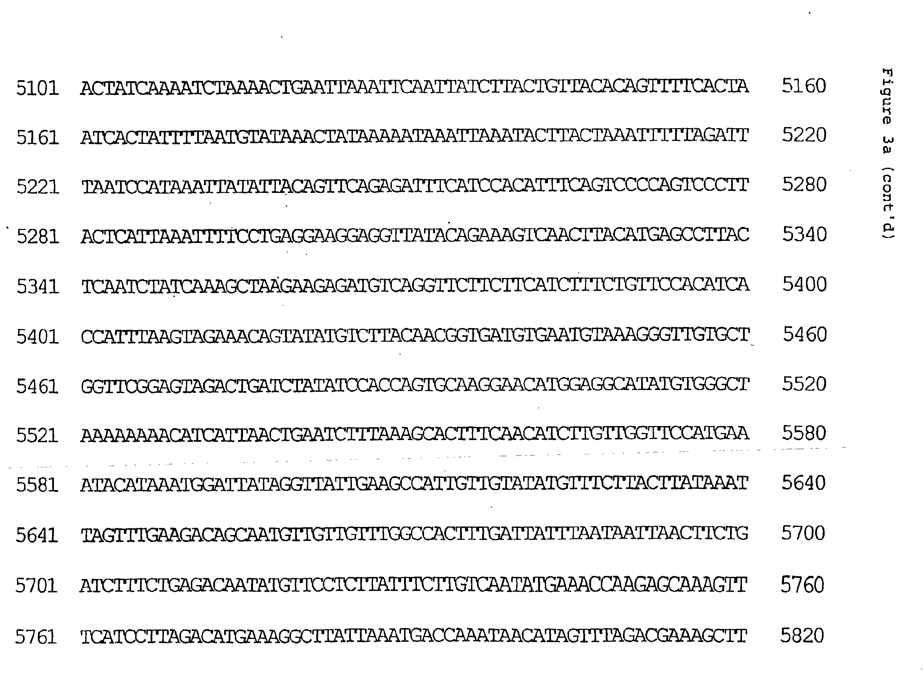

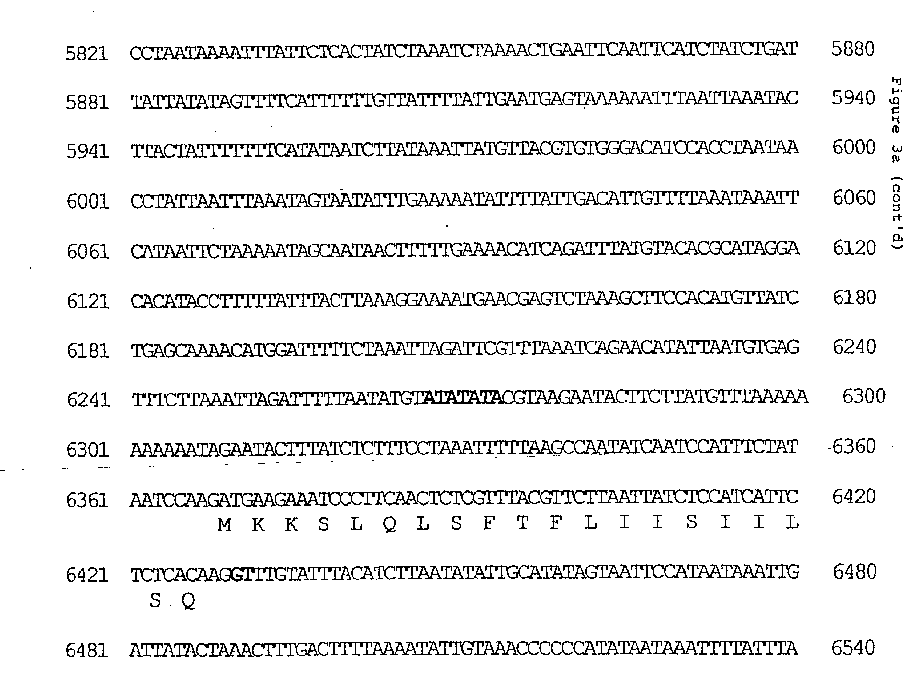

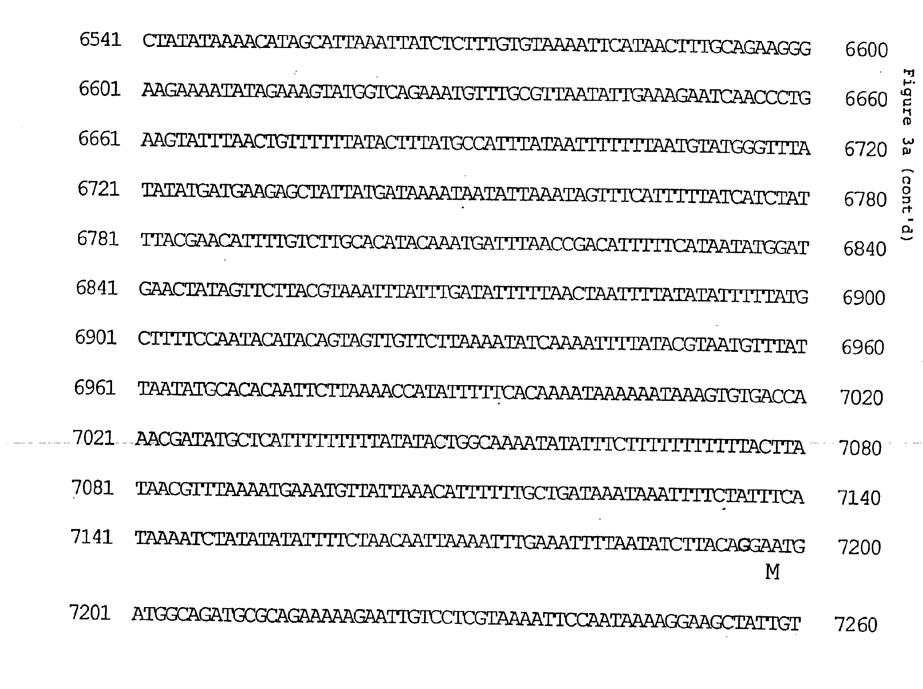

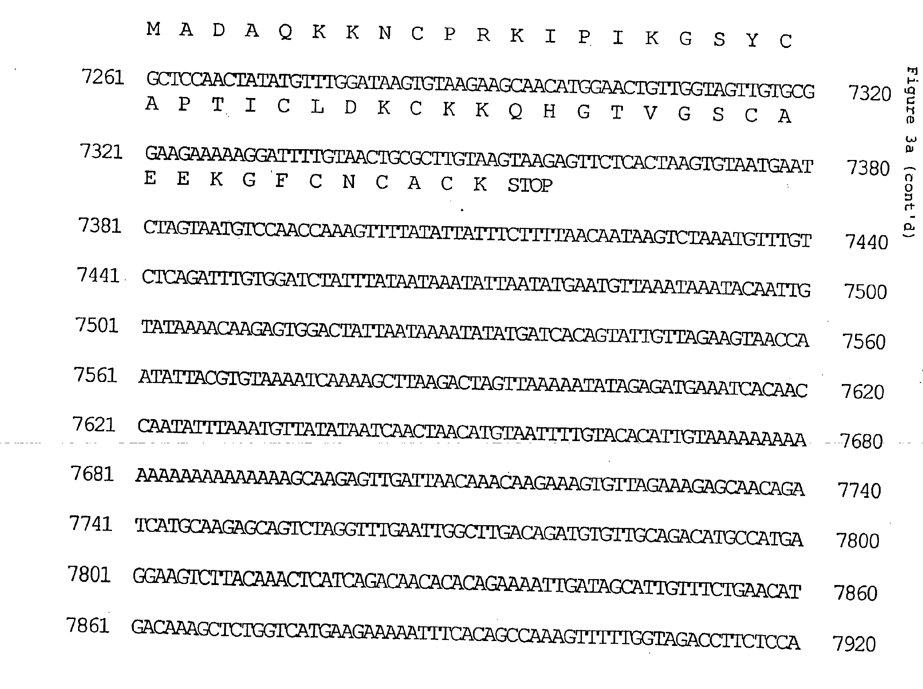

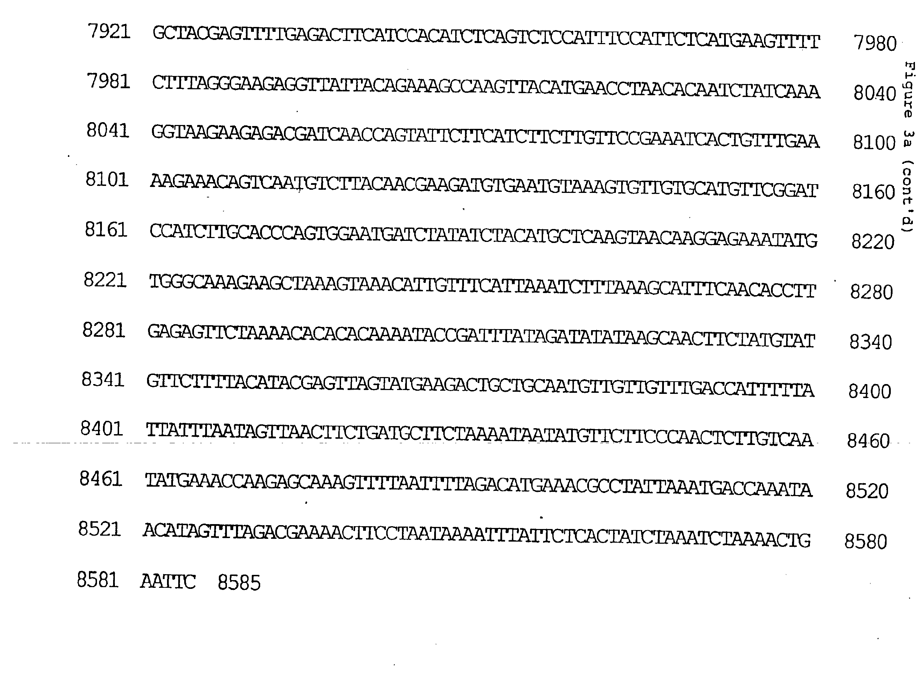

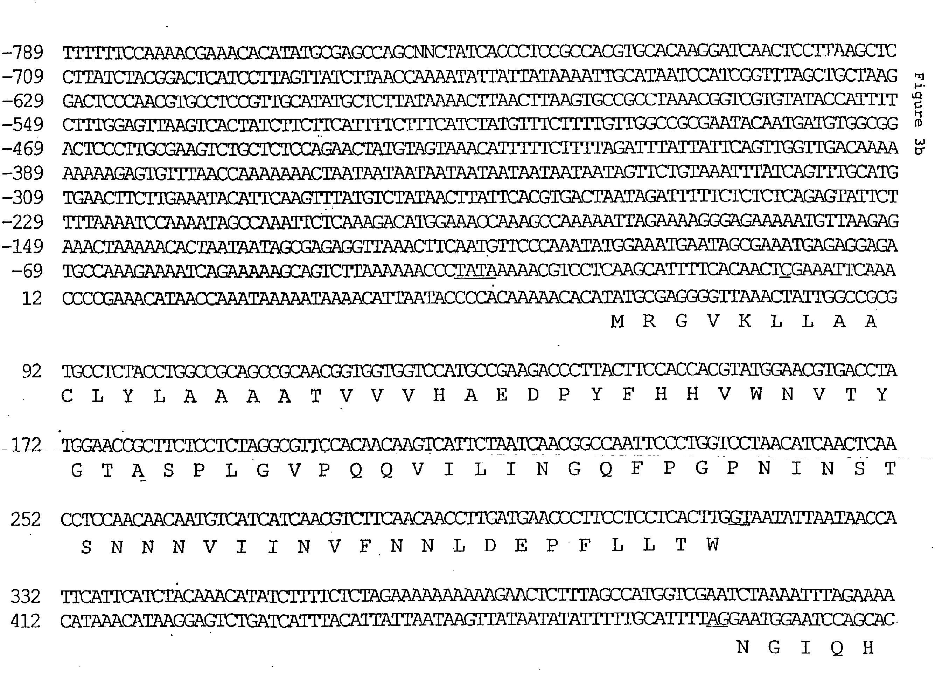

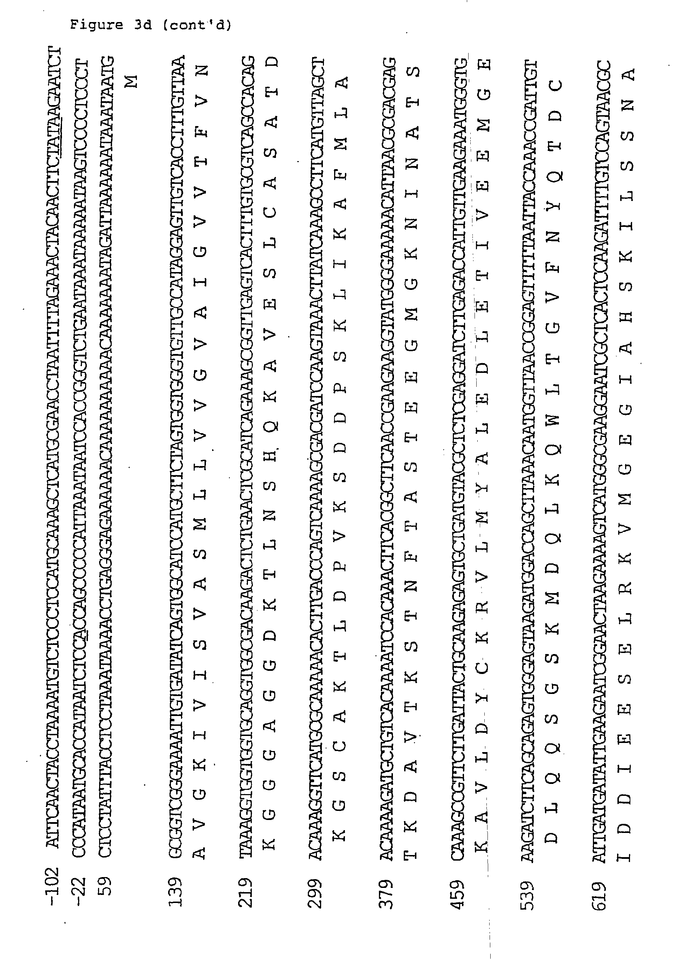

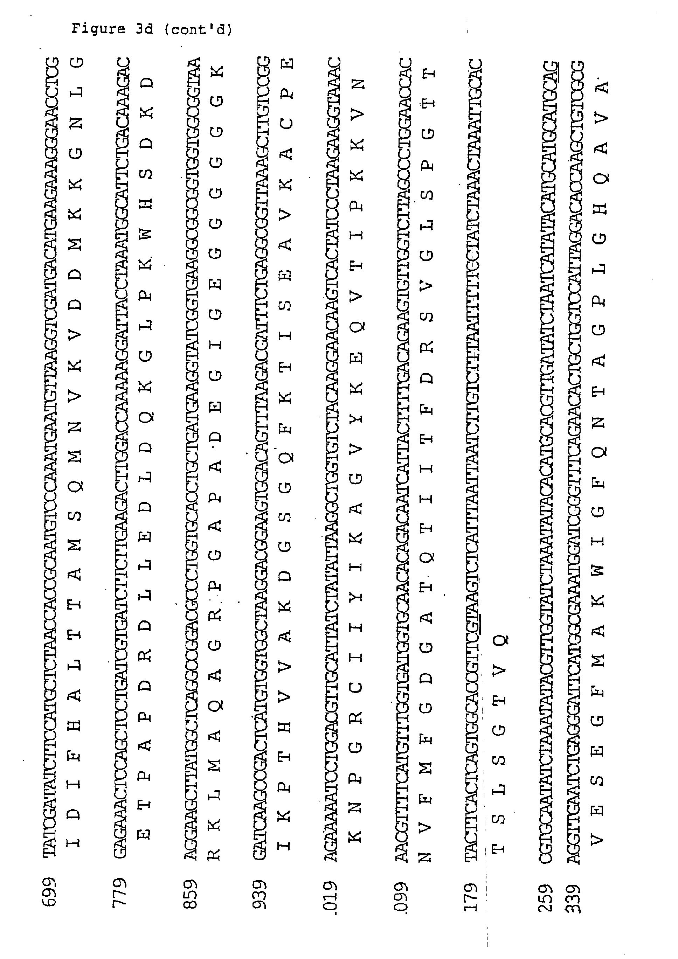

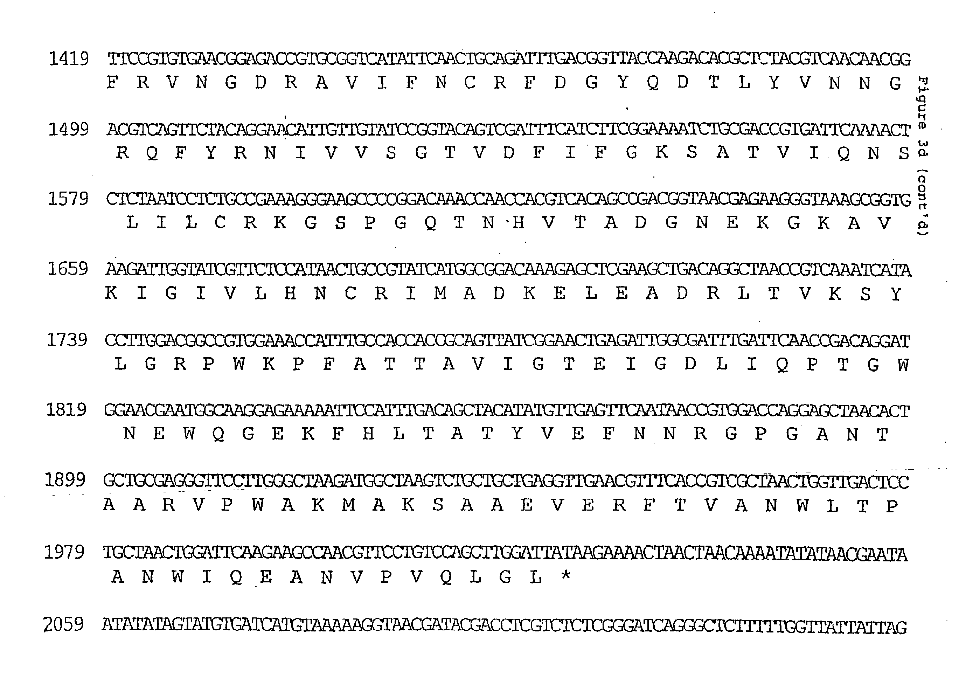

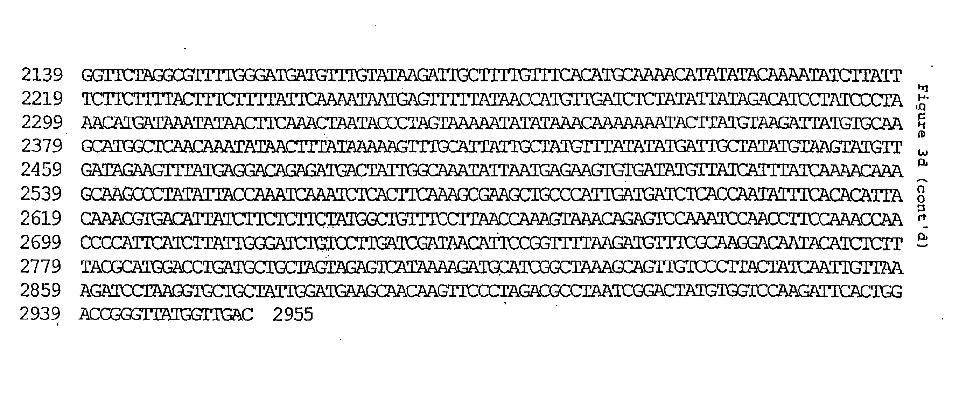

In Figures 3a-d, the complete DNA sequence of the clone L4 is shown along with the DNA sequence of the portions of the clones L10, L16 and L19 that are identified in Figures 2a-d. In Figure 3a, clone L4, nucleotide 1 in the complete sequence is at the left-most Eco R1 site while nucleotide 8579 is at the first nucleotide of the rightmost Eco R1 site. The start of transcription of gene 1 in clone L4 is nucleotide 235. The 5' and 3' intron splice sites are identified in boldface type. The start ATG codon is shown as well as the stop termination codon. The deduced amino acid sequence of the proteins encoded for by these genes are also shown. The end of transcription for gene 1 is approximately nucleotide 1427. The second gene in clone L4 is most likely non-functional due to a insertion and a deletion that occurs in the region of the promoter and first exon. This gene was not utilized for constructs. The third gene in clone L4 has a transcriptional start at position number 6298 in the DNA sequence and transcription ends at approximately nucleotide 7490. The ATG start codon, intron splice sites and termination stop codon are all identified as above. Vectors were constructed from this clone by using promoter fragments from both genes 1 and 2, as well as promoter fragments from genes 1 and 2 that contained the first exon and intron sequences and a short portion of the second exon for each of the genes. The specific promoter fragment constructs are detailed below.

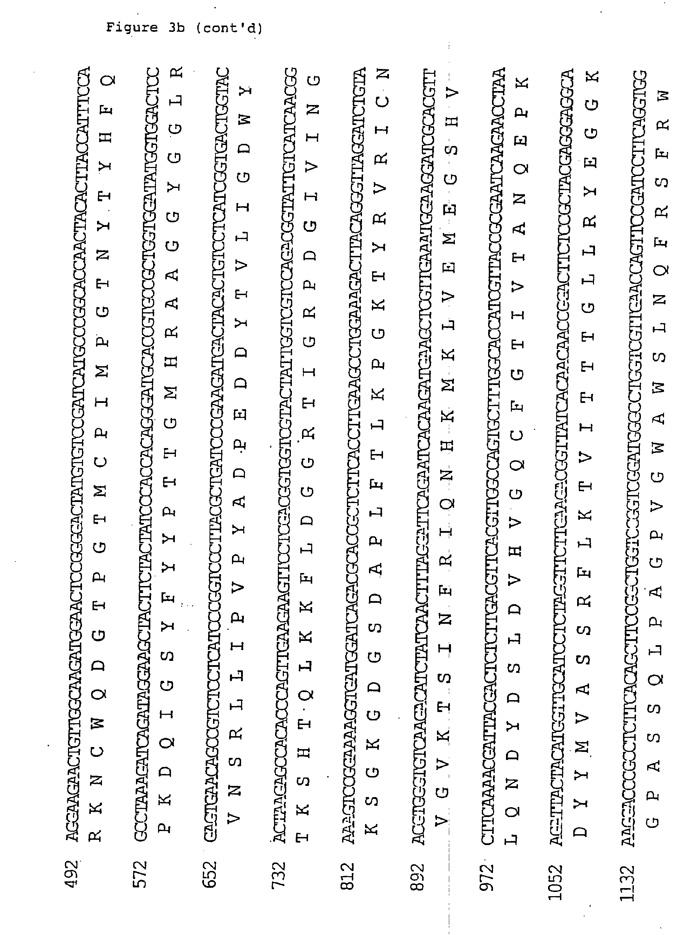

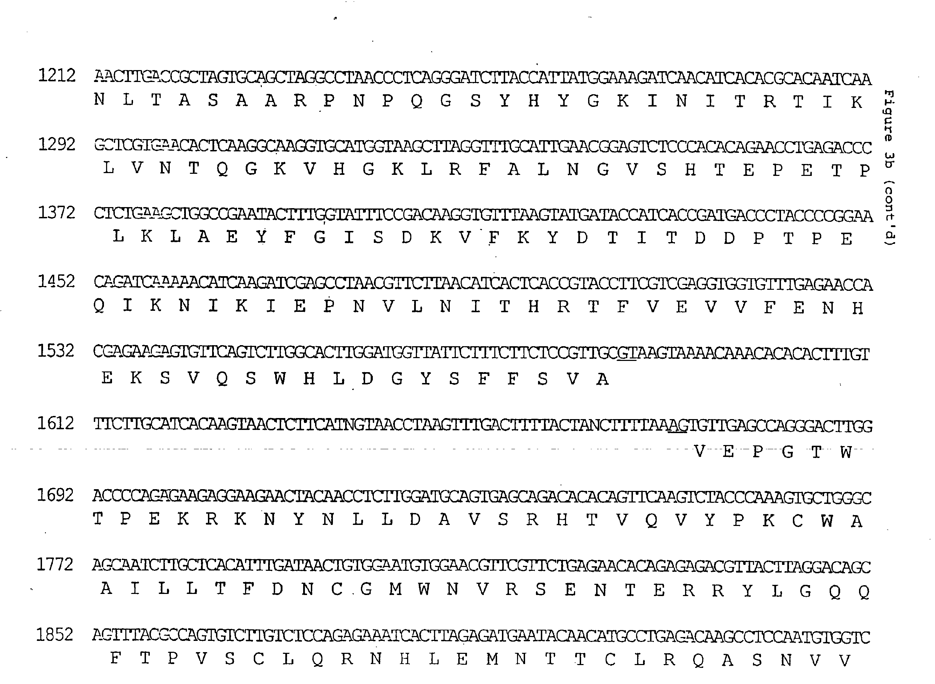

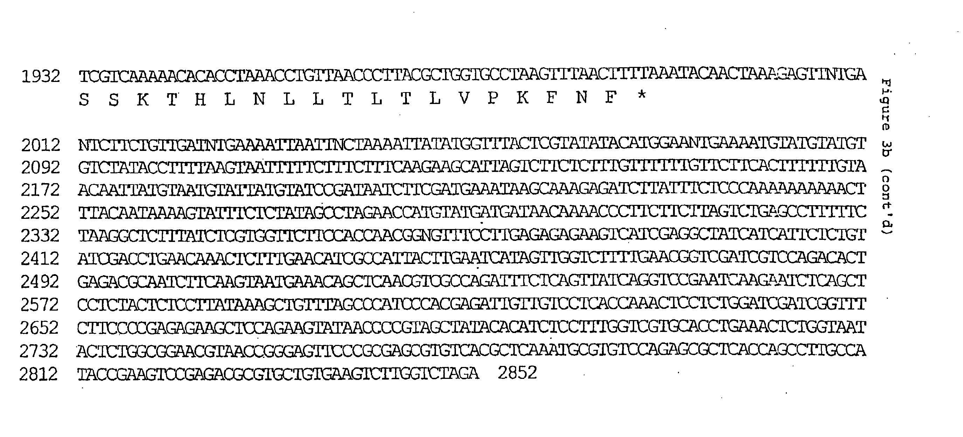

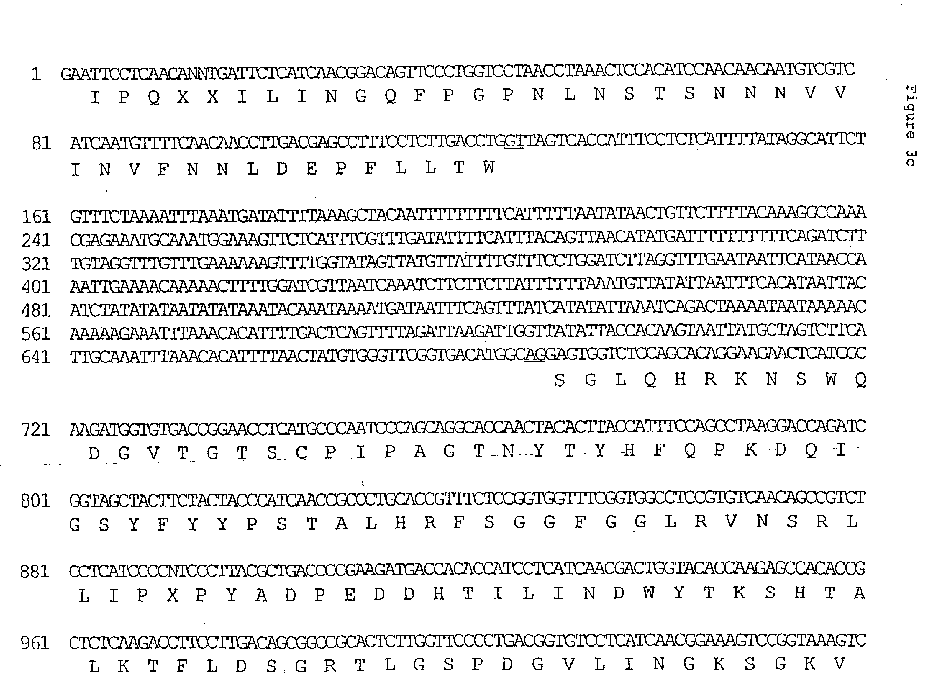

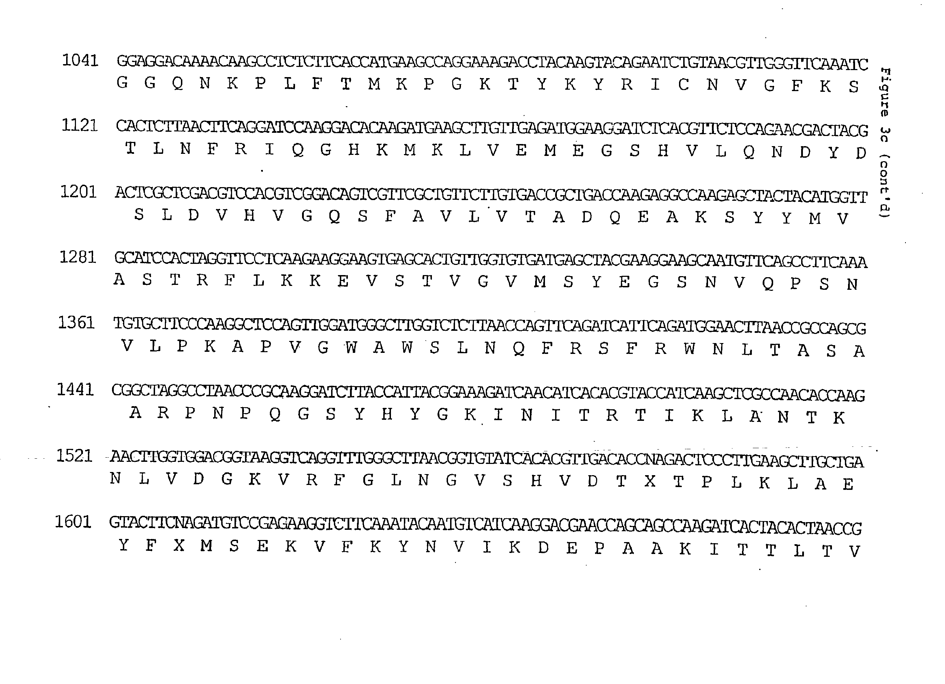

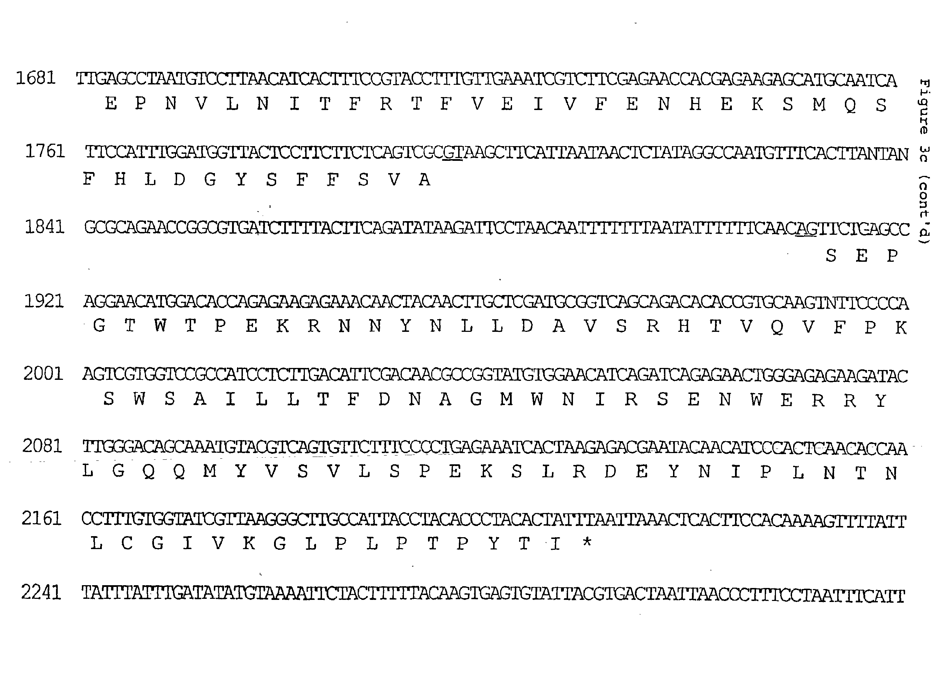

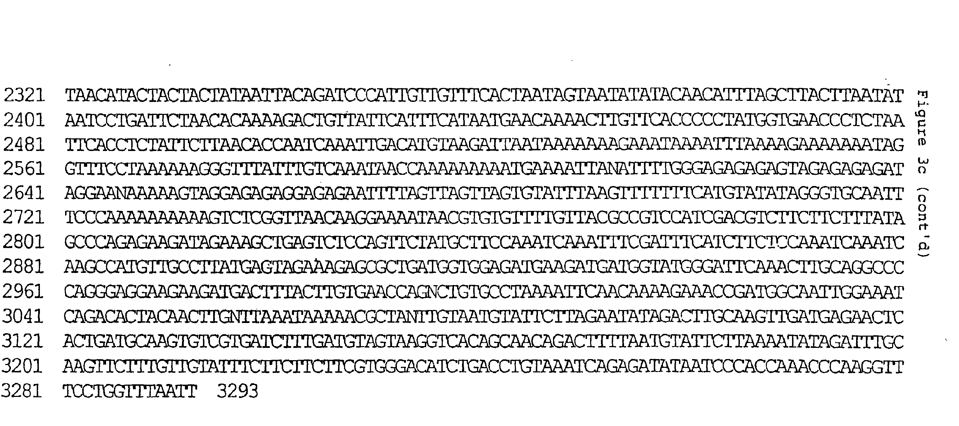

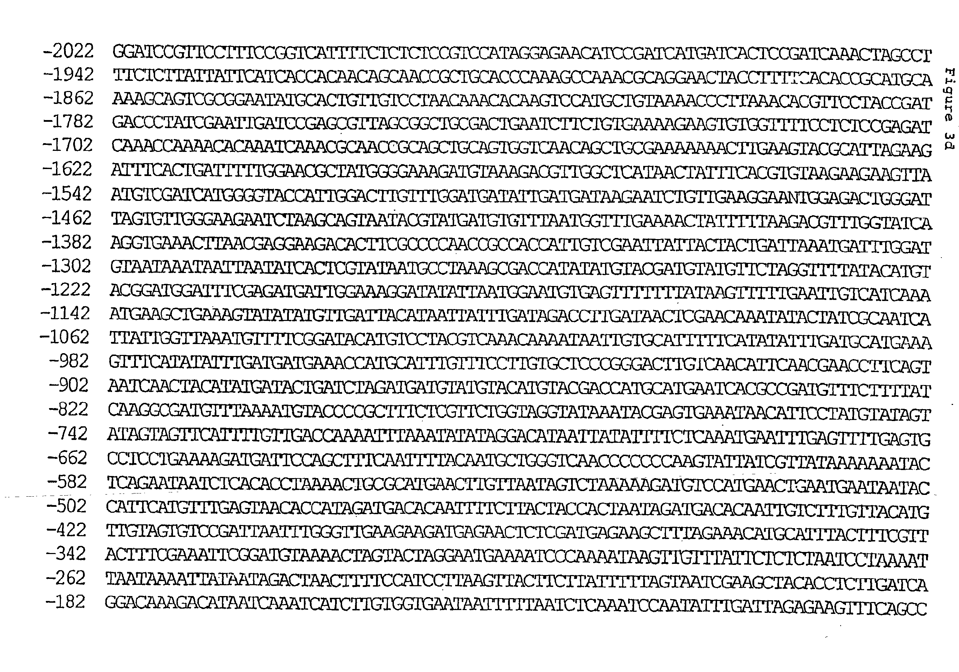

In Figure 3b, the nucleotide sequence of the region of clone L10 demarcated in Figure 2b is shown. The start of transcription is at nucleotide 1. In this sequence the ATG start codon is at nucleotides 45-47, the first exon ends at nucleotide 315 the second exon starts at nucleotide 476 and extends to nucleotide 1586. The third exon starts at 1673 and extends approximately to nucleotide 1989, the precise end of transcription was not determined. The deduced amino acid sequence is also shown. For some promoter constructs the region of the clone 5' to the sequenced portion were used. The specific details of the constructs are listed below.

In Figure 3c, the nucleotide sequence of clone L16 is shown. Clone L16 shows considerable homology to clone L10 specifically in the portions of the two clones that code for protein sequence. The intron sequences between the two clones differ considerably however. Clone L16 does not contain a 5' promoter region and as such was used only as a source of coding sequences for anti-sense RNA constructs. Nucleotide 1 demarcates an Eco RI site that occurs in a coding region of the DNA that is homologous to the first exon of clone L10. By homology, this coding region extends to nucleotide 124, where the first intron is located. This intron, which is located at the same relative position as the first intron of clone 10, is longer than the intron in clone L10 and extends to nucleotide 688. Nucleotide 689 is the start of the second exon and this exon, which shows strong homology to the second exon of clone L10, extends to nucleotide 1793. There is a second intron at this point and this intron extends to nucleotide 1909. The third exon starts at 1910 and extends to aproximately nucleotide 2210. The deduced amino acid sequence is also shown for specific regions of the clone that show considerable homology to clone L10. The precise nucleotide where transcription stops has not been determined.

In Figure 3d the nucleotide sequence of the region of clone L19 demarcated in Figure 2d is shown. The start of transcription is located at position 1 in the sequence. The ATG start codon is at nucleotides 136-138 and the first intron starts at nucleotide 1201. This intron ends at nucleotide 1338 wherein the second exon starts. The end of transcription occurs at approximately nucleotide 2074. The deduced amino acid sequence is also shown.

In Figure 4, the DNA sequences of three cDNA clones that are homologous to the genes contained in the clone L4 are shown. The DNA sequence of these three cDNA clones as well as the sequence of the correctly spliced transcribed regions of genes Bp4A and Bp4C in the genomic clone L4 are aligned, only the nucleotide differences within these clones are shown, nucleotides that are conserved between the sequences are only shown on the upper sequence. The asterisks shown in Fig. 4 mark the 5' end of the cDNA clones of cBp401, cBp405 and cBp408.

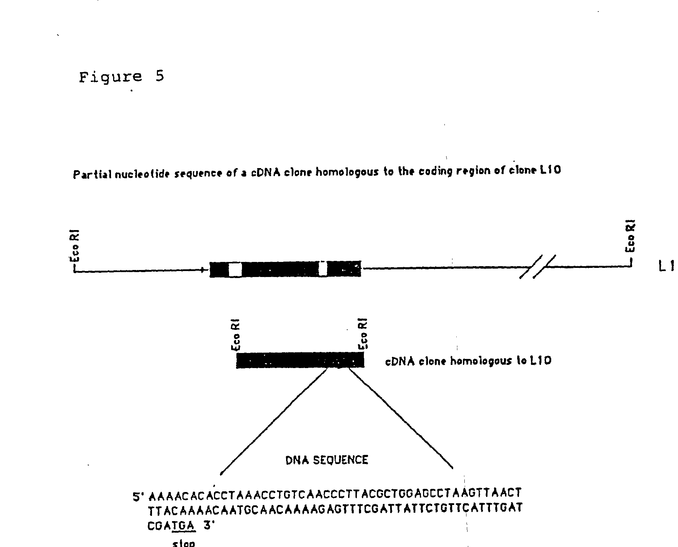

In Figure 5, the partial nucleotide sequence of a cDNA clone that is homologous to the coding region of clone L10 is shown. This cDNA clone is approximately 1.3 Kb in length and has Eco R1 sites at the 5' and 3' ends of the cDNA sequence that were added via synthetic linkers in the cDNA cloning procedure.

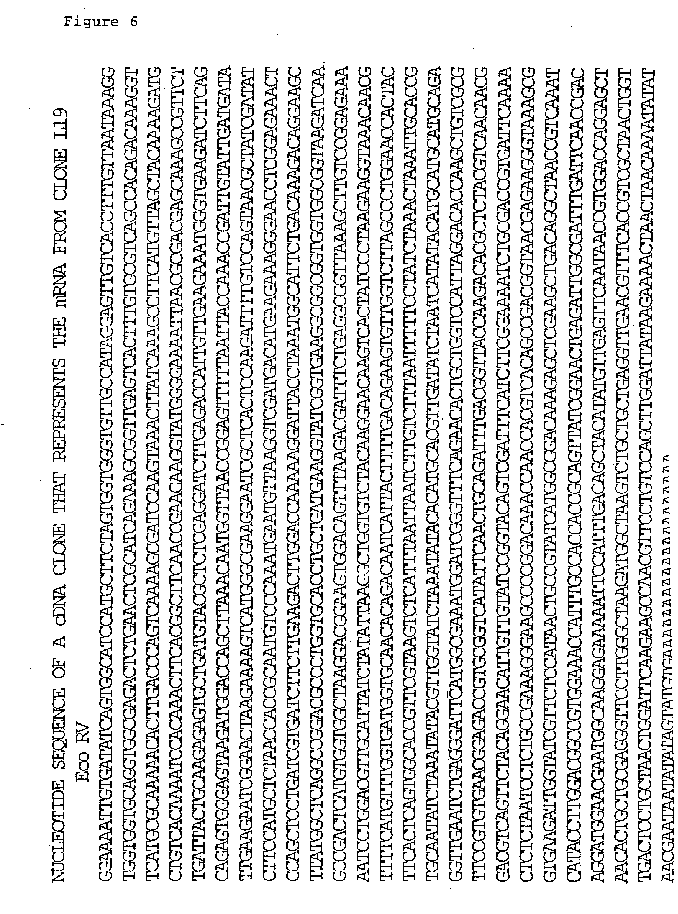

In Figure 6, the nucleotide sequence of the cDNA clone that corresponds to the coding region of clone L19 is shown. Identified in this sequence is the Eco RV site present at the 5' end of the transcribed region of L19. A portion of the poly A tail is shown. Not shown are the Eco RI sites that were added as linkers in the cDNA cloning procedure; these sites are present adjacent to the 5' and 3' ends of the cDNA clone.

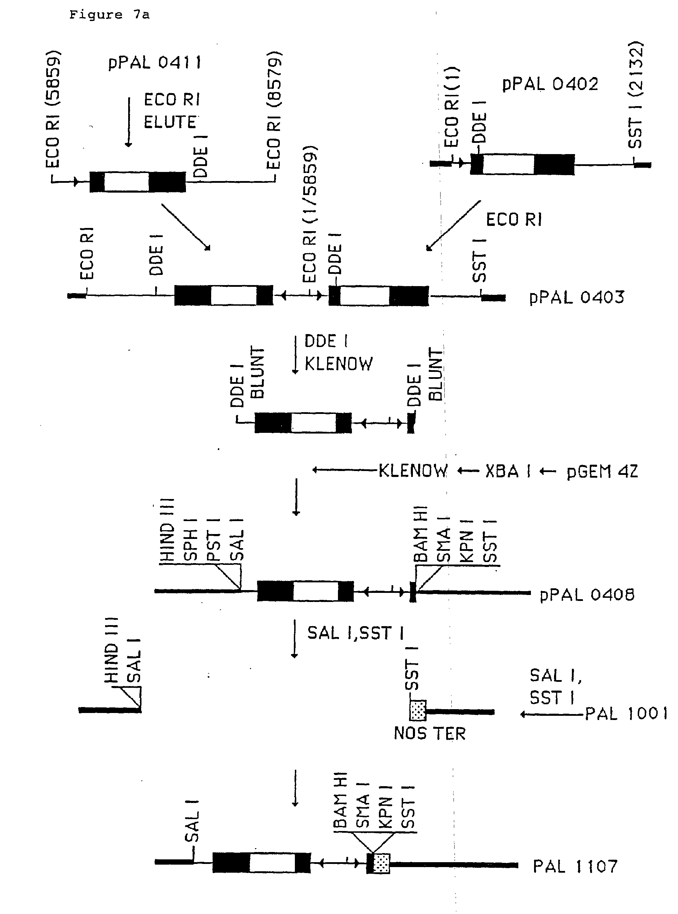

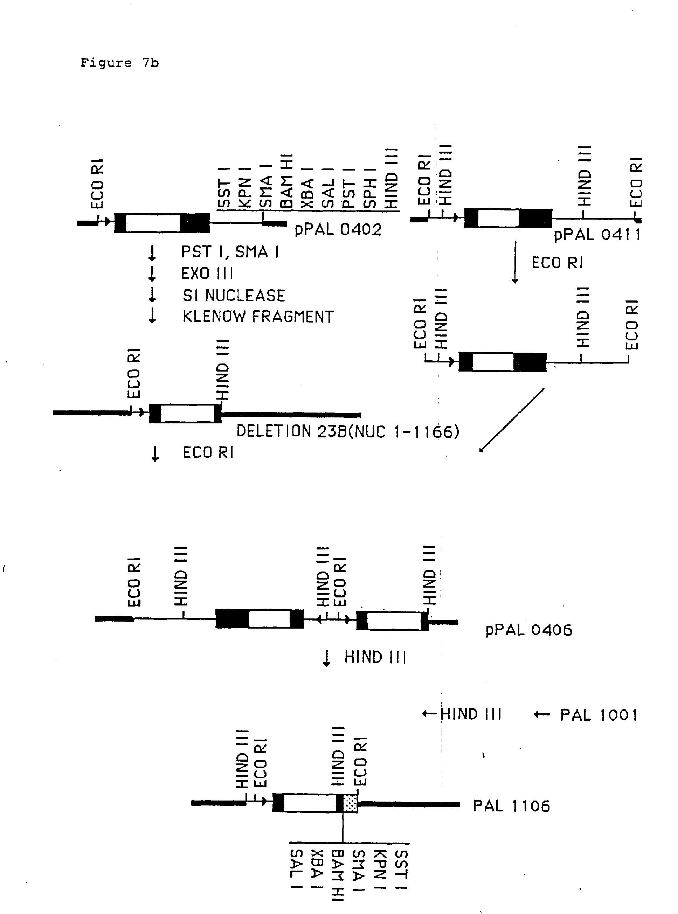

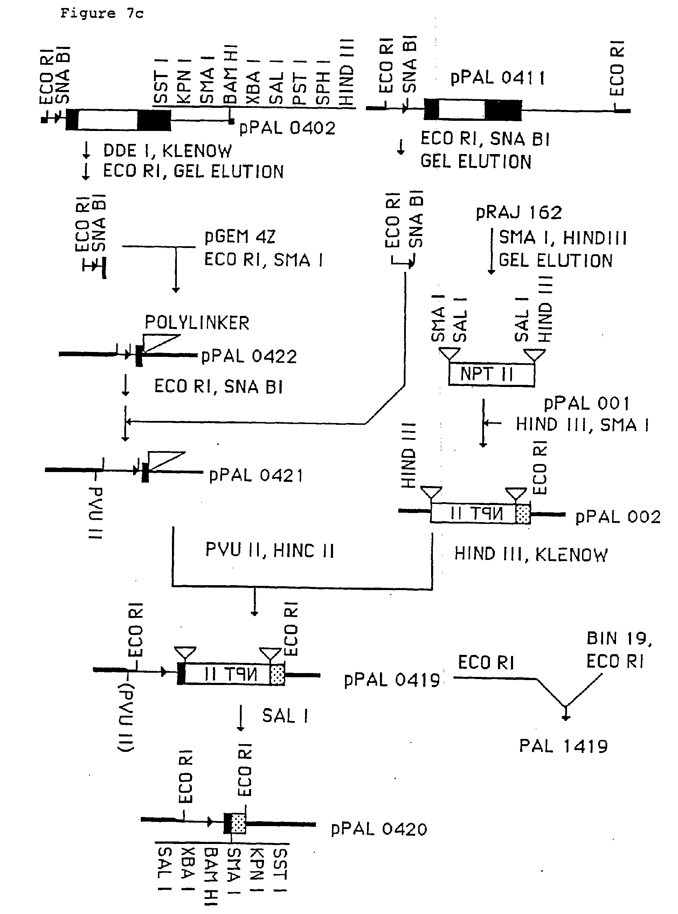

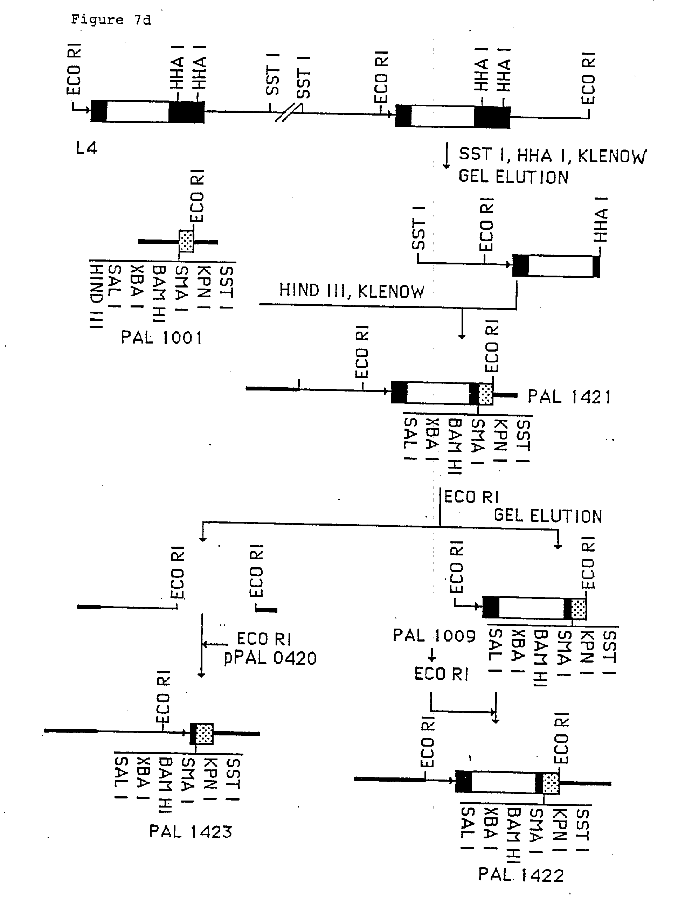

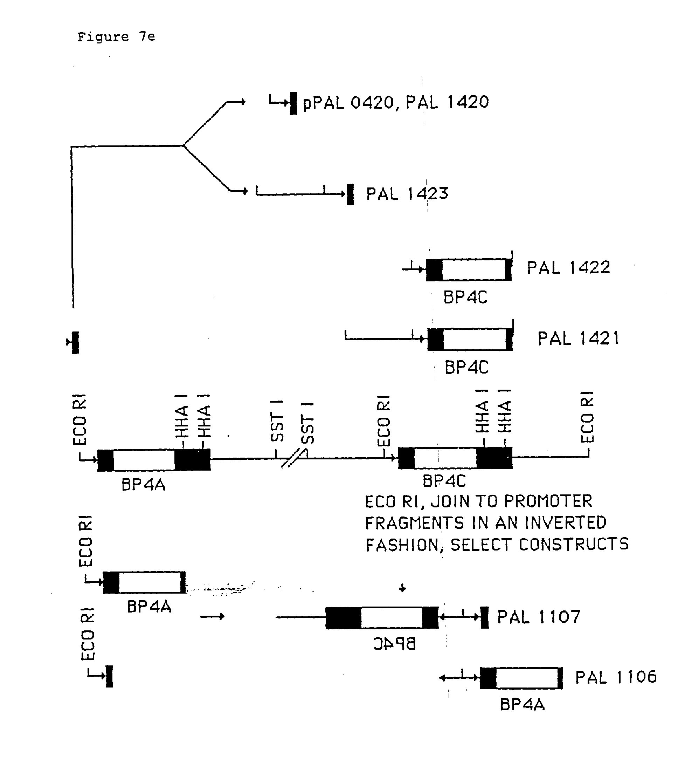

In Figure 7 (a,b,c,d,e) the construction of 6 vectors containing promoter and promoter fragments from the clone L4 is described. The first step in the construction of these vectors was accomplished by first subcloning the Eco R1-Sst 1(nucl.1-2132) fragment containing the first gene of clone L4 (235 base pairs of promoter/exon/intron/second exon) in the commercially available vector pGEM-4Z(Promega Biotech, Madison, WI, USA) using the Eco R1 -Sst 1 sites of the polylinker of this vector. This plasmid was named pPAL 0402. The 2.7Kb Eco RI fragment of clone L4 that contains the third gene (Bp4C) was then cloned into the Eco RI site of pGEM 4Z, leading to a plasmid called pPAL 0411. The plasmid pPAL 0402 was then digested with Eco R1 and the 2.7 Kb Eco R1 fragment from pPAL 0411(nucl. 5859-8579) that contains the gene number three (Bp4C) from clone L4 was added to it. Clones were recovered that contained this inserted 2.7 Kb Eco R1 fragment in both orientations relative to the promoter region of the first gene. A clone that contained this third gene fragment in a orientation such that the promoter from the third gene was opposite to the promoter in the first gene was chosen and called pPAL 0403. The plasmid pPAL 0403 contains the entire third gene from clone L4 oriented in such a fashion as to have the promoter region immediately adjacent to the 235 basepair promoter region of the first gene in pPAL 0403. This plasmid, pPAL 0403 was digested with Dde I, producing a fragment of approximately 1.9 Kb. The Dde I sites are located at nucleotides 303 and 7366. Because of the orientation of these fragments, digestion with Dde I produces a 1.9 Kb fragment. This 1.9 Kb fragment contains a copy of the third gene (Bp4C) oriented such that the direction of transcription of this third gene is from right to left, fused to the 235 base pair promoter fragment from the first gene of clone L4 (Bp4A) which is transcribed from left to right, ending in a Dde I site that is located 67 basepairs down stream of the major start site of transcription and precedes that ATG start of translation codon by 2 nucleotides. This 1.9 Kb Dde I fragment was made blunt with Klenow fragment and cloned into the Xba 1 site of the polylinker region of pGEM 4Z previously made blunt ended with Klenow fragment. The resultant plasmid pPAL 0408, was recovered and subsequently was digested with Sal 1 and Sst 1, which releases the cloned Dde 1 fragment bordered by on the left hand side, (nucl 7366) Sal 1 and on the right hand side (nucl 303) of this construct and contains a portion of the polylinker of pGEM 4Z containing the following unique sites: Bam HI, Sma I, Kpn I, and Sst I restriction enzyme sites. This Sal 1 - Sst 1 fragment was cloned into the Sal 1 - Sst 1 sites of PAL 1001. PAL 1001 is the binary vector Bin 19 (described by Bevan, M., Nucleic Acids Res., 1984, 12:8711-8721) to which has been added the nos ter polyadenylation signal as a 260 bp Sst 1 - Eco R1 fragment isolated from the plasmid pRAJ 221 (available from Clonetech Laboratories, Palo Alto, CA USA) in the Sst 1 - Eco R1 sites of the polylinker region of Bin 19. This nos ter is identified as a stippled box. The binary transformation vector that resulted from the insertion of the Sal I - Sst I fragment of pPAL 0408 into PAL 1001 was named PAL 1107. The details of the construction are shown in Figure 7a. This vector has a copy of the third gene oriented such that the direction of transcription of this third gene is from right to left, fused to the 235 base pair promoter fragment from the first gene of clone L4 which is transcribed from left to right, followed by a polylinker with unique sites for the insertion of DNA which consist of: Bam HI, Sma I, Kpn I and Sst I followed by the nos ter signal. This vector has the feature in that additional 5' non-coding sequences were placed upstream to the 235 base pair core promoter on Bp4A, but these additional 5' sequences were in a opposite orientation. The provision of these sequences in this orientation does not affect the pollen specificity of the core 235 base pair promoter.

In addition to this vector, similarly structured vectors were made which contained essentially the same type of gene promoter arrangement but contained the intron of the first gene (Bp4A) of clone L4. Intron sequences in plant genes have been shown in some cases to play a role in gene expression. This intron containing vector was constructed by making a deletion series of the clone pPAL 0402. pPAL 0402 was first digested with Pst I and Sma I. Exonuclease III was used to unidirectionally digest the DNA as shown (Fig. 7b). After S1 nuclease treatment and repair with Klenow, the plasmid was relegated and clones that have had different portions of the coding regions of gene Bp4A digested out of them were recovered. Deletion subclones were sequenced. One was chosen for vector constructs. This is referred to as deletion 23B. This subclone represented a deletion that has most of the second exon of gene Bp4A removed but contains the intron splice site and first exon of gene Bp4A. This subclone contains a portion of the clone L4 that extends from nucleotide 1 to nucleotide 1166. To this subclone was added the 2.7 Kb Eco R1 fragment from pPAL 0411 that contains the third gene of L4 (Bp4C) in such an orientation that the direction of transcription of the third gene is from right to left (as in PAL 1107, pPAL 0408), fused to the 235 base pair promoter region from the first gene of clone L4 which is oriented to transcribe from left to right followed by the first exon of gene 1, the entire intron of gene 1 and 33 nucleotides of the second exon of gene Bp4A from clone L4. This plasmid containing deletion 23B and the 2.7 Kb Eco RI fragment containing the third gene fragment was named pPAL 0406. This. plasmid was digested with Hind III, which yields a fragment containing a small portion of the promoter of the third gene as well as the entire promoter of the first gene, first exon, intron and a portion of the second exon. This Hind III fragment was inserted into the Hind III site of PAL 1001, resulting in the vector PAL 1106 (deletion 23B derived). This vector has in the following order, A portion of the promoter from the third gene in clone L4, the entire 235 base pair promoter of the first gene in clone L4, followed by the first exon, the intron and a portion of the second exon of gene 1 of clone L4, followed by a polylinker containing the following unique cloning sites: Sal I, Xba I, Bam HI, Sma I, Kpn I and Sst I and the nos ter polyadenylation signal. The construct is shown in Figure 7b.

Additional constructs with the promoter regions of the genes contained in clone L4 were done in order to provide a number of suitable vectors that are useful for pollen specific expression of gene sequences. The three genes within clone L4 (Bp4A, Bp4B, Bp4C) show very near-exact DNA homology and this is most apparent between the first (Bp4A) and third (Bp4C) gene. The second gene (Bp4B) is a homologous copy that has undergone sequence changes that have appear to have lead to inactivation. The extensive similarity between the first, second and third genes in clone L4 is also maintained in the promoter region such that out of the first 235 nucleotides of the first and third gene promoter regions there are only 5 nucleotides that differ between them. Downstream of the TATA box in these two promoters the only difference between them is the presence of one additional nucleotide at the start of transcription. For example, comparison of Promoter 1, Bp4A, partially represented as: ...TATGTTTtAAAA ... with Promoter 3,Bp4C, partially represented as: .....TATGTTTAAAA.... shows that the transcribed region underlined and the single nucleotide difference in lower case. However, within the sequence of the first gene there is a nucleotide change that introduces a Dde I site (nucl 303) in the untranslated 5' leader sequence upstream of the ATG start codon that is not present in the untranscribed leader sequence of the third gene in clone L4. Chimeric promoter constructs were made which utilized this Dde I site in the first gene to combine with sequences from the third gene promoter. The region of the first promoter used for these constructs consisted of the sequences contained between the Sna BI site (nucl 21) near the TATA box to the Dde I site located immediately upstream of the ATG start codon in the first gene (nucleotide 303 is the first nucleotide in the recognition sequence for Dde I). The other region of this chimeric promoter (5' of the TATA box) was a fragment extending from the Eco R1 site of the third promoter (nucleotide 5858) to the Sna B1 site near the TATA box (nucleotide 6273). Therefore to facilitate construction of these pollen specific vectors, the following reconstructions were performed.

The Eco R1 to Dde 1 fragment that encompasses the promoter region of the first gene in clone L4 was isolated by first cutting pPAL 0402 with Dde 1, blunting with Klenow, and then cutting with Eco R1. The 235 base pair fragment corresponding to this region was cloned into the Eco R1 - Sma 1 sites of pGEM 4Z. This plasmid (pPAL 0422), was then cut with Eco R1 and Sna B1. A DNA fragment that contained the Eco RI to Sna BI portion of the promoter for gene 3 in clone L4 was isolated by digesting pPAL 0411 with Eco R1 and Sna B1. This released an approximately 415 base pair Eco RI (nucl.5858) to Sna BI (nucl. 6273) fragment that represents most of the 5' region of the gene 3 promoter from clone L4 (the Sna B1 recognition site is 2 base pairs downstream of the TATA box). This Eco R1 - Sna B1 fragment was used to replace the shorter Eco R1 - Sna B1 fragment removed for the first promoter subclone (pPAL 0422), reconstructing a promoter fragment of approximately 550 base pairs. This plasmid is referred to as pPAL 0421. This chimeric promoter fragment contains 415 base pairs of the promoter of gene three in clone L 4, followed by approximately 99 Nucleotides of the first gene promoter/untranslated leader sequence.

For construction of a pollen specific cassette vector, the following plasmids were first constructed. The first plasmid constructed contained the nos ter polyadenylation signal with a polylinker in front of the nos ter signal. This was accomplished by first isolating from pRAJ 221 the nos ter as a Sst 1 - Eco R1 fragment and this fragment was cloned in pGEM 4Z using the Sst 1 and Eco R1 sites in the polylinker. This subcloned is referred to as pPAL 001. To pPAL 001, a fragment coding for neomycin phosphotransferase (NPT II) derived from the plasmid pRAJ 162 was added to it in the anti-sense orientation as follows: The plasmid pRAJ 162 contains the NPT II gene from the transposon TN 5 inserted as a Sal I fragment and bounded by a polylinker in the plasmid pUC-9 (which was obtained from the Plant Breeding Institute, Cambridge, UK). pRAJ 162 was digested with Hind III and Sma I. The DNA fragment containing the NPT II gene was isolated by elution from an agarose gel. pPAL 001 was digested with Hind III and Sma I and the NPT II gene fragment was inserted. The resultant plasmid was called pPAL 002 and had such orientation of restriction sites and the NPT II gene and nos ter as follows: HIND III, Pst I, Sal I, 3' end NPT II coding sequence 5'end, Sal I, Bam HI, Sma I, Kpn I, Sst I, nos ter, Eco RI. pPAL 002 was cut with Hind III and the site made blunt ended by the use of Klenow fragment. pPAL 0421 was digested with Hinc II and Pvu II, both of which leave blunt ends, and the promoter fragment was ligated into Hind III cut blunt ended pPAL 002. Plasmids were obtained that contained the promoter in both orientations relative to the nos ter signal. One plasmid was chosen with the proper orientation (5' promoter/anti-sense NPT II/nos ter) and was named pPAL 0419. pPAL 0419 has the following DNA fragments: A small (approx. 130 bp) of pGEM 4Z that contains the SP6 promoter, the 550 base pair chimeric promoter, the NPT II gene in the anti-sense orientation relative to the promoter, followed by the nos ter polyadenylation signal. This entire promoter/NPT II/nos ter construct is excisable by Eco RI. pPAL 0419 was digested with Eco RI, and the promoter NPT II nos ter structure was cloned into BIN 19 using the single Eco RI site in the polylinker of BIN 19. The resultant transformation vector was named PAL 1419. In addition to the anti-sense NPT II gene, the vector contains a constitutive NPT II gene under the control of the nos promoter. This vector therefore confers resistance to kanamycin in all cell types with the exception of pollen cells where the gene expression from the constitutive promoter is inhibited by the anti-sense RNA produced from the promoter/NPT II/nos ter construct contained in PAL 1419.

In order to provide promoter sequences that could be utilized with additional gene constructs, the plasmid pPAL 0419 was digested with Sal I. This digest removes the NPT II coding region and this Sal I digested pPAL 0149 was relegated giving rise to pPAL 0420. pPAL 0420 represents the pollen specific promoter followed by a polylinker for insertion of genes that has the following unique sites: Hinc II, Pst I, Sal I, Bam HI, Sma I, Kpn I, Sst I, followed by the nos ter polyadenylation signal. The entire promoter/polylinker/nos ter construct can be conveniently excised as a single Eco RI fragment. The details of this construct is shown in Figure 7c.

For additional pollen specific promoter constructs, the following approach was used. The intact L4 clone in the lambda cloning vector was digested to completion with the restriction enzymes Sst I and Hha I. The resultant fragments were separated by gel electrophoresis and a 2.65 Kb fragment that contains the promoter/first exon/intron/partial second exon region of gene three in clone L4 and corresponds to nucleotides 4565 to 7216 in the sequence of clone L4 was isolated. This fragment was made blunt ended with Klenow and cloned into the binary transformation vector PAL 1001 previously described. PAL 1001 was first cut with Hind III and made blunt ended with Klenow. Clones containing this fragment (promoter/first exon/intron/partial second exon) were recovered. A clone was chosen that contained this fragment in the proper orientation such that the direction of transcription was towards the nos ter in PAL 1001. This vector was named PAL 1421. This vector contains approximately 1.9kb of upstream promoter region from the gene 3 in clone L4 followed by the first exon, the complete intron and 15 bases of the second exon of gene three followed by a polylinker containing the following unique sites: Sal I, Xba I, Bam HI, Sma I, Kpn I, SstI, and finally the nos ter polyadenylation signal. A variant of this vector was constructed by digesting PAL 1421 with Eco RI and isolating the fragment from this clone that contains the promoter polylinker nos ter sequences but contained less of the upstream region of the promoter. This fragment was re-cloned into PAL 1009. PAL 1009 is a BIN 19 derived vector from which most of the polylinker has been removed. This vector was constructed by digesting BIN 19 with Hind III and Sst I, making these sites blunt ended with Klenow and relegating such that a vector was recovered that contained a single unique Eco RI site for the insertion of fragments. PAL 1009 was digested with Eco RI and the Eco RI fragment from PAL 1421 that contains a shorter promoter/exon/intron/second exon/polylinker/nos ter structure was 'added to it. This gave rise to the vector PAL 1422, a vector that is essentially the same as PAL 1421 with the exception that there is less 5' promoter region. It should be noted that both PAL 1421 and PAL 1422 contain the intron from the third gene. For constructs which the presence of the intron may not be desired, intron sequences were removed from PAL 1421 by first digesting PAL 1421 with Eco RI and replacing the promoter/exon/intron/second exon/polylinker/noster structure with the promoter/polylinker/nos ter structure from PPAL 0420 using Eco R1 such that a longer 5' promoter region is reconstructed in the binary transformation vector. The resultant vector was named PAL 1423. The outline of this construction is shown in Figure 7d.

In Figure 7e, a schematic diagram of the relationship of the above described vectors is presented. It should be noted that the vectors outlined in this Figure fall into three categories: 1, vectors which contain 5' upstream promoter regions that are substantially derived from the upstream region of the gene Bp4C (pPAL 0420, PAL 1420, PAL 1423), 2, promoter constructs that contain 5' upstream promoter regions and intron sequences from the gene Bp4C (PAL 1422, PAL 1421) and, 3, promoters which contain a chimeric 5' upstream region in which a portion of the 5' DNA sequence is inverted relative to the arrangement which appears in the genomic clone and uses the promoter fragment of Bp4A as a core promoter structure (PAL 1107, PAL 1106). It should be noted that the functioning of each of these constructs can vary from plant species to plant species and it may be desirable to test a number of these promoter constructs when carrying out certain aspects of this invention.

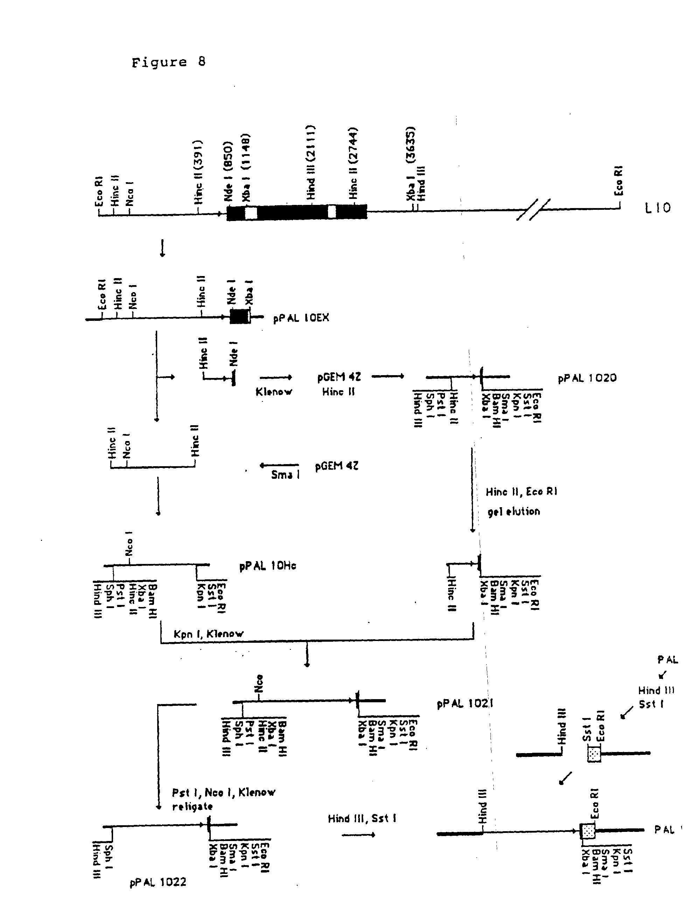

The construction of pollen specific vectors that utilize the promoter regions of clones L10 and L19 was conducted as follows. The construction of the pollen specific vectors depicted in Figure 8 utilizes promoter regions from clone L10. The start of transcription of clone L10 is located at nucleotide 1. The ATG start codon is located at nucleotides 45-47. The promoter region of this clone was excised by first subcloning the Eco RI - Xba I fragment of the clone that encompasses the entire promoter region and a portion of the first exon (the Xba I site is nucleotide 358 in the DNA sequence). This subclone (pPAL 10EX) was then digested with Hinc II and Nde I. The Nde I site is located immediately upstream of the ATG start codon at nucleotide 60 and the Hinc II site is located at nucleotide number -399. The digestion with these two enzymes releases a DNA fragment of 459 nucleotides which contains 62 nucleotides of untranslated transcribed leader sequence, and 397 nucleotides of 5' promoter region. The Nde I site in this fragment was made blunt ended by the use of Klenow, and this fragment was subcloned into the Hinc II site of the polylinker of pGEM 4Z. Clones were recovered in both orientations and the clone that contained the fragment in the orientation: Hind III, Sph I, Pst I. Hinc II, promoter-62 base pair leader fragment (Nde I blunt/Hinc II, does not cut with either Hinc II or Nde I) Xba I, Bam HI, Sma I, Kpn I, Sst I, Eco RI was chosen and named pPAL 1020. To add additional upstream regions, the Hinc II-HincII fragment that is approximately 1 Kb in length and is immediately upstream of the Hinc II site at position 391 in the DNA sequence was isolated from pPAL 10EX by digestion with Hinc II and gel elution of this fragment. This Hinc II fragment was cloned into the Sma I site of pGEM 4Z. Clones which contained the fragment in both orientations were recovered and a clone that contained the fragment in the following orientation was chosen: Hind III, Sph I, Pst I, Hinc II, Sal I, Xba I, Bam HI, the Hinc II fragment in the same orientation as in the genomic clone, that being right to left, 5'-3' (as a Hinc II/Sma I insertion which does not cut with either enzyme), Kpn I, Sst I, Eco RI. This subclone (pPAL10Hc) was digested with Knp I, made blunt end by the use of Klenow, then digested with Eco RI. To this cut subclone was added the promoter/untranslated leader sequence of pPAL 1020 by digesting pPAL 1020 with Hinc II and Eco RI, and adding this promoter fragment to the cut pPAL 10Hc. The resultant subclone contained a reconstructed promoter region of clone L10 differing from the intact region by only the filled in Kpn I site used for the joining of the two promoter fragments. This construct was named pPAL 1021. This vector contains in the following order: Hind III, Pst I, Sph I, Hinc II, Sal I, Xba I, Bam HI, the approximately 1 Kb Hinc II fragment joined to the Hinc II-Nde I promoter fragment followed by Xba I, Bam HI, Sma I, Kpn I, Sst I, and Eco RI. This subclone allows for the convenient removal of the promoter region of clone L10 such that the promoter can be easily used in cassette transformation vectors. The outline of this construction is shown in figure 8. The promoter region of pPAL 1021 was used for the construction of a pollen specific cassette transformation vector by carrying out the following constructs: The plasmid pPAL 1021 was digested with Nco I and Pst I. The plasmid was treated with Klenow and religated. This procedure effectively removed the portion of the polylinker that was 5' to the promoter in pPAL 1021. This plasmid was then digested with Hind III and Sst I, and cloned into the Hind III and Sst I sites of PAL 1001, giving rise to PAL 1121. PAL 1121 has in the following order: the pollen specific promoter of clone L10 (approximately 1.1-1.2 Kb), followed by a polylinker with the following unique sites: Xba I, Bam HI, Sma I, Kpn I, Sst I, followed by the nos ter. The construction of this is outlined in Figure 8.

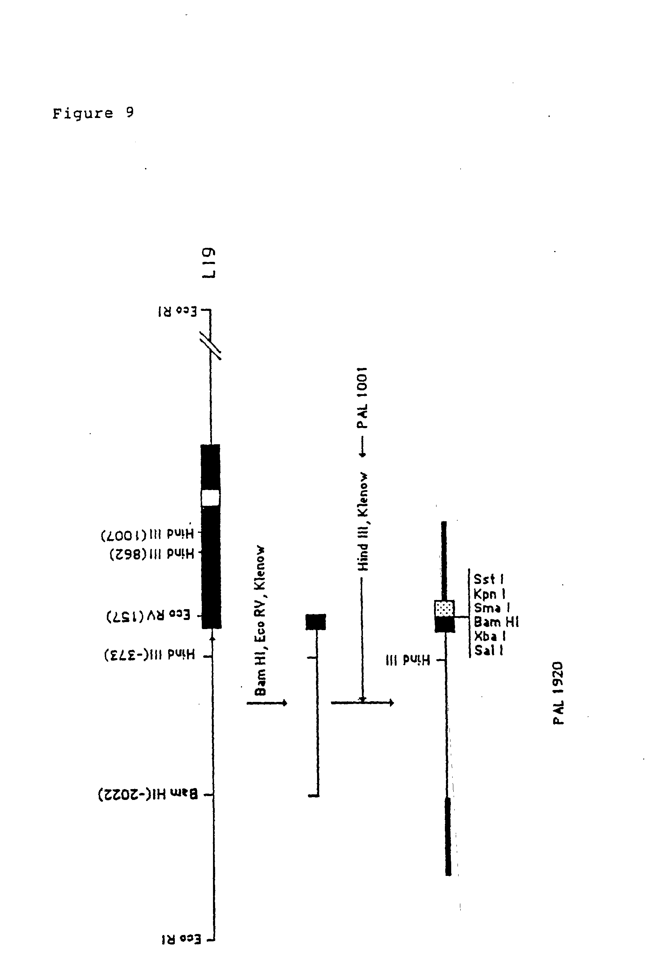

The promoter region of the clone L19 was also used for construction of pollen specific vectors. The construction of these vectors is as shown in Figure 9. Clone L19 has a single pollen specific gene contained with it. The start of transcription in this gene is located at position 1 in the DNA sequence. The ATG start codon is located at nucleotide position 136-138. The only intron is located at nucleotides 1202-1387, the stop translation codon is located at nucleotides 2024-2026. The end of transcription is located at approximately nucleotide 2074. The entire Eco RI fragment of this clone was subcloned into PGEM 4Z by using the Eco RI site located in the polylinker. The resultant clone was named pPAL 1901. The promoter region of this clone was excised as a single fragment by digesting pPAL 1901 with Bam HI and Eco RV, and a 2177 basepair fragment corresponding to the promoter region was isolated. This fragment covers from nucleotide -2200 (Bam HI) to nucleotide 156 (Eco RV). This promoter fragment contains over 2Kb of 5' upstream region of the promoter in clone L19, 134 basepairs of 5' untranslated leader sequence and 20 basepairs of translated sequence. The Bam HI site in this fragment was made blunt ended by the use of Klenow and cloned into PAL 1001. This step was accomplished by cutting PAL 1001 with Hind III, making this site blunt ended by the use of Klenow and inserting the blunt ended Bam HI - Eco RV fragment in such an orientation that the promoter was oriented 5' to 3' with respect to the polylinker/nos ter polyadenylation signal. This vector was named PAL 1920 and contained within it in the following order: The promoter from clone L19 containing 134 base pairs of 5' untranslated leader sequence, 20 base pairs of translated sequence fused to a polyliker containing a former Hind III site inactivated by blunt ending, Sph I, Pst I, Sal I, Hinc II, Xba I, Bam HI, Sma I, Kpn I, Sst I (the unique cloning sites are underlined), the nos ter polyadenylation signal. This vector is convenient for the insertion of DNA sequences to be transcribed in pollen cells. The outline of this construct is shown in Figure 9.

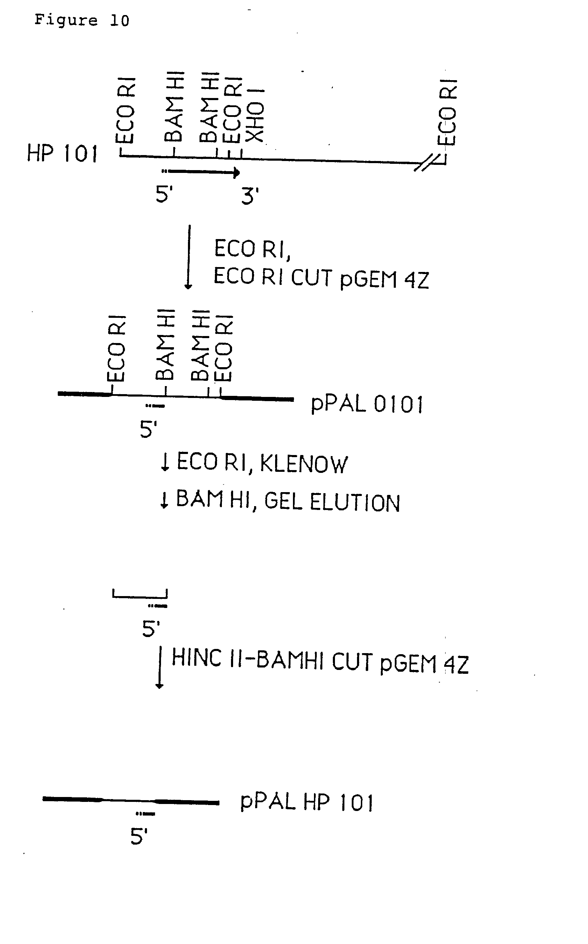

In Figure 10, the restriction may of a Brassica napus genomic clone (HP 101) that contains a constitutively expressed gene is shown and the fragment of this clone that contains a 5' promoter region along with a portion of transcribed sequence is identified. This fragment was isolated by first cloning the small 2.5 kb Eco RI fragment in pGEM 4Z, and obtaining a subclone that had this fragment inserted in the indicated orientation relative to the polylinker of pGEM 4Z. This clone was called pPAL 0101 and was deposited January 26, 1990 with the American Type Culture Collection (ATCC), 12301 Parklawn Drive, Rockville, MD, 20852, USA as pPAL0101/E. coli strain DH5 alpha under acession number ATCC 68210. This E. coli strain grows on standard E. coli media (LB) with 100 micrograms per ml of ampicillin. This subclone, pPAL 0101, was then digested with Eco RI, treated with Klenow fragment, then digested with Bam HI, which releases the promoter/transcribed region indicated. This fragment was cloned into Hinc II - Bam HI cut pGEM 4Z, resulting in the subclone pPAL HP101. The subclone can be used for the isolation of promoter sequences in vector constructs that utilize a constitutive promoter to synthesize pollen specific anti-sense RNA.

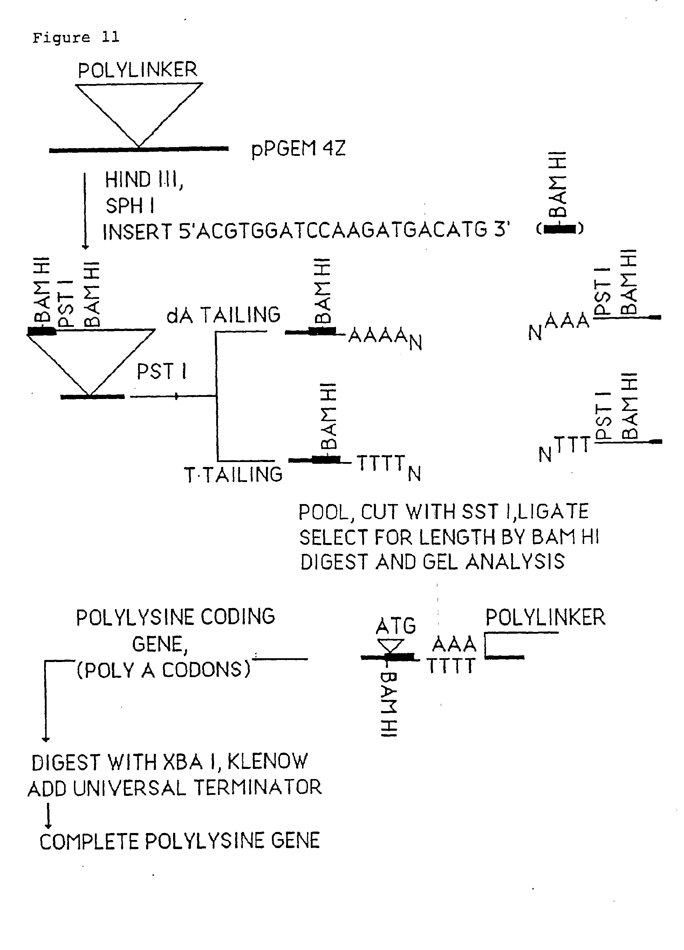

In Figure 11 a schematic representation of producing a polylysine coding gene is shown. In this construct, the cloning vector pGEM 4Z was used as a recipient of a synthetic oligonucleotide containing a ATG start codon and to this was added a polynucleotide consisting of solely one nucleotide. This gene therefore, depending on the nucleotide used is a gene that has predominantly one codon, and codes for a protein that is composed of a polyamino acid. The construct was carried out in the following fashion: To provide a ATG start codon in a favourable initiation context, a synthetic oligonucleotide was constructed and inserted into pGEM 4Z between the Hind III and Sph I sites. This nucleotide had the sequence: 5'-ACGTGGATCCAAGATGACATG-3'. The resultant subclone therefore had the DNA sequence (restriction site for a introduced Bam HI site is underlined, the ATG start codon in bold) AACGT GGATCC AAG ATG ACA TGC GCA ACA TGG at the 5' region such that there was a ATG start codon in a favourable initiation context and a Bam HI site upstream of this site for excision of the coding sequence. This subclone was digested with Pst I, divided into two aliquots, one was tailed with T residues using terminal transferase and TTP, one was tailed with A residues using terminal transferase and dATP. The two tailed plasmids were mixed together, extracted with phenol-chloroform, ethanol precipitated and resuspended. The plasmid mixture was cut with Sst I, and relegated. Clones that were recovered were either clones that contained on the coding strand polyA or poly T. Clones were cut with Bam HI, the size of the insert determined by gel electrophoresis, and sequenced to determine if the clone coded for poly-lysine (polyA) or poly-phenylalanine (poly T, poly U in the mRNA). A clone that coded for poly-lysine and was approximately 300 nucleotides was chosen and called pPAL pLys. This clone was cut with Sma I, and a universal translation terminator (available from Pharmacia PL Biochemicals, Montreal, Canada) was added to it, completing the construction of the gene. This construction is shown in figure 10.

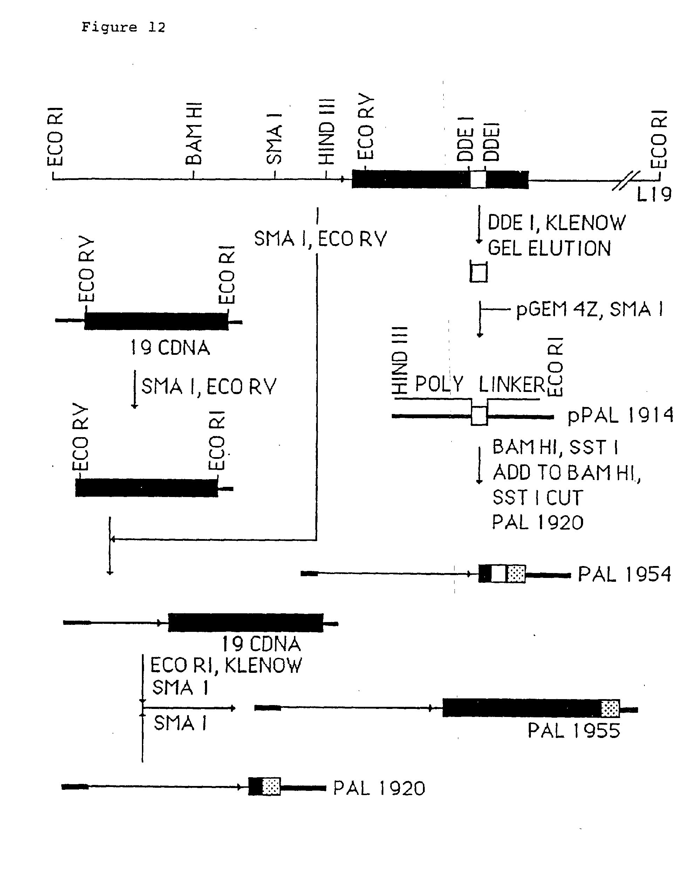

In Figure 12 a scheme for the production of an anti-sense gene specific to the intron region of clone L19 and the production of an intronless version of the number L19 gene is shown. For the first step in this, a restriction fragment from clone L19 was isolated that is substantially all intron. This fragment was isolated by using the restriction enzyme Dde I which cuts at a number of sites in the genomic clone, but the sites at nucleotides 1186 and 1348 give rise to a restriction fragment that is substantially intron sequences, having only approximately 16 nucleotides at the 5' side of the intron that are included in the final transcript, and 10 nucleotides at the 3' side of the intron that are included in the final transcript. This Dde I fragment was isolated by gel electrophoresis, made blunt ended and cloned into Sma I cut pGEM 4Z. Clones in both orientations were obtained, and the clone that contained the intron region in the orientation: Hind III, Sph I, Pst I, Sal I, Xba I, Bam HI (former Sma I) 3' end of the intron, intron sequences, 5' end of the intron, (former Sma I), Kpn I, Sst I, Eco RI was chosen. This clone was named pPAL 1914 and was digested with Bam HI and Sst I, and inserted into PAL 1920 previously cut with Bam HI and Sst I, creating the vector PAL 1954.

To create a specific restorer vector, the cDNA clone 19 cDNA (figure 5) was fused to the promoter region as follows: The cDNA clone was digested with Eco RV and Sma I as shown in Figure 11. To this cut vector was added the Eco RV - Sma I fragment from clone L19. Clones were recovered that contained the reconstructed 5' region of the promoter and coding sequence, but carried coding sequences that lacked the intron, most of the coding regions being derived from the cDNA clone. This clone was digested with Eco RI, made blunt ended with Klenow, then digested with Sma I. A DNA fragment that represents the entire coding region and a portion of the promoter region was isolated and cloned into PAL 1920 cut with Sma I, leading to a reconstruction of the promoter region and coding region lacking an intron. This vector was called PAL 1955.

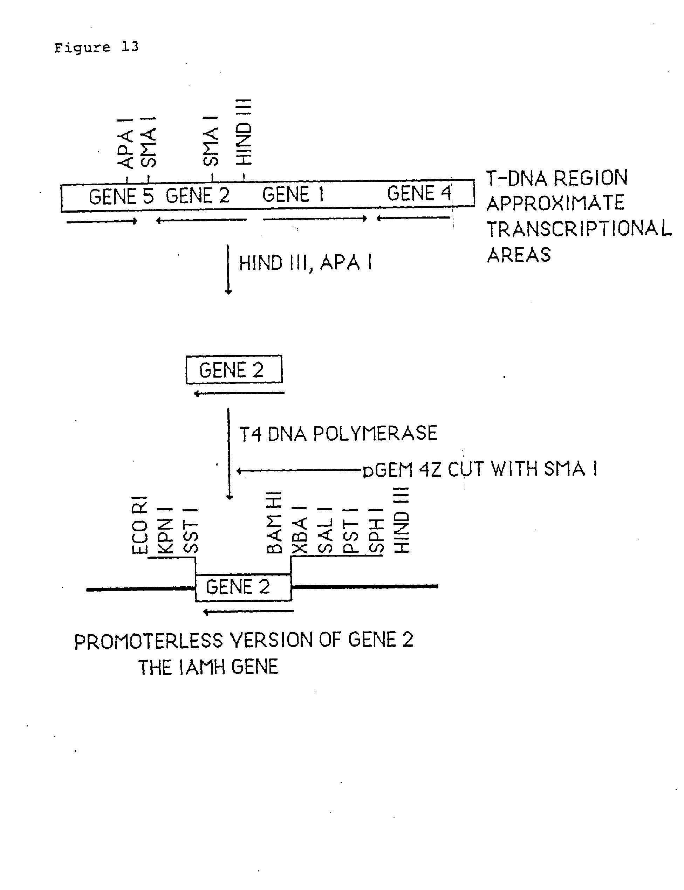

In Figure 13 the IamH gene of the plasmid pTiC58 of Agrobacterium tumefaciens was obtained by isolating the gene 2 region from the plasmid pPCV311 (Koncz,C. and Schell, J., Molecular and General Genetics, (1986), 204:383-396). This region was isolated by digestion with Hind III and Apa I, giving rise to a gene fragment that contains DNA sequence approximately 47 nucleotides upstream of the ATG start codon and contains approximately 500 nucleotides of DNA sequence past the TAA stop codon. This fragment was converted to blunt end by the use of T4 DNA polymerase I, and inserted into Sma I cut pGEM 4Z. Clones were chosen that had the gene in the orientation shown and one of these was called pPAL IamH. This clone was digested with Bam HI and Sst I, and inserted into Barn HI - Sst I cut PAL 1402, giving rise to PAL 1424.

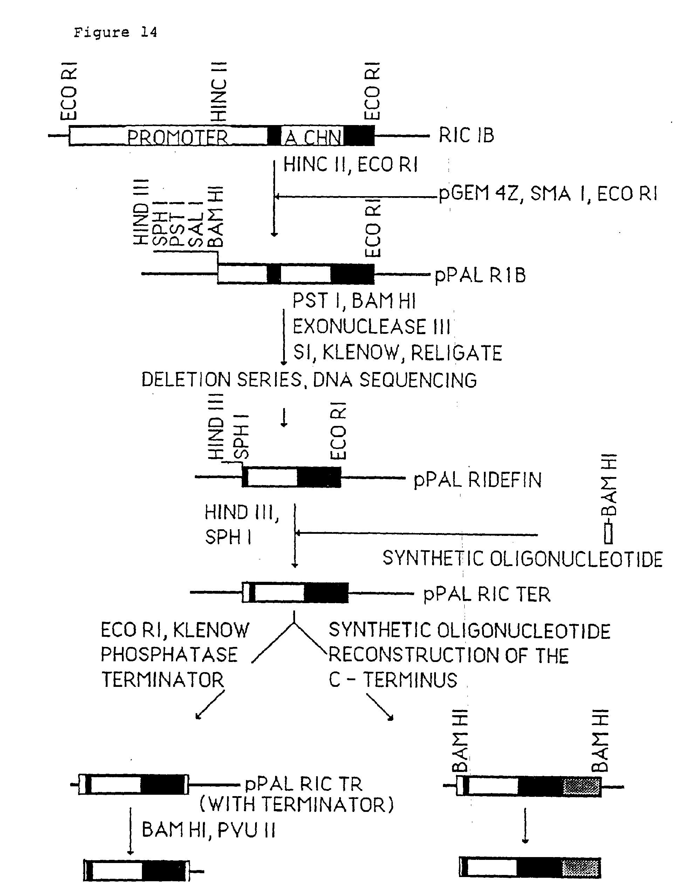

In figure 14, the production of clones containing the coding sequences of a protein functionally related to the ricin A chain protein isolated from Ricinus communis is detailed. This was accomplished by first isolating a genomic clone homologous to ricin from a genomic library of Ricinus zanzabarenis DNA constructed in the vector lambda gt10 using standard protocols. The library was screened with a DNA probe that corresponds to the N-terminal leader sequence of the ricin gene. The probe sequence was obtained from the published sequence of a Ricinus communis ricin gene (Hailing, et al., Nucl. Acids Res 13:8019-8033, 1985). A genomic clone was isolated that contained the leader sequence and a portion of the A chain was isolated and called RIC 1B. This clone contained the promoter region, the 5' untranslated region, the N-terminal leader sequence and coding region that extended to amino acid 191(lle) in the published sequence (Halling, et al., Nucl. Acids Res 13:8019-8033, 1985). The difference between the published sequence and RIC 1B was that the published nucleotide sequence at the region of lie 191 was: (lie 191 underlined) ACG AGA ATT CGG which codes for the amino acids: The Arg lle Arg while in RIC 1B the nucleotide sequence is ACG AGA ATT CGG, which codes for the same amino acids (The Arg lle Arg), the only difference being the last Arg is coded for by CGG in RIC 1B while in the published sequence it is coded for by AGG. This single nucleotide substitution has the effect of introducing a Eco RI site at 11e 191. The clone RIC 1B therefore was missing the amino acids present after 11e 191 since the clone was isolated as a single Eco RI fragment. This truncated version of ricin was used for construction of ricin A chain N-terminal deletions as follows: The clone RIC 1B was digested with Hinc II and Eco RI. The fragment was cloned into Eco RI-Sma I cut pGEM 4Z. The resulting clone, pPAL RlB was digested with Pst I and Bam HI. This cut clone was digested with Exo III nuclease, treated with S1 nuclease and Klenow fragment and then relegated. Subclones were obtained that had various portions of the 5' region deleted, and some of these deletions were sequenced. One deletion, named pPAL-Ridefin had the majority of the N-terminal leader DNA sequence removed and had Hind III and Sph I sites 5' to this region such that the DNA sequence was as follows: AAGCTT GCATGC GCA ACA TGG.... wherein the first six nucleotides code for a Hind III site found in pGEM 4Z, the next six nucleotides codes for the Sph I site in pGEM 4Z and the following three triplets code for amino acids -20, -19, -18 .... (Ala The Trp...) in the published sequence (Halling, et al., Nucl. Acids Res 13:8019-8033, 1985). This subclone therefore had a deletion that removed the first 15 amino acids of the A chain leader sequences of ricin. To provide a ATG start codon in a favourable initiation context, a synthetic oligonucleotide was constructed and inserted into the subclone between the Hind III and Sph I sites. This nucleotide had the sequence: 5'-ACGTGGATCCAAGATGACATG-3'. The reconstructed clone (pPAL Rictr) therefore had the DNA sequence (restriction site for a introduced Bam HI site is underlined, the ATG start codon in bold) AACGT GGATCC AAG ATG ACA TGC GCA ACA TGG at the 5' region such that there was a ATG start codon in a favourable initiation context and a Bam HI site for excision of the coding sequence. The clone pPAL Rictr was digested with Eco RI, end filled with Klenow, and dephosphorylated with alkaline phosphatase. To the vector was added the universal translational terminator purchased from Pharmacia-PL biochemicals (Montreal, Canada) to provide a termination codon. The coding region from this clone was isolated by digestion with Bam HI and Pvu II, releasing the coding region and a small portion of pGEM 4Z, and this fragment can be cloned into the Bam HI and Sma I sites of transformation vectors. This DNA fragment codes for a version of a ricin A chain in which a C-terminal portion has been deleted. It should be noted that a number of C-terminal and N-terminal deletions of the ricin A chain have been tested in vitro for toxicity, and these reports have concluded that the N-terminal half of the ricin A chain is sufficient for cytotoxicity in vitro (for example see: Sudan et al., Nucl. Acids Res, 17: 1717-1732, 1989). In order to obtain a complete coding region for the ricin A chain, synthetic version of the rest of the A chain was synthesized using the published sequence. This synthetic portion of the gene extended the DNA sequence to nucleotide 1182 in the published sequence and had a Eco RI sites at both ends that allowed for the joining of this fragment to the Eco RI site at the amino acids numbers 190-192. This reconstructed version of the gene also had a Bam HI site after the stop codon such that the DNA sequence of the gene at the 3' end was as follows: (nucl. 1181 is marked *) CCT CCA* TAA GGATCC GAATTC coding for amino acids: Pro Pro stop. followed by Bam HI and Eco RI, the Eco RI site being used for the insertion of the synthetic portion of the gene, the Bam HI being used for excision of the complete ricin A chain sequence since a synthetic Bam HI site is at the 5' end of the coding region also. The clone was called pPAL Riccom.

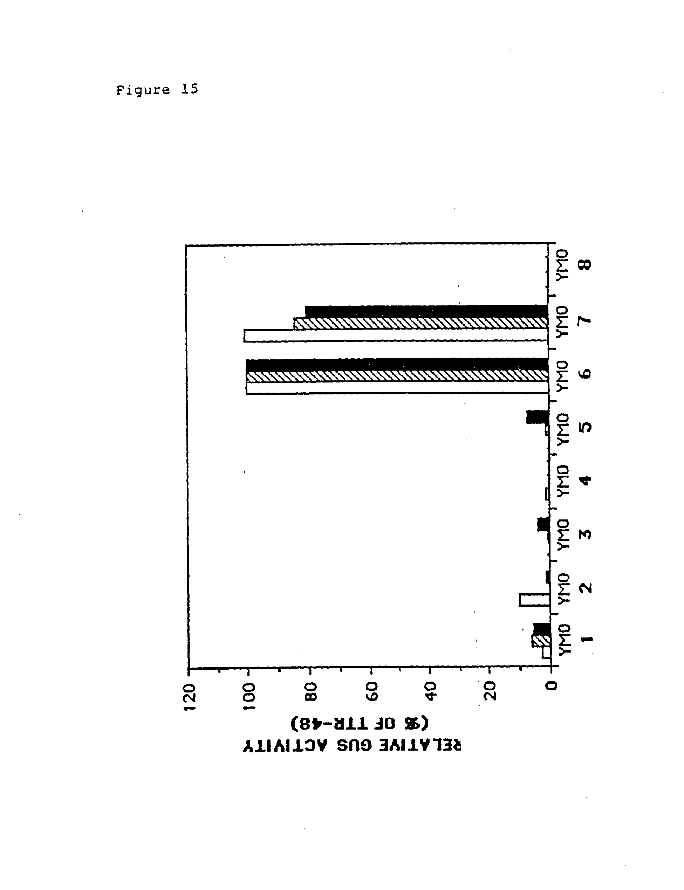

In figure 15 the results of anti-sense RNA inhibition of Beta-glucuronidase gene activity is shown in a histogram of GUS gene activity found in transgenic plants that contained a sense GUS gene and were retransformed with a vector containing an anti-sense GUS gene. GUS activity levels were expressed as a percentage of the GUS activity found in the original GUS+ plant TTR-48. Tissue was obtained from young (Y), medium (M) and old (O) leaves and GUS activity was assayed spectrophotometrically. Lanes 1-5, samples from the retransformed plants TTR-1 to TTR-5 (TTR-48/PAL 1302); lane 6, samples from plant TTR-48; lane 7, samples from TTR-88(TTR-48/pVU 1011) and lane 8, samples from untransformed tobacco.

### BEST MODES FOR CARRYING OUT THE INVENTION

It is known that the number of species of plants that have been successfully genetically transformed still represents a modest percentage of the total number of plant species that are of potential commercial interest. It is true, however that the number of species that have been transformed has increased steadily and there is every reason to expect that transformation systems can be developed for any crop of interest in due course. Routine transformation was initially achieved with species from two plant families: Solanaceae and Brassicaceae. Examples of species of commercial interest from within these families that have been transformed include: tobacco, Nicotiana tabacum L. tomato, Lycopersicon esculentum Mill, potato, Solanum tuberosum L., and petunia, Petunia hybrida (Solanaceae); Canola/Rapeseed, Brassica napus L., cabbage, broccoli, kale etc., Brassica oleracea L., mustards, Brassica juncea L., Brassica nigra L., and Sinapis alba L. (Brassicaceae).

Recently transformation has been reported of commercially important species from other families such as sugar beet, Beta vulgaris, (Chenopodiaceae), cucumber, Curcurbita sp. (Curcurbitaceae), cotton, Gossypium sp., (Malvaceae), sunflower, Helianthus annuus and lettuce Lactuca sativa, (Asteraceae=Compositae), and pea, Pisum sativum, soybean, Glycine max and alfalfa, Medicago sp (Fabaceae=Leguminoseae). Transformation has also been achieved with tree species such as poplar, Populus sp. (Salicaceae) and walnut, Juglans nigra, (Juglandaceae).

Transformation success with monocotyledonous species has not progressed as rapidly, since these species are generally not very susceptible to Agrobacterium mediated transformation. However, progress which has been noteworthy includes asparagus, Asparagus officinalis; gladiolus, Gladiolus sp.,( Lilaceae); corn, Zea mays and rice, Oryza sativa (Poaceae). The recent discovery that transformation with Agrobacterium can be accomplished by infecting germinating seeds without the requirement of regeneration from cell culture (Chee, P.P., et al., 1989, Plant Physiol. 91: 1212-1218) opens new horizons for species that may be difficult to regenerate. Additionally, widespread studies on the use of particle guns to transfer microprojectiles coated in DNA into plant cells of species that are not readily suceptible to other methods holds great promise. It is expected that the present invention may be carried out with any one of the above species and with any other species that is capable of being genetically transformed.

The pollen specific promoters referred to above were isolated from a plant of the species Brassica napus. It is believed that, in respect of each of the plant species enumerated above that it is possible to use said promoters to limit the expression of a given DNA sequence to microspores and to a specific period during pollen development. The published scientific literature has clearly shown that plant genes are universal and the plant tissue-specific promoter fragments retain their function in other species. For example, wheat endosperm promoter fragments function to give appropriate seed specific expression in tobacco (Simpson, J. et al, EMBO Jour. 4:2723-2729, 1985) and the alcohol dehydrogenase promoter (Adh-1) from corn (Zea mays) can be used in conjunction with other promoter fragments to give appropriate expression in tobacco (Ellis, J. G., et al., 1987, EMBO J. 6: 11-16). Additionally the maize transposable element Ac is active in tobacco and other plant species (Taylor, et al., 1989, Plant Mol. Biol. 13: 109-118) providing further evidence of the universality of plant gene structure and function. These examples demonstrate the equivalent tissue specific function of the same promoters in very widely divergent species (monocots to dicots). Our own research and studies of others have shown equivalent promoter function in more closely related species within the same family or between families such as the Solanaceae and Brassicaceae.

It is appreciated, however, that refinements in promoter function may be required for individual plants or species to maximize or modulate the appropriate timing or level of expression to carry out aspects of the invention. Accordingly methods for the modification of promoters to modify or improve function in various plants of different origin are provided herein.

According to the present invention, the following advantages are achieved over other hybridization systems:
(a) Hybrid seed production is not labour intensive and can be achieved on a large scale with commercially acceptable costs;
(b) Male sterility is simply inherited and stable in response to environmental stresses that limit the effectiveness of self-incompatibility and CMS based schemes;
(c) Seed that is produced will be relatively uncontaminated by selfed seed;
(d) The system avoids the use of defective cytoplasmic organelles that may detract from the performance of hybrid seed;
(e) The system will greatly speed the development and increase the number of lines that can be tested as parents in a hybrid cross because it can be imposed on any plant or inbred line capable of being transformed and regenerated into plants without the inclusion of additional genomic DNA. Additionally plant lines can be tested for combining ability before inclusion of the hybridizing system which can modify breeding strategy.

The following terms and expressions, when used throughout the disclosure and claims herein, are to be interpreted in accordance with the definitions set out below unless the context dictates that said terms and expressions are not to be so limited.

The foregoing description of the invention sets forth, in general terms, the steps that can be employed to produce male sterile plants, restorer plants and hybrid seed with a mixture of male fertile and male sterile plants or with restored male fertility in accordance with the invention. It is to be understood that these various steps may be accomplished by a variety of different procedures. In the following description of preferred procedures, alternative ways to accomplish these steps are disclosed. However, it is contemplated that other variations will be apparent to those skilled in the art. Accordingly, the scope of the present invention is intended to be limited only by the scope of the appended claims.

The isolation of genes that are critical to pollen function may be accomplished by a variety of procedures. In accordance with one aspect of the method of the invention, genes that are only expressed at specific stages during pollen development whose regulation is tightly controlled are identified. The genes may be isolated by cloning techniques in accordance with the detailed method set out below. The identification step may also be accomplished by ascertaining, according to standard cloning techniques described below, whether a given gene which is known to be critical to pollen formation and expressed exclusively in pollen in one plant, has the same utility in another plant. It is also known that certain genes are critical to cellular function and expressed in all living cell types. These genes may be isolated using the published DNA sequences for these essential genes, some of which are conserved amongst the plant and animal species, and in conjunction with a promoter that limits gene expression only to pollen tissue to be used for the construction of a recombinant DNA that causes male sterility.

Anti-sense genes may be constructed according to any one of a variety of known methods. The preferred method of construction detailed below is to excise the double stranded coding region of the sense gene or a functional fragment thereof and to insert said double stranded DNA molecule downstream from a promoter, in an inverted orientation relative to its normal presentation for transcription. A terminator structure is preferably added to the end of this anti-sense gene. It is possible within the scope of the present invention to synthetically produce said anti-sense gene, or a functional fragment thereof, according to known methods, and insert said sequence by known methods downstream, from a promoter that will cause timely transcription of same into sufficient quantities of RNA.

The anti-sense gene may be introduced into the plant cell by any one of a variety of known methods preferably by first inserting said gene into a suitable vector and then using said vector to introduce said gene into a plant cell. The transformed cell is selected for by the activity of a marker gene which is capable of conferring resistance to transformed cells to a toxic agent. Transformed plant cells thus selected can be induced to differentiate into plant structures which will eventually yield whole plants. Additionally, it is to be understood that whereas some aspects of this invention may require the transformation of a plant cell with two different genes, that these genes may be physically linked by both being contained on the same vector or physically separate on different vectors. The transformation of the cell can take place with two vectors simultaneously providing that each vector has a unique selectable marker. Alternatively, the transformation with two vectors can take place sequentially allowing an intermediate regeneration step after transformation with the first vector. In addition, it is understood it is possible to perform a sexual cross of individual plants containing different recombinant DNA molecules and select from the progeny of said cross plants containing both recombinant DNA molecules.

Where cost is warranted, and maintenance cannot be readily accomplished as discussed above, transformed plant cells can be grown in culture according to routine methodology to produce a cell line comprising a large number of transformed cells. A large number of transformed cells can be regenerated according to routine methodology from said transformed cell line to increase and maintain the male sterile line. Routine methods for culturing such cells and regenerating transformed plants from such cells is described in standard plant tissue culture hand books. (Plant Tissue and Cell Culture, Green, C. E., Somers, D. A., Hackett W. P., and Biesboer, D. D. Eds, 1987, Alan R. Liss, Inc., New York, Experiments in Plant Tissue Culture, Dodds, J. H. and Roberts, L. W. Eds, 1985, Cambridge University Press, or Cell Structure and Somatic Cell Genetics of Plants, Vasil, I. K., Scowcroft, W.R., and Frey K.J., Eds., 1984, Academic Press, New York, Handbook of Plant Cell Culture, Vol 1-4, Evans, D. A., Sharp, W. R., Ammirato, P. V., and Yamada, Y. Eds.,1984-1986, Macmillan, New York, Bio-technology in Agriculture and Forestry, Vol 1 and 2, Bajaj, Y. P. S. Ed., 1986, Springer-Verlag, Berlin, or Plant Propagation by Tissue Culture - Handbook and Directory of Commercial Laboratories, George, E. F., and Sherrington, P. D., 1984, Eastern Press, Reading).

It may also be possible to produce male sterile plants by fusing cells of the transformed plant cell line with cells of plant species that cannot be transformed by standard methods. A fusion plant cell line is obtained that carries a genetic component from both plant cells. Fused cells can be selected that carry the recombinant gene and in many cases regenerated into plants that carry the male sterile trait.

It is to be understood that any one of a number of promoters can be used to regulate the expression of the recombinant gene provided that the promoter causes transcription of said gene at the proper time and in sufficient quantities of RNA to block proper microspore development. If the aspect of the invention involves anti-sense then complete inhibition of expression of target genes may not be needed, only expression that is reduced to the point that normal pollen formation is blocked. It is possible to use a combination of different genes and promoter structures to interfere with normal pollen development. A promoter or a method that could be used to amplify the expression of an anti-sense gene could be useful to ensure adequate production of anti-sense RNA.

When using a pollen specific promoter to inactivate a sense gene that is critical to pollen development or function, as hereinbefore discussed, it may be difficult to predict, a priori, which pollen specific promoter or modified promoter construct will effectively block the function of that gene. It is preferable to use a pollen specific promoter that displays a similar developmental pattern to that gene. A convenient method to determine when the sense gene targeted for inactivation is expressed is to isolate RNA from developing microspores at different stages and to analyze this RNA for the expression of the target gene by the so-called Northern blot analysis. This procedure will allow for the determination of the exact developmental period in which the sense gene is expressed. In order to determine the period in pollen development in which the pollen specific promoter sought to be used to inactivate said sense gene is expressed, a similar series of analyses can be carried out using as a probe for the expression of said pollen specific promoter a reporter gene joined to said promoter or the naturally occurring sense gene under the control of the pollen specific promoter found in the plant originally used for the isolation of the sense gene. When the pollen specific promoter is isolated from one plant and used in a different plant species the preferred method is the use of a reporter gene joined to said promoter to determine the exact developmental timing that the promoter fragment has in that particular plant species.

Furthermore, it is understood that the activity of the pollen specific promoters, whether intended for the same or different species may be modified in structure to change or alter activity in said plants. Changes that are contemplated include but are not necessarily limited to: addition or deletion of sequences, orientation of upstream or downstream sequences, and the inclusion of introns or parts of the coding sequence of the pollen specific gene. The above modification may serve to increase expression or improve regulation of expression to targeted stages of development.

It is also to be understood that the anti-sense gene does not necessarily have to code for RNA that is complementary to the entire mRNA chain encoded by the sense gene provided that the mRNA encoded by the anti-sense gene is otherwise capable of hybridizing with and blocking translation of the native mRNA species targeted for inactivation. Accordingly the term anti-sense gene when used in disclosure and claims herein encompasses a functional fragment thereof.

To isolate genes that are specifically expressed in developing microspores, a genomic library of plant DNA may be constructed from DNA isolated from fresh young leaves according to standard methodology. (Molecular Cloning, a Laboratory Manual Maniatis, T., Fritsch, E. F., and Sambrooks, J., Cold Spring Harbour Laboratory, Cold Spring Harbour, New York, 1982) and screened with probes derived from several tissues, one of which is made from microspore RNA. The other probes should be made from RNA from different tissues so as to represent genes expressed in tissues of the plant that would not be expected to include genes that are expressed in microspores. Examples include but are not limited to such tissues as leaf, roots, seeds, stigma, stem and other organs. Some genes are expressed in all tissues and some only in a restricted number of tissues, by comparison of many plant tissues it is possible to isolate genes expressed exclusively in microspores.

The microspore RNA may be isolated from microspores that are at the early to late uninucleate stage. Although it is possible to use other stages isolation of the microspores may be technically difficult at earlier developmental stages and older cells may have limited nuclear gene activity and promoters may not be suitable for use as described in the present invention.

Microspores may be conveniently isolated by manual dissection of buds to remove anthers that are subsequently disrupted by gentle grinding in a mortar and pestle in 10 % sucrose. The extract is then filtered through a 44 um nylon mesh and the microspores collected by centrifugation at 3000 x g for one minute. The pelleted microspores are re-suspended in 10 % sucrose, filtered and pelleted as before.Other methods of isolation can also be used.

Tissues other than microspores can be disrupted by a variety of methods and the disrupted tissue used for RNA isolation. It is convenient to disrupt the tissue by using a motor driven homogenizer with 10 mls of a solution of 6M guanidinium HCl, 0.1 M Na acetate, pH 6.0, 0.1 M beta-mercaptoethanol per gram of tissue. The homogenate is cleared by centrifugation at 5,000 x g and the supernatant is layered over a solution of 6M CsCl in Tris-EDTA buffer (TE buffer). Centrifugation at 100,000 x g for 12-20 hrs at 15°C is used to pellet RNA which is subsequently re-suspended in water and re-precipitated in the presence of 0.3 M Na acetate and two volumes of ethanol. RNA is recovered by centrifugation and re-suspended in water. The RNA obtained by such method can be fractionated by oligo-d-T cellulose chromatography to separate the polyadenylated mRNA from the bulk of the non-polyadenylated RNA. The microspore RNA is isolated by using a tight fitting motor driven glass homogenizer to disrupt the microspores. The homogenization of 300 ul of packed microspores is conducted in 1 ml 6M guanidinium-HCl, 0.1 M Na acetate, pH 6.0, 0.1 M beta-mercaptoethanol. The homogenate is centrifuged at 5000 x g and the cleared supernatant is layered over a solution of 6M CsCl in TE buffer. An overnight centrifugation at 100,00 x g is used to pellet the RNA which is subsequently re-suspended in water and re-precipitated in the presence of 0.3 M Na acetate and two volumes of ethanol. Other methods of RNA extraction can be used to obtain the RNA from the tissues described.

Standard methodology using oligo-dT cellulose is used to obtain polyadenylated mRNA from these total RNA preparations.

The mRNA is labelled for the purpose of detection. It is convenient to make radioactive cDNA by using said mRNA and AMV reverse transcriptase in the presence of random hexanucleotide primers and alpha-[³²P]-dCTP. Probes are used for hybridization to nitrocellulose plaque lifts of plates containing the clones of the genomic library. Clones that can be identified as strongly hybridizing only to microspore cDNA and not cDNA from any other tissue examined are chosen. These clones are plaque purified and grown for DNA isolation. Alternative techniques for manipulation of DNA and RNA as well as recombinant DNA, growing and isolating clones can be found in standard laboratory manuals, such as Molecular Cloning, A Laboratory Manual (Maniatis, T., Fritsch, E. F., and Sambrook, J., Cold Spring Harbour Laboratory, New York, 1982).

For applications where the genomic DNA sequence of L4, L10, L16 or L19 from Brassica napus are used to carry out certain aspects of this invention, the preferred method to obtain (isolate) a sense gene that is critical to pollen formation or function is to synthetically produce a homologous DNA sequence according to standard methodology (see Gait, M. J., Ed., {1984} Oligonucleotide synthesis, a practical approach, pp 1-22, IRL Press, Oxford, U.K.), label said sequence for the purpose of detection and use said labelled sequence to screen a Brassica napus genomic library produced according to the methods described.

The identity of the promoter and coding region of a given genomic clone is determined by restriction mapping and hybridization analysis. This may be accomplished by hybridization of cDNA probes made from microspore RNA with restriction fragments of said DNA clones immobilized on nitrocellulose. Restriction endonuclease fragments which contain both the coding region and regions of DNA on either side of the coding region are isolated by sub-cloning in appropriate vectors. Once isolated, it is convenient to use techniques such as S1 mapping and DNA sequencing to obtain exact coding regions and restriction sites within the sub-cloned DNA. This analysis is easily accomplished once the polarity with respect to gene transcription is known.

In order to determine the polarity of transcription of the sense gene individual restriction fragments may be sub-cloned in commercially available vectors such as pGEM3, pGEM4,or pGEM3Z, pGEM4Z (available from Promega Biotech, Madison, Wisconsin, USA). By using these vectors one is able to generate single stranded RNS probes which are complementary to one or the other strands of the DNA duplex in a given sub-clone. These strand specific probes are hybridized to mRNA, in order to establish the polarity of transcription. Among these probes, one can isolate those probes which hybridize with and hence are complementary to the mRNA. Using this information it is possible to clearly determine from what DNA strand of the double-stranded genomic DNA molecule the sense mRNA has been transcribed.

In order to delineate and isolate promoter DNA sequences the pGEM series of vectors can be used for the uni-directional deletion of sequences from the individual sub-clones in hybridization-protection experiments. Detailed descriptions of these experimental procedures can be found in a number of laboratory handbooks and in the manufacturers technical notes supplied with the pGEM series of vectors. These experiments will clearly establish the promoter and coding regions of the pollen specific genomic clones.

The sequence of individual deletions in the pGEM vectors can be determined by dideoxy sequencing of plasmid mini-preps as described in the manufacturer's technical notes. Deletion sub-clones that are deleted to very near the start of transcription or specific restriction fragments that encompass the promoter region or the promoter region and the start of transcription are chosen for-the construction of genes that are expressed only in developing microspores of pollen bearing plants. Usually the promoter fragment is inserted upstream of a terminator such as the nos terminator found in pRAJ-221 (available from Clonetech Laboratories, Palo Alto, CA) and specific restriction fragments which are to be transcribed into anti-sense RNA are inserted between the promoter and terminator sequences. The entire construct is verified by combination of sequencing and restriction digests. The anti-sense gene thus constructed and verified may be inserted in T-DNA based vectors for plant cell transformation. T-DNA vectors that contain a selectable marker are preferred. It is to be understood that the anti-sense gene can be constructed in a variety of ways depending on the choice of vectors, restriction enzymes and individual genes used. For example, it may convenient to insert restriction fragments intended to be transcribed into anti-sense RNA into a T-DNA based vector to which a promoter and terminator structure have been previously added. Alternatively, it is possible to insert a promoter fragment upstream of a coding region and terminator that has been previously added to a T-DNA based vector. In addition, it may be desirable in some crops not to insert the anti-sense gene into a T-DNA based vector but rather into a vector'suitable for direct DNA uptake. Promoters other than microspore specific promoters can be used and joined with specific restriction fragments of genes and terminators provided that these promoters function in microspores.

The introduction of foreign DNA into plants has been accomplished in a variety of ways. The most widely used and generally successful methods are dependent upon the use of an infectious agent, such as the Agrobacterium tumefaciens Ti plasmid, as a vector for delivery of the foreign DNA. Other methods that have been employed involve mechanical means such as direct DNA uptake, liposomes, electroporation (Guerche, P. et al, 1987, Plant Science 52: 111-116) and micro-injection (Neuhaus, G., et al, 1987, Theor. Appl. Genet. 75: 30-36). Recently the possibility of using microprojectiles and a gun or other devise to force small metal particles coated with DNA into cells has received considerable attention (Klein, T.M. et al., 1987, Nature 327: 70-73). To date success with this and other mechanical methods has not been widely reported.

The use of Cauliflower Mosaic Virus (CaMV) (Howell, S. H., et al, 1980, Science 208: 1265) and gemini viruses (Goodman, R. M., 1981, J. Gen. Virol. 54: 9) as vectors has been suggested but by far the greatest reported successes have been with Agrobacteria sp. (Horsch, R. B., et al, 1985, Science 227: 1229-1231). Methods for the use of Agrobacterium based transformation systems have now been described for many different species. Generally strains of bacteria are used that harbour modified versions of the naturally occurring Ti plasmid such that DNA is transferred to the host plant without the subsequent formation of tumors. These methods involve the insertion within the borders of the Ti plasmid the DNA to be inserted into the plant genome linked to a selectable marker used to identify and select transformed cells. Bacteria and plant tissues are cultured together to allow transfer of foreign DNA into plant cells then transformed plants are regenerated on selection media. Any number of different organs and tissues can serve as targets for Agrobacterium mediated transformation as described specifically for members of the Brassicaceae. These include thin cell layers (Charest, P. J., et al, 1988, Theor. Appl. Genet. 75: 438-444), hypocotyls (DeBlock, M., et al, 1989, Plant Physiol. 91: 694-701), leaf discs (Feldman, K.A., and Marks, M. D., 1986, Plant Sci. 47: 63-69), stems (Fry J., et al, 1987, Plant Cell Repts. 6: 321-325), cotyledons (Moloney M.M., et al, 1989, Plant Cell Repts 8: 238-242) and embryoids (Neuhaus, G., et al, 1987, Thoer. Appl. Genet. 75: 30-36). It is to understood, however, that it may be desirable in some crops to choose a different tissue or method of transformation.

Plants which are regenerated from transformed plant cells are tested for the expression of the marker gene which is usually the gene that confers resistance to the selective agent. In the case of the commonly used gene neomycin phosphotransferase (NPT II) which confers resistance to kanamycin and the anti-biotic G-418, gene expression is tested for by in vitro phosphorylation of kanamycin using techniques described in the literature or by testing for the presence of the mRNA coding for the NPT II gene by Northern blot analysis of RNA from the tissue of the transformed plant.

Expression of anti-sense genes is monitored using the same Northern blot techniques. Single stranded RNA probes which are either homologous to sense or anti-sense RNA transcripts are used to detect said transcripts in developing microspores such that the expression of the sense gene and anti-sense gene can be ascertained. It is preferable to use agarose gel electrophoresis to separate transcripts from the sense and anti-sense genes according to size and to do so under denaturing conditions. In the case where microspore specific gene expression of anti-sense genes is sought to be accomplished it is advisable to test for the expression of anti-sense genes in microspores and tissues other than microspores such as leaves, roots, etc., so that tissue specific gene expression of anti-sense genes in microspores can be verified.

The presence of a stably integrated sense or anti-sense gene in the genetic component of the plant cell may also be ascertained by using the so called Southern blot techniques. In this procedure, total cellular or nuclear DNA is isolated from the transformed plant or plant cell and preferably restricted with a restriction enzyme that usually cuts the vector used for transcription at discreet sites, thereby giving rise to discreet fragments. These discreet vector fragments can be detected in the nuclear or total DNA of the transformed plant or plant cells by employing standard gel electrophoresis or hybridization techniques.

Formation of microspores in plants which contain the anti-sense genes is monitored first by visual microscopic examination of the anther structure. As maturation of the flower occurs, anther formation is expected to be delayed or completely inhibited such that no mature pollen grains are formed or released.

As the activity and hence effectiveness of any introduced recombinant DNA molecule is influenced by the chance position of insertion of the recombinant DNA molecule into the plant DNA the degree to which the inserted recombinant DNA molecule may cause or render the plant sensitive to agents that cause reduced male fertility may vary from plant to plant. Accordingly it will be necessary to select a plant which produces no functional pollen grains.

Production of hybrid seed is accomplished by pollination of transformed male sterile plants with pollen derived from selected male fertile plants. Pollination can be by any means, including but not limited to hand, wind or insect pollination, or mechanical contact between the male fertile and male sterile plant. For production of hybrid seeds on a commercial scale in most plant species pollination by wind or by insects are to be preferred. Selection of plants for pollen donation is determined by standard crossing of different plants with subsequent analysis of the progeny and selection of lines with the best combining ability and superior agronomic traits. Restoration of fertility in the hybrids is accomplished by using the methodology detailed in the specific examples.

The invention is illustrated but not limited by the following examples:

### Example 1

In this example, anti-sense RNA was used specifically for the inhibition of gene activity in plants. A tobacco plant expressing the Beta-glucuronidase gene under the control of the CaMV 35S promoter was produced by transformation of a non-transformed control tobacco cultivar, N. tabaccum, cv. Delgold. To accomplished this, tobacco leaves less than 8 inches in length were surface sterilized by exposure to ethanol for 5-6 seconds, then subsequent exposure to 1% sodium hypochlorite for a few minutes, usually 5-10 minutes, or until the cut edge of the petiole turned white, then rinsed several times in sterile distilled water. Leaf segments of approximately 0.5 to 1.0 square centimetres were cocultured for two days with Agrobacterium tumefaciens GV 3101 carrying the Ti plasmid pMP 90 to provide vir functions in trans (described by Koncz, C. and Schell J., 1986, Mol. Gen. Genet. 204:383-396) carrying the binary vector pBI 121.1 on shoot inducing media. This vector is a derivative of Bin 19 which contains the GUS gene driven by the CaMV 35S promoter and terminated by the nos ter and is available from Clonetech Laboratories, Palo Alto, CA., USA. Transformed tobacco cells were selected on a shoot-inducing medium containing 0.8% agar, MS salts, B5 vitamins, 3% sucrose, 1 mg per L of benzyladenine, 0.1 mg per L of alpha naphthalene acetic acid, (NAA) 300 ug/ml kanamycin and 500 ug/ml carbenicillin (essentially as described by Horsch et al, 1985, Science, 227:1229-1231). Regenerated shoots were then transferred to a root-inducing medium consisting of B5 medium with 2% sucrose, 500 ug/ml carbenicillin and 0.5 mg/L each of NAA and indoleacetic acid (IAA). Following selection on kanamycin, a tobacco transformant which displayed relatively high constitutive levels of GUS activity and contained a single unrearranged insertion of the 35S CaMV promoter-GUS-noster construct was selected. This plant (TTR-48, GUS+) was then re-transformed with a binary vector PAL 1302 which contains an anti-sense GUS gene, the construction of which is described in figure 1. In experiments involving the retransformation of the TTR-48 tobacco plant with PAL 1302, the shoot-inducing medium contained 20 ug/ml hygromycin and 300 ug/ml kanamycin to insure the selection of plants containing both the sense and anti-sense GUS constructs. Transformants were grown to maturity and self-pollinated in the greenhouse.

The leaves of tobacco plants resulting from the retransformation of TTR-48 (GUS+) with the anti-sense GUS vector PAL 1302 were assayed for GUS activity. The GUS activity in leaf extracts was assayed spectrophotometrically. Approximately 0.5 g of leaf tissue were ground with a Polytron in 2 ml of GUS extraction buffer (50 mM NaPO4 pH 7.0, 1 mM EDTA, 0.1% Triton X-100, 10 mM B-mercaptoethanol, 1 mM p-nitrophenyl glucuronide, 100 ug/ml bovine serum albumin, 0.02% sodium azide) incubated at 30°C for 6 h and the reaction stopped with the addition of 0.4 ml of 2.5M 2-amino-2-methyl-1, 3-prppanediol. The amount of p-nitrophenol produced was calculated by measuring the absorbance at 415 nm. A stopped reaction containing an identical amount of leaf extract was used as a blank. Relative enzyme activities in the extracts were calculated and expressed as nanomoles of p-nitrophenol produced per mg of protein per minute. Ten plants were screened and they all displayed a large reduction in GUS activity levels relative to those observed in TTR-48. Five plants (TTR-1 to TTR-5) were chosen for a detailed analysis of the effects of anti-sense RNA inhibition of GUS gene activity. The retransformed tobacco plants TTR-1 to TTR-5 (fig. 15, lanes 1-5) all shoed a considerable to complete (lane 4) reduction in GUS activity regardless of the developmental stage of the leaves examined. Comparison of the highest level of GUS activity observed in the original GUS+ plant TTR-48 (fig. 15, lane 6) with the highest level found in any of the plants retransformed with PAL 1302 (lanes 1-5) shows that the reduction in GUS activity was at least 90%. The levels of GUS activity found in the control plant TTR-88 (fig. 15, lane 7) were similar to those of the original GUS+ plant (lane 6) indicating that the retransformation and regeneration process undergone by TTR-48 were not responsible for the decline in GUS activity observed in TTR-1 to TTR-5. Western blot analysis of total protein extracts obtained from young leaves were performed. For extraction of leaf proteins, approximately 100 mg of tissue were ground in a 1.5 ml Eppendorf tube containing 0.7 ml of GUS extraction buffer described above. An equal volume of SDS PAGE 2X sample loading buffer (1.3 M Tris-Cl pH 6.8, 2% B-mercaptoethanol, 50% glycerol, 5 mM EDTA, 0.1% bromophenol blue) was added and the samples incubated at room temperature for 15 min. The extract was centrifuged at 12,000 rpm for 2 min and the supernatant was frozen at -80°C until use. Proteins were resolved in 10% SDS PAGE gels and immediately transferred electrophoretically onto a nitrocellulose filter. The GUS protein was then detected in the gels using the rabbit anti-B-glucuronidase antibody obtained from Clontech Laboratories and an anti-rabbit lgG alkaline phosphatase conjugate kit (Promega Biotech, Madison WI, USA) according to the manufacturers instructions. Equal amounts of protein were separated by sodium dodecyl sulfate polyacrylamide gel electrophoresis (SDS PAGE), transferred to nitrocellulose and cross-related with antibodies specific to the GUS protein. The amount of GUS protein detected on the Western blot correlated well with the GUS activity found in the leaves of all the tobacco plants examined whether these displayed high levels of activity such as TTR-48 or no discernible activity as in TTR-3 (fig. 15, lane 3). The reduction in GUS activity in TTR-1 to TTR-5 can therefore be directly attributed to lower quantities of GUS enzyme within these plants. Southern blot analyses were performed to confirm that the sense GUS gene was still present and intact in TTR-1 to TTR-5 and to verify that the anti-sense GUS construct had correctly integrated in the DNA of these plants. It was found that the arrangement of the original sense GUS gene in TTR-48 was unaffected by the transformation with the anti-sense gene containing vector, and it was also further ascertained that amongst the plants selected for this analysis, between 1-3 copies of the anti-sense gene were inserted into the genome of the plant which contained the sense gene. It was also determined that a single inserted anti-sense gene could lead to a near total reduction in sense gene activity. Northern blot analyses were conducted on total leaf RNA to determine if the reduction in the amounts of GUS enzyme observed in TTR-1 to TTR-5 correlated with their levels of GUS mRNA. Total RNA was prepared from tobacco leaves by grinding 0.5 g of tissue in 2 ml of extraction buffer (6 M guanidine-HCl, 0.1 M NaAc pH 6.0, 1.0% b-mercaptoethanol) for 10-30 sec using a Polytron. The mixture was then centrifuged at 5,000 rpm for 3 min, the supernatant layered on an equal volume of 5.7 M CsCl in 0.1 M NaCl, 10 mM Tris-Cl pH 7.5, ImM EDTA and centrifuged again at 35,000 rpm for 16 h at 15°C. The resulting RNA pellet was resuspended in 0.1 M NaAc pH 6.0, 0.1% SDS and extracted with an equal volume of phenol-chloroform (50:50, v/v). The aqueous phase was adjusted to 0.3 M NaAc and the RNA precipitated with 2 volumes of ethanol. Following centrifugation, the pellet was washed in 70% ethanol and resuspended in sterile distilled water. RNA samples were resolved in the presence of methylmercuric hydroxide in 1.3% agarose gels and transferred onto a nylon membrane. The membranes were probed with (32P) UTP labelled sense or anti-sense GUS RNA transcripts. These transcripts were made from pGEM-GUS, a plasmid obtained by inserting the Bam HI-Sst I fragment of pBI 221.1 (which contains the entire GUS coding sequence) into the Bam HI-Sst I sites of pGEM-42. Probes were made by cutting pGEM-GUS with Eco RI and then using T7 RNA polymerase to provide a transcript which can hybridize to anti-sense GUS RNA or by digestion with Hind III and transcription with SP6 RNA polymerase giving an anti-sense transcript that can hybridize to sense GUS mRNA only. A radiolabelled sense-specific GUS RNA probe demonstrated that the levels of GUS mRNA found in TTR-1 to TTR-5 were considerably lower than those observed in TTR-48, the original GUS+ plant. As predicted, untransformed tobacco did not posses the GUS transcript. The levels of sense m RNA correlate well with the amount of GUS protein and activity observed in these plants. Northern blot analysis using an anti-sense-specific GUS RNA probe demonstrated the presence of anti-sense GUS transcript in the retransformed plants. The reduced amounts of GUS protein and GUS activity observed in TTR-1 to TTR-5 can thus be attributed to the low levels of GUS mRNA found in these plants. Low levels of GUS mRNA are always associated with the presence of the anti-sense GUS RNA. These results clearly indicates that a target sense gene can be successfully inhibited using anti-sense RNA.

### Example 2

We now turn our attention to the use of constitutive promoters producing pollen specific anti-sense RNA. The CaMV 35S promoter form pBl 221 was isolated as a Hind III - Xba I fragment and cloned in PAL 1001 previously cut with Hind III and Xba I. This produced a vector that had the CaMV 35S promoter joined to the nos ter and in between the promoter and terminator were unique sites for : Xba I Bam HI, Sma I, Kpn I and Sst I. This vector was named PAL 1007. PAL 1007 was digested with Bam HI and to this digested vector was added a 2.4 Kb Bam HI fragment containing coding region from clone L16 in the anti-sense orientation. This vector was called PAL 1305 and was used to transform Brassica napus. Transformation was carried out either using the method described in Moloney, M.M., et al. (Plant Cell Reports (1989) 8:238-242) or, transformation can be carried out with surface sterilized stem epidermal layers. For this procedure, seeds of B. napus L. ssp. oleifera cv. Westar were sown in 'Promix' mixed with 2g/L of the slow-release fertilizer 'Nutricoate' in 8" pots. Plants were grown in the greenhouse under a 16 photoperiod (using natural and artificial lighting). For coculture and regeneration experiments stem-sections from the top three stem internodes of approximately 1.5 month old plants were used (i.e. those with elongated floral spikes and several open flowers). Intact stem-sections were surface sterilized for 30 seconds in 70% ethanol and 10 minutes in 1% sodium hypochlorite followed by three rinses in sterile distilled water. For transformation Agrobacterium tumefaciens GV 3101 carrying the Ti plasmid pMP 90 to provide vir functions in trans and the binary vector PAL 1110 was grown on YEP media (which consists of 10 gm per L of Yeast Extract, 10 gm per L of Bacto-pepetone and 5 gm per L of NaCl, pH 7.0 containing 100 ugs per mL kanamycin for selection of bacterial cells that contained the binary vectors). Cells were grown from one to two days at 28C. The cells were collected by centrifugation and were resuspended at an approximate density of 10⁶ - 10⁷ cells per mL in liquid EL which consists of MS micro- and macro- nutrients and B5 vitamins containing 40 mg/L of FeNa-EDTA (obtained from BDH chemicals) and 3% sucrose, 10 mg/L BenzylAdenine, and 0.5 mg/L alpha naphthalene acetic acid (NAA) and 18.8 mM KNO³ plus 20.6 mM NH₄NO³. Medium was solidified with 0.8% agar (Sigma) when the EL media was used for solid media plates.

The cell suspension was poured into the bottom of a sterile petri dish and sterilized stems were dissected directly in the bacterial suspensions. The segments were sectioned longitudinally into half segments and cut into approximately 5 mm sections. The dissected segments were placed on filter paper discs on solid EL media for a 3 day coculture under continuous fluorescent light (60 microeinsteins/m2/sec2) at 25C. After a 2-3 day coculture, explants were transferred to solid EL media containing 500 ug/mL carbenicillin, and 100 ug/mL bekanamycin (Sigma). Shoots formed in 4-8 weeks, sections were transferred to fresh solid EL media with carbinicillin and bekanamycin every 3-4 weeks. Shoots that formed and did not bleach were excised and rooted on PDR media (B5-with 2% sucrose and 0.5 mg/L each of NAA and IAA). In some cases, green non-regenerating callus growing on selective medium was separated from explants and transferred to fresh medium to stimulate regeneration. Transformed plants were placed in a misting chamber, and after two to four weeks transferred to the greenhouse. Plants were grown under a 16 hour photoperiod and allowed to flower. Clonal propagation was used to increase plant lines as well as hand crossing and selection of seedlings from crossed plants on kanamycin containing media. This media consisted of 0.8% agar, one-tenth MS salts an 100ugs per mL bekanamycin with no sucrose in the media. Surface sterilized seeds were used. The seeds were surface sterilized by rinsing in 70% ethanol for a few seconds, soaking in 1% sodium hypochlorite for 15 minutes, followed by rinsing three times in sterile distilled water. Seeds were place on the surface of the agar in sterile dishes and allowed to sprout. Plants which did not carry the kanamycin gene linked to the anti-sense gene bleached and died, while those that carried the antisense gene stayed green and were subsequently transferred to soil and allowed to flower.

### Example 3

In this example we use another pollen specific coding region with the vector PAL 1007. In this case, a 1.3 Kb Hind III fragment from clone L19 was isolated, made blunt ended and cloned into the Sma I site of pGEM 4Z. This subclone was called pPAL 1914 and was then digested with Xba I and Sst I. This fragment was added to Xba I - Sst I cut PAL 1007, giving rise to a vector called PAL 1307. This vector contains the CaMV 35S promoter fused to a coding region from clone L19 in the anti-sense orientation. This vector was used to transform Brassica napus as detailed in example 2.

### Example 4

We now turn our attention to the use of inducible promoters for the production of pollen specific anti-sense RNA. In this example, the 1.2 Kb Hind III - Pst I fragment of the D. melanogaster 70 KD heat shock protein promoter was isolated from the subclone pPW 229 (Holmgren, R. et al., 1979, Cell 18: 1359-1370) and cloned into Hind III - Pst I cut pGEM 4Z. The heat shock promoter was excised as a Hind III - Sma I fragment and cloned into Hind III - Sma I cut PAL 1001. This produced a vector (PAL 1009) that contains a heat shock promoter followed by a portion of the polylinker and nos ter. The Sma I site was used to clone the 1.3 Kb Hind III fragment from clone L19 following making this fragment blunt ended. The clone containing this fragment in the anti-sense orientation relative to the heat shock promoter was called PAL 1403. This vector was used to transform Brassica napus as in example 2. Additionally, since a single Eco RI site exists at the end of the nos ter in this construct, a marker gene was added to this construct, the enzyme Beta-glucuronidase driven by the CaMV 35S promoter using this unique Eco RI site for the insertion of this gene. This vector, which is the same as PAL 1403 except that it now contains a convenient gene for scoring transformation was named PAL 1408 and was used for transformation of Brassica napus as described in example 2.

### Example 5

This example relates to the use of a pollen specific promoter to specifically express pollen specific anti-sense RNA in pollen cells. For this, the vector PAL 1107 was used for the production of anti-sense RNA from the cDNA clone 4F described in figure 4. To construct this anti-sense vector, the Eco RI fragment from the cDNA clone 4F was isolated, made blunt ended with Klenow and cloned into the Sma I site of PAL 1107. A vector that contained the cDNA clone in the anti-sense orientation (as determined by restriction enzyme analysis) was chosen. This vector was named PAL 11074F and used to transform Brassica napus as described in example 2.

### Example 6

For this example, the vector PAL 1107 was used for the production of anti-sense RNA from the 2.4 Kb Bam HI fragment of clone L16 described in example 4. To construct this anti-sense vector, the 2.4 Kb Bam HI fragment from cone L16 was isolated and cloned into the Bam HI site of PAL 1107. A vector that contained this fragment in the anti-sense orientation (as determine by restriction enzyme analysis) was chosen. This vector was named PAL 1107-16CRAS and used to transform Brassica napus as described in example 2.

### Example 7

For this example, the vector PAL 1107 was used for the production of anti-sense RNA from the 1.3 Kb Hind III 99 fragment of clone L19 described in example 5. To construct this anti-sense vector, the 1.3 Kb Hind III fragment from clone L19 was isolated and made blunt ended and cloned into the Sma I site of PAL 1107. A vector that contained this fragment in the anti-sense orientation (as determine by restriction enzyme analysis) was chosen. This vector was named PAL 1107-19CRAS and used to transform Brassica napus as described in example 2.

### Example 8

For this, the vector PAL 1107 was used for the production of anti-sense RNA from the cDNA clone related to clone L10 described in figure 5. To construct this anti-sense vector, the Eco RI fragment from the cDNA clone was isolated, made blunt ended with Klenow and cloned into the Sma I site of PAL 1107. A vector that contained the cDNA clone in the anti-sense orientation (as determined by restriction enzyme analysis) was chosen. This vector was named PAL 110710G and used to transform Brassica napus as described in example 2.

### Example 9

For this, the vector PAL 1107 was used for the production of anti-sense RNA from the cDNA clone related to clone L19 described in figure 6. To construct this anti-sense vector, the Eco RI fragment from the cDNA clone was isolated, made blunt ended with Klenow and cloned into the Sma I site of PAL 1107. A vector that contained the cDNA clone in the anti-sense orientation (as determine by restriction enzyme analysis) was chosen. This vector was named PAL 110719 and used to transform Brassica napus as described in example 2.

### Example 10

In this example, the pollen specific promoter in the vector PAL 1421 was used for the production of anti-sense RNA using the cDNA clone homologous to the gene contained with L10. This was accomplished by excising the cDNA clone from the cloning vector with Eco RI and making this fragment blunt ended with Klenow. This blunt ended cDNA fragment was cloned into the Sma I site of PAL 1421. Clones were recovered that contained the cDNA insert in both orientations, and one was chosen that contained the insert in the anti-sense orientation relative to the promoter of PAL 1421, resulting in the formation of a binary transformation vector PAL 1492. PAL 1492 was used to transform Brassica napus as described in example 2.

### Example 11

In this example, the pollen specific promoter in the vector PAL 1121 was used for the production of anti-sense RNA using the cDNA clone homologous to the gene contained within L10. This was accomplished by excising the cDNA clone from the cloning vector with Eco RI and making this fragment blunt ended with Klenow. This blunt ended cDNA fragment was cloned into the Sma I site of PAL 1121. Clones were recovered that contained the cDNA insert in both orientations, and one was chosen that contained the insert in the anti-sense orientation relative to the promoter of PAL 1121, resulting in the formation of a binary transformation vector PAL 1110. PAL 1110 was used to transform Brassica napus as described in example 2.

### Example 12

In this example, the pollen specific promoter from clone 19 was used for the expression of pollen specific anti-sense RNA. The transformation vector PAL 1920 was digested with Sma I. To the Sma I site was added the DNA fragment corresponding to the cDNA clone homologous to clone L10 by digesting the vector containing this clone with Eco RI and making the fragment blunt ended. This blunt ended cDNA fragment was cloned into the Sma I site of PAL 1920. Clones were recovered that contained the cDNA insert in both orientations, and one was chosen that contained the insert in the anti-sense orientation relative to the promoter of pPAL 1920, resulting in the formation of a binary transformation vector PAL 1921. PAL 1921 was used to transform Brassica napus as described in example 2.

### Example 13

In this example, anti-sense RNA is made specifically to the intron region of clone L19 and a specific restorer gene is made which lacks the intron from clone L19. The construction of these two vectors is outlined in figure 12. PAL 1954 was used to transform Brassica napus as in example 2 to make a male sterile line. To create a specific restorer plant line, the vector PAL 1955 and was used to transform Brassica napus as in example 2.

### Example 14

In this example, we use the highly active promoter fragment of clone HP 101 to synthesize anti-sense RNA to the intron region of clone 19. For this example, the subclone containing the intron region of clone 19 that is detailed in figure 12, pPAL 1914 was digested with Hind III and Bam HI. The promoter fragment from clone pPAL HP101 was added to this construct as a Hind III - Bam HI fragment giving rise to clones containing the promoter fragment in the anti-sense orientation relative to the intron. This clone contained the promoter in the orientation such that transcription of the promoter would cause the production of an RNA a portion of which would contain anti-sense RNA homologous to the intron region of clone L19. This clone was called pPAL 19HP. The clone pPAL 19HP was digested with Hind III and Sst I, and cloned into the vector PAL 1001 using the Hind III and Sst I sites of PAL 1001, creating PAL HP19. PAL HP19 was used to transform Brassica napus as in example 2. It should be noted that the vector PAL 1955 can be used for fertility restoration in plants that carry PAL HP19.

### Example 15

The pollen specific promoter contained in the subclone pPAL 0420 was used for the construction of an anti-sense RNA gene under the control of a pollen specific promoter as follows: A DNA fragment coding for a polyubiquitin of Arabadopsis was isolated from a plasmid which contains a polyubiquitin gene that has 5 copies of the ubiquitin monomeric protein was obtained from the University of Wisconsin, Madison, WI, USA and is described in Burke et al., Molecular and General Genetics, in press. A Bam HI-Bgl II fragment was isolated that contains 3 of the 5 copies of the polyubiquitin gene and this fragment was inserted into pPAL 0420 by using the single Bam HI site of the polylinker of PAL 0420. This gave rise to a plasmid containing the pollen specific promoter from clone L4 followed by an DNA fragment containing ubiquitin coding sequences in the anti-sense orientation followed by the nos ter polyadenylation signal. This promoter/anti-sense gene construct was excised from pPAL 0420 by digestion with Eco RI. The Eco RI fragment that contains the promoter anti-sense gene was inserted into the Eco RI site of the polylinker of BIN 19, resulting in the formation of a binary transformation vector was named PAL 1479. PAL 1479 was used to transform Brassica napus as described in example 2.

### Example 16

The vector PAL 1479 was used to transform tobacco as described in example 1.

### Example 17

The pollen specific promoter contained in the subclone pPAL 0420 was used for the construction of a pollen specific anti-sense RNA gene as follows: A DNA fragment coding for pine actin was obtained from J. Kenny-Byrne, Petawawa Canada. Two clones were obtained, Pac 1-A and Pac 2, the clone Pac 1-A being described in: Canadian Journal of Forestry Research (1988) 18:1592-1602, and the second clone Pac 2 (the sequence being closely homologous to that in Pac 1-A and the nucleotide sequence of which having been submitted for publication). A Sph I fragment was isolated from Pac 2 that contains the complete coding sequence of pine actin. This fragment also contains a small amount of 5' and 3' non-coding region. This Sph I fragment was cloned into the unique Sph I site of pGEM 4Z. From this subclone, a Xba I fragment was isolated that contains only coding region and this Xba I fragment was cloned into the unique Eco RI site of pGEM 4Z. A clone was chosen that had the orientation such that the 5' end of the gene was place next to the Bam HI site in the polylinker and the 3' end of the gene was next to the Sal I site in the polylinker. This plasmid was called pPAL PAC. The actin coding region was isolated form pPAL PAC by digestion with Bam HI and Sal I. This Bam HI - Sal I fragment was cloned into pPAL 0420 using the Bam HI and Sal I sites contained within the polylinker of pPAL 0420. This gave rise to a plasmid containing the pollen specific promoter from clone L4 followed by an DNA fragment containing the actin coding sequence in the anti-sense orientation followed by the nos ter polyadenylation signal. This promoter/anti-sense gene construct was excised from pPAL 0420 by digestion with Eco RI. The Eco RI fragment that contains the promoter anti-sense gene was inserted into the Eco RI site of the polylinker of BIN 19, resulting in the formation of a binary transformation vector PAL 1498. PAL 1498 was used to transform Brassica napus as described in example 2.

### Example 18

The vector PAL 1498 was used to transform tobacco plants as outlined in example 1.

### Example 19

In this example, we transform tobacco that has been previously transformed to hygromycin resistance with an anti-sense gene that specifically blocks the hygromycin resistance in the pollen cells by virtue of the fact that the anti-sense gene is under the control of a pollen specific promoter in the vector PAL 1106. Transformed tobacco that were resistant to hygromycin were obtained using the vector PAL 1302 which is described in example 1. Selection of tobacco plant ells resistant to hygromycin was via coculture with PAL 1302 and selection of 50 ug per ml hygromycin. Southern blot analysis demonstrated the presence of 5-6 copies of the sense hygromycin phosphotransferase gene in one plant. This plant, referred to as TTR-122, was retransformed with a vector called PAL 1107A. Pal 1107A is the vector PAL 1106 to which has been added the 0.8 Kb Bam HI hygromycin phosphotransferase fragment isolated from PAL 1302 and inserted into PAL 1106 in the anti-sense orientation relative to the pollen specific promoter in PAL 1106. The resulting vector was called PAL 1107A. Plants obtained from this transformation were resistant to both hygromycin and kanamycin in the leaf tissue and were shown by southern blot analysis to contain the anti-sense gene. These plants were allowed to grow in the greenhouse and self fertilize. Clonal propagation of these plants were used as a preliminary increase of single plants, and these clonally propagated plants were used for the production of male sterile plant line. For example, a plant which contained a single copy of the anti-sense gene and was derived from the transformation of TTR-122 was planted in a sand soil mixture and allowed to grow in the greenhouse. This plant is referred to as TTR-203. Measurement of the hygromycin phosphotransferase activity in this plant demonstrated high activity in leaves, petals, stigma and pistal and anther walls, but very low levels in pollen. TTR-122 showed high levels of hygromycin phosphotransferase activity in leaves, petals, stigma and pistal and anther walls and in pollen. This demonstrated that the anti-sense gene was effective in blocking the expression of the sense gene only in the pollen. Northern blot analysis confirmed the presence of the anti-sense transcript specifically in the pollen of TTR-203 and also demonstrated low levels of the sense gene mRNA. When flower buds first appeared, TTR-203 was watered three times weekly with a solution of hygromycin (250 ugs per mL), thoroughly saturating the sand soil mixture. This watering was continued for approximately four weeks, or the period of time in which the major flowering was occurring. Flowers produced during this time on plants containing the anti-sense gene were male sterile. Anther and pollen formation was inhibited, and mature pollen failed to develop. Female fertility was unaffected by this treatment as hand pollination could be used for the pollination of the female portion of the male sterile flowers. Pollen that was from a hygromycin resistant plant was used for the hybrid seed production. Watering of the plants with hygromycin was stopped, and normal watering was resumed. Flowers that formed on plants after the hygromycin watering was stopped were male fertile and set selfed seed.

### Example 20

The pollen specific vector PAL 1107 was used for the production of male sterile plants . The plant TTR-122 described above, was retransformed with a vector called PAL 1107HYGAS. Transformation was conducted as described in example 2.Pal 1107HYGAS is the vector PAL 1107 to which has been added the 0.8 Kb Bam HI hygromycin phosphotransferase fragment isolated from PAL 1302 and inserted into PAL 1107 in the anti-sense orientation relative to the pollen specific promoter in PAL 1107. Plants obtained from this transformation were resistant to both hygromycin and kanamycin in the leaf tissue and were shown by southern blot analysis to contain the anti-sense gene. These plants were allowed to grow in the greenhouse and self fertilize. Clonal propagation of these plants were used as a preliminary increase of single plants, and these clonally propagated plants were used for the production of male sterile plant lines as in example 19.

### Example 21

For the production of male sterile plants, the vector PAL 1419 was used to transform Tobacco as outlined in example 1. The vector PAL 1419 contains the pollen specific promoter from clone L4 controlling the expression of the NPT II gene oriented in the anti-sense orientation relative to the pollen specific promoter. This vector also contains a constitutive version of the NPT II gene in the sense orientation driven by the nos promoter. This vector therefore can confer resistance to kanamycin in all plant cells except pollen cells wherein the expression of the sense gene is inhibited by the expression of the anti-sense gene which is specifically expressed in the pollen. Tobacco plants were obtained following transformation of the vector PAL 1419. These plants were rooted and allowed to set flower. Plants were watered with kanamycin while flowering.

### Example 22

The vector PAL 1419 was used to transform Brassica napus as outlined in example 2.

### Example 23

The vector PAL 1419 was used for the transformation of petunia leaf discs.

### Example 24

In this example, the complete ricin A chain (pPAL Riccom) described in figure 14 was inserted into the vector PAL 1420 as a Bam HI fragment. Vectors were recovered that contained the ricin gene in both the sense and anti-sense orientation. A vector that contained the ricin A chain in the sense orientation was recovered and called PAL 1420RIC. A vector that contained the gene in the anti-sense orientation was called PAL 1420RICAS. PAL 1420RIC and PAL 1420RICAS were used for transformation of Brassica napus as described in example 2 giving rise to plants that carried either the sense or anti-sense copy of the ricin A chain gene under the control of the pollen specific promoter in PAL 1420.

### Example 25

In this example, the truncated version of the ricin A chain (pPAL Rictr) described in figure 14 was inserted into the vector PAL 1420 as a Bam HI fragment. Vectors were recovered that contained the truncated ricin gene in both the sense and anti-sense orientation. A vector that contained the ricin A chain in the sense orientation was recovered and called PAL 1420tRIC. A vector that contained the gene in the anti-sense orientation was called PAL 1420tRICAS. PAL 1420tRIC and PAL 1420tRICAS were used for transformation of Brassica napus as in example 2 giving rise to plants that carried either the sense or anti-sense copy of the truncated ricin A chain gene under the control of the pollen specific promoter in PAL 1420.

### Example 26

PAL 1420RIC and PAL 1420RICAS were used for transformation of tobacco plants as in example 1, giving rise to plants that carried either the sense or anti-sense copy of the ricin A chain gene under the control of the pollen specific promoter in PAL 1420.

### Example 27

The vectors PAL 1420tRIC and PAL 1420tRICAS were used for transformation of tobacco plants as in example 1 giving rise to plants that carried either the sense of anti-sense copy of the truncated ricin a chain gene under the control of the pollen specific promoter in PAL 1420.

### Example 28

For this example, the vector PAL 1423 was used to express the polylysine gene contained in the subclone pPAL pLys. The coding region from pPAL pLys was isolated by Bam HI digestion and cloned in the sense orientation into Bam HI cut PAL 1423, giving rise to PAL 1487. PAL 1487 was used to transform tobacco as in example 1.

### Example 29

For this example, the vector PAL 1920 was used to express the polylysine gene contained in the subclone pPAL pLys. The coding region from pPAL pLys was isolated by Bam HI digestion and cloned in the sense orientation into Bam HI cut PAL 1920, giving rise to PAL 1987. PAL 1987 was used to transform tobacco as in example 1.

### Example 30

In this example we use a pollen specific promoter to synthesize a protein molecule that is destructive to cellular function and development, namely the protease trypsin. The cDNA sequence coding for trypsin has been described by Stevenson et al., Nucl Acids Res, 1986, 14:8307-8330. The cDNA clone pMPt9 was obtained and used for the production of a modified trypsin molecule in which the N-terminal amino acid residues were removed to give a protein that consisted of solely the active protease form of trypsin and differed from the mature form in that there was a methionine residue at the N-terminal position of the mature protein, replacing the Isoleuecine found at this position in the active mature protein. This was accomplished by digesting the plasmid pMPt9 with Fok I and Pst I, and recovering a fragment that encompasses nucleotides 81 to 835, the nucleotides after 835 being the G:C tail used for cloning of the cDNA, and treating this fragment with Klenow, and cloning into blunt ended Sma I cut M13mp19RF, and isolating a single stranded phage clone that was used for site specific metagenesis to change the isoleucine codon at nucleotides 84-86 in the published sequence from ATT to ATG, introducing a initiation codon where the isoleucine codon was. The mutated gene recovered was excised with Sal I and Sst I, and inserted into PAL 1421 and named PAL 1456. The vector PAL 1456 was used to transform tobacco as in example 1. It should be noted that a restorer gene can be made if a trypsin inhibitor is inserted in an analogous fashion using a clone L4 derived promoter and transformed into a male parent line. The expression of the trypsin inhibitor in the hybrid will specifically block the activity of the trypsin enzyme. A number of cDNA and genomic DNA sequences can be found for soybean and other trypsin inhibitors, for example see: Jofuku, K.D. and Goldberg, R.B., The Plant Cell 1989, 1:1079-1093.

### Example 31

In this example, we use a pollen specific promoter to synthesize the enzyme IamH specifically in pollen cells. The enzyme has activity that can cause the production of NAA from NAM, the substance NAA functioning as a plant hormone that is substantially toxic to developing pollen grains, while the precursor MAN being relatively non-toxic. For this example, the IamH gene was inserted into the vector PAL 1423. The IamH gene was isolated from pPCV311 as described in figure 12 and cloned as a Bam HI-Sst I fragment in the Bam HI-Sst I sites of PAL 1423, creating PAL 1424. This vector has the IamH gene (T-DNA gene 2) under the control of a pollen specific promoter from clone L4. PAL 1424 was used to transform Tobacco as outlined in example 1.

### Example 32

The vector PAL 1424 was used to transform Brassica napus as outlined in example 2.

### Example 33

In this example, the vector PAL 1107 was used for the production of tissue-specific GUS (beta-glucuronidase) enzyme. The gene for this enzyme is available from Clonetech Laboratories, Palo Alto, CA., USA. The gene was inserted into PAL 1107 as a Bam HI-SstI fragment and was used to transform tobacco as in example 1. Plants produced had detectable GUS activity only in developing pollen cells, and not any other tissue tested. It should be noted that application of non-toxic analog of glucuronic acid to which has been conjugated a toxic molecule such as glyphosate could be applied to these plants and cleavage of the toxic moiety from the glucuronic acid would occur only in pollen cells. This provides an example of an enzyme that could be used for the production, in a tissue-specific fashion, of a toxic substance from a non-toxic analog.

**Thus, the present application describes:**
(A) A method of producing hybrid seed from plants selected Irom those species of pollen producing plants which are capable of being genetically transformed comprising the steps of:
   (a) producing a genetically transformed plant by:
      (i) inserting into the genome of a plant cell of said pollen producing plant which is capable of being regenerated into a differentiated whole plant, one or more recombinant DNA sequences comprising antisense DNA which block the production of functional pollen grains or render developing pollen grains susceptible to a chemical agent or physiological stress which blocks the production of functional pollen grains;
      (ii) obtaining a transformed plant cell of said plant; and
      (iii) regenerating from said plant cell a plant which is genetically transformed with said DNA sequences described in (a)(i) above and which is male sterile or carries the male sterile trait;
   (b) increasing the number of genetically transformed plants by:
      (i) fertilizing the genetically transformed plant described in step (a) with pollen produced by a suitable male fertile plant, in a manner to effect a seed increase of genetically transformed plants; or
      (ii) clonal propogation of said genetically transformed plant described in step (a) using tissue explants thereof, or other in vitro propagation techniques; or
      (iii) when applicable, selfing the genetically transformed plant carrying the male sterile trait described in (a), and selecting a plant homozygous for the male sterile trait and increasing said plant by selfing in isolation; or
      (iv) when applicable, conducting anther or isolated microspore culture of the genetically transformed plant carrying the male sterile trait described in (a) and selecting a plant homozygous for the male sterile trait and increasing said plant by selfing in isolation.
   (c) effecting a hybrid cross by pollinating said genetically transformed plants with pollen from suitable male fertile plant donors.
(B) The method of (A) wherein step (a) is accomplished by:
   (a)
      (i) identifying and isolating, preferably from said pollen producing plant, a sense gene or coding sequence that is critical to pollen formation or function in said plant;
      (ii) inserting into the genome of a plant cell of said plant that is capable of regeneration into a differentiated whole plant, a gene which confers on said plant resistance to a chemical agent or physiological stress and, linked to said gene, a gene comprising:
         (A') a DNA sequence that codes for RNA that is complimentary, in whole or in part, to the RNA sequence encoded by said sense gene or coding sequence;
         (B') a promotor which functions in said plant cell to cause transcription of said DNA sequence into RNA at about the time of transcription of the RNA encoded by said sense gene or coding sequence; and preferably;
         (C') a terminator sequence which defines a termination signal during transcription of said of said DNA sequence;
      (iii) obtaining a transformed plant cell of the said plant; and
      (iv) regenerating from said transformed plant cell a plant which is genetically transformed with the genes described in step (a) ii) above and which is male sterile; and
   and wherein step (b) is accomplished by:
   (b)
      i)crossing the genetically transformed plant described in step (a) iv) above with suitable male fertile plant;
      ii) using the same chemical agent or physiological stress to eliminate plants which do not contain the genes described in step (a) ii) above among plants grown from seed produced by such cross; and
      iii) repeating such a cross over several generations with the plants obtained as in step (b) ii) above in the presence of said chemical agent or physiological stress to
         increase the numbers of male sterile plants;
         and wherein step (c) is accomplished by:
   (c) effecting a hybrid cross by pollinating said male sterile plants with pollen from suitable male fertile donors.
(C) The method of (A), wherein step (a) is accomplished by:
   (a)
      (i) identifying and isolating, preferably from said pollen producing plant, a sense gene or coding sequence that is critical to pollen formation or function in said plant;
      (ii) inserting into the genome of a plant cell of said plant that is capable of regeneration into a differentiated whole plant, a recombinant double stranded DNA molecule comprising:
         (A') a DNA sequence that codes for RNA that is complimentary to the RNA sequence encoded by said sense gene or coding sequence;
         (B¹) an inducible promoter which can function in said plant cell to cause transcription of said DNA sequence into RNA during the time of transcription of said sense gene or coding sequence; and preferably
         (C') a terminator sequence which defines a termination signal during transcription of said DNA sequence
      (iii) obtaining a transformed plant cell of said plant; and
      (iv) regenerating from said transformed plant cell a plant which is genetically transformed with said double stranded DNA molecule described in (ii) above and can be rendered male sterile by said inducer;
   and wherein step b) is accomplished by:
   (b) increasing the number of genetically transformed plants by growing the genetically transformed plant described in step (a)(iv) above in the absence of the relevant inducer to produce a male-fertile plant, permitting self-fertilization and growing seed of such a plant, over a number of generations, in the absence of the inducer to increase the number of genetically transformed plants;
   and wherein step c) is accomplished by:
   (c) effecting a hybrid cross by growing said genetically transformed plants alongside plants of a suitable line of male fertile donors in the presence of the relevant inducer during pollen formation in order to produce male sterile plants and permit cross-pollination of said male sterile plants.
(D) The method of (A), wherein step a) is accomplished by:
   (a)
      (i) inserting concomitantly or independently into the genome of a plant cell of said pollen producing plant which is capable of being regenerated into a differentiated whole plant, a sense gene which confers on said plant resistance to a chemical agent or a naturally occurring or artificially induced physiological stress and a recombinant double stranded DNA molecule
         (A') a DNA sequence that codes for RNA that is complimentary to the RNA sequence encoded by said sense gene;
         (B') a pollen specific promoter which functions in said plant cell to cause transcription of said DNA sequence into RNA; and preferably
         (C') a terminator sequence whch defines a termination signal during transcription of said DNA sequence;
      (ii) obtaining a plant cell of a plant which has been transformed with the genes described in step (i) above;
      (iii) regenerating from said transformed plant cell a plant which is genetically transformed with the genes described in step (i) above and can be rendered male sterile by said chemical agent or stress;
      and wherein step b) is accomplished by:
   (b)
      i) growing the genetically transformed plant described in step (a)(iii) above in isolation from the same stress or chemical agent to produce a self-fertile plant;
      ii) permitting self-fertilization; and
      iii) growing seed of such self-fertile plant, over a number of generations in isolation from the same stress or chemical agent to increase the number of genetically transformed plants;
      and wherein step c) is accomplished by:
   (c) effecting a hybrid crossing growing said genetically transformed plants alongside plants of a suitable line of male fertile donors in the presence of the same stress or chemical agent during pollen formation to produce male sterile plants and to permit pollination of said male sterile plants.
(E) A method of producing a male sterile plant from a plant selected from those species of pollen producing plants which are capable of being genetically transformed, which method comprises the steps of:
   (a) identifying and isolating, preferably from said plant, a sense gene or coding sequence, that is critical to pollen formation or function in said plant;
   (b) inserting into the genome of a plant cell of said plant that is capable of regeneration into a differentiated whole plant, a recombinant double stranded DNA molecule comprising:
      (i) a DNA sequence that codes for RNA that is complimentary to the RNA sequence encoded by said sense gene or coding sequence ;
      (ii) a promotor which functions in said plant cell to cause transcription of said DNA sequence into RNA at about the time of transcription of said sense gene or coding sequence; and preferably
      (iii) a terminator sequence which defines a termination signal during transcription of said DNA sequence.
   (c) obtaining a transformed plant cell in said plant; and
   (d) regenerating from said transformed plant cell a genetically transformed plant which is male sterile.
(F) The method of (E), wherein said sense gene is expressed only in pollen.
(G) The method of (B), (E) or (F), wherein said sense gene comprises the sequence of nucleotides, 600-2430, shown in figure 2a.
(H) The method any one of (B), (D), (E) or (F), wherein said promotor is the promotor of the sense gene.
(I) The method of (E) or (F), wherein said promotor is an inducible promotor that can be induced by the application of a known chemical or a temperature stress.
(K) A method of producing a male sterile plant from a plant selected from those species of pollen bearing plants that are capable of being genetically transformed which method comprises the steps of:
   (a) identifying and isolating preferably from said pollen producing plant, a sense gene or coding sequence that is critical to cellular function or development and expressed in metabolically competent cell types;
   (b) inserting into the genome of a plant cell a recombinant double stranded DNA molecule comprising:
      i) a DNA sequence that codes for RNA that is complimentary to the RNA encoded by said sense gene or coding sequence;
      ii) a pollen specific promoter which functions in said plant cell to cause transcription of said DNA sequence into RNA in a time frame which enables said RNA to block the function of the RNA encoded by said sense gene or coding sequence; and preferably
      iii) a terminator sequence which defines a termination signal during transcription of said DNA sequence;
   (c) obtaining a plant cell that has been genetically transformed with said DNA sequence;
   (d) regenerating from said transformed plant cell a genetically transformed plant which is male sterile.
(L) A method of producing hybrid seed from plants selected from those species of pollen producing plants which are capable of being genetically transformed comprising the steps of:
   (a) producing a male sterile plant by:
      i) identifying and isolating preferably from said pollen producing plant, a sense gene or a coding sequence that is critical to cellular function or development and expressed in metabolically competent cell types.
      ii) inserting into the genome of a plant cell of said plant that is capable of regeneration into a differentiated whole plant, a gene which confers said plant on resistance to a chemical agent or physiological stress and, linked to said gene, a recombinant DNA sequence comprising:
         (A') a DNA sequence that codes for RNA that is complimentary to the RNA sequence encoded by said sense gene or coding sequence;
         (B') a promoter which functions in said plant cell to cause transcription of said DNA sequence into RNA in a time frame which enables said RNA to block the function of the RNA encoded by said sense gene or coding sequence; and preferably;
         (C') a terminator sequence which defines a termination signal during transcription of said DNA sequence;
      iii) obtaining a transformed plant cell of said plant; and
      iv) regenerating from said transformed plant cell a plant which is genetically transformed with the genes described in step (a)(ii) above and which is male sterile; and
   (b) increasing the number of genetically transformed plants by:
      i) crossing the genetically transformed plant described in step (a)(iv) above with a suitable male fertile plant;
      ii) using a chemical agent or physiological stress to eliminate plants which do not contain the genes described in step (a)(ii) above among plants grown from seed produced by such cross; and
      iii) repeating such a cross over several generations with the plants obtained as in step (b)(ii) above in the presence of said chemical agent or physiological stress to increase the numbers of male sterile plants;
   (c) effecting a hybrid cross by pollinating said male sterile plants with pollen from a suitable male fertile donors.
(M) A method of producing a male sterile plant from a plant selected from those species of pollen producing plants which are capable of being genetically transformed, which method comprises the steps of:
   (a) identifying and isolating preferably from said pollen producing producing plant a sense gene or coding sequence that is critical to pollen formation or function in said plant;
   (b) inserting into the genome of a plant cell of said plant that is capable of being regenerated into a differentiated whole plant; a gene which confers on said plant resistance to a selective agent and, linked to said gene, a gene or coding sequence comprising:
      (i) a DNA sequence that codes for RNA that is complimentary to the RNA sequence encoded by said sense gene or coding sequence ;
      (ii) a promotor which functions in said plant cell to cause transcription of said DNA sequence into RNA at about the time of transcription of said sense gene; and preferably
      (iii) a terminator sequence which defines a termination signal during transcription of said DNA sequence.
   (c) obtaining a transformed plant cell in said plant; and
   (d) regenerating from said transformed plant cell a genetically transformed plant which is male sterile.
(N) A method of producing hybrid seed from plants selected from those species of pollen producing plants which are capable of being genetically transformed comprising the steps of:
   (a) producing a male sterile plant by:
      (i) identifying and isolating, preferably from said pollen producing plant a sense gene or coding sequence that is critical to pollen formation or function in said plant;
      (ii) inserting into the genome of a plant cell of said plant which is capable of being regenerated into a differentiated whole plant, a gene which confers on said plant resistance to a chemical agent or physiological stress and, linked to said gene, a gene comprising:
         (A) a DNA sequence that codes for RNA that is complimentary to the RNA sequence encoded by said sense gene or coding sequence;
         (B) a promotor which functions in said plant cell to cause transcription of said DNA sequence into RNA at about the time of transcription of said sense gene or coding sequence; and preferably
         (C) a terminator sequence which defines a termination signal during transcription of said DNA sequence
      (iii) obtaining a transformed plant cell of said plant; and
      (iv) regenerating from said transformed plant cell a plant which is genetically transformed with the genes described in step (a)(ii) above and is male sterile; and
   (b) increasing the number of genetically transformed plants by:
      (i) crossing the genetically transformed plant described in step (a)(iv) above with a suitable male fertile plant;
      (ii) using said chemical agent or physiological stress to eliminate plants which do not contain the genes described in step (a)(ii) above among plants grown from seed produced by such cross; and
      (iii) repeating such a cross over several generations with the plants selected for as in step (b)(ii) above in the presence of said chemical agent or physiological stress to increase the numbers of male sterile plants;
   (c) effecting a hybrid cross by pollinating said male sterile plants with pollen from suitable male fertile donors.
(O) The method of (L) or (N), wherein said suitable male fertile donors used in step (c) are resistant to said chemical agent or physiological stress.
(P) The method of (B), (K),(L), (M),(N), or (O), wherein the selective agent is a herbicide.
(Q) The method of any of one of (B), (K), (L), (M), (N), (O), or (P), wherein the selective agent is glyphosate or chlorsulfuron.
(R) A method of producing hybrid seed with restored male fertility among plants selected from those species of pollen producing plants which are capable of being genetically transformed comprising the steps of:
   (a) producing a male sterile plant by:
      (i) identifying and isolating preferably from said pollen producing plant, a sense gene or coding sequence that is critical to pollen formation or function in said plant;
      (ii) inserting into the genome of a plant cell of said plant that is capable of being regenerated into a differentiated whole plant, a recombinant double stranded DNA molecule comprising:
         (A') a DNA sequence that codes for RNA that is complimentary to the RNA sequence encoded by said sense gene or coding sequence;
         (B') an inducible promotor which functions in said plant cell to cause transcription of said DNA sequence into RNA during the time of trancription of said sense gene; and preferably
         (C') a terminator sequence which defines a termination signal during transcription of said DNA sequence
      (iii) obtaining a transformed plant cell of said plant; and
      (iv) regenerating from said transformed plant cell a plant which is genetically transformed with said double stranded DNA molecule described in (ii) above and is capable of being rendered male sterile by said inducer;
   (b) increasing the number of genetically transformed plants by:
      (i) growing the genetically transformed plant described in step (a)(iv) above in the absence of the relevant inducer to produce a male fertile plant; and
      (ii) permitting self-fertilzation; and
      (iii) growing seed of such a male fertile plant, over a number of generations, in the absence of the relevant inducer to increase the number of genetically transformed plants;
   (c) pollinating said genetically transformed plants in the presence of the relevant inducer during pollen formation with pollen from a suitable line of male fertile donors.

(S) The methods of (M), (N), or (R), wherein said sense gene or coding sequence is expressed only in pollen.
(T) A method of producing a male sterile plant from a plant selected from those species of pollen producing plants which are capable of being genetically transformed, which method comprises the steps of:
   (a) inserting into the genome of a plant cell of said plant that is capable of being regenerated into a differentiated whole plant, a recombinant double stranded DNA molecule comprising:
      (i) a pollen specific promotor;
      (ii) a DNA sequence that codes for a cytotoxic molecule; and
      (iii) a terminator sequence which defines a termination signal during transcription of said DNA sequence.
   (b) obtaining a transformed plant cell; and
   (c) regenerating from said transformed plant cell a genetically transformed plant which is male sterile.
(U) A method to produce hybrid seed with restored male fertility from plants selected from those species of pollen producing plants which are capable of being genetically transformed comprising the steps of:
   (a) inserting into the genome of a plant cell of said pollen producing plant that is capable of being regenerated into a differentiated whole plant, a gene which confers on said plant resistance to a chemical agent or physiological stress and linked to said gene a recombinant double stranded DNA molecule comprising:
      (i) a DNA sequence which codes for a cytotoxic molecule;
      (ii) a pollen specific promoter which functions in said plant cell to cause transcription of said DNA sequence; and
      (iii) a terminator sequence which defines a termination signal during transcription of such DNA sequence.
   (b) obtaining a transformed plant cell;
   (c) regenerating from said plant cell a genetically transformed plant which is male sterile;
   (d) increasing the number genetically transformed plants by:
      i) crossing the geneticaly transformed plant described in step (c) above with a suitable male fertile plant;
      ii) using a chemical agent or physiological stress to eliminate plants which do not contain the genes described in step (a) above among plants grown from seed produced by such cross; and
      iii) repeating such a cross over several generations with the plants obtained as in step (d) ii) above in the presence of said chemical agent or physiological stress to increase the numbers of male sterile plants;
   (e) inserting into a plant cell of suitable male fertile plant selected from the same species a recombinant double stranded DNA molecule comprising:
      (i) a DNA sequence which codes for RNA that is complimentary to the RNA sequence coding for said cytotoxic molecule;
      (ii) a promoter which causes transcription of the DNA sequence defined in step (d)(i) above at about the time of transcription of the DNA sequence defined in step (a)(i);
      (iii) a terminator sequence which defines a termination signal during transcription of the DNA sequence described in step (e)(i) above;
   (f) obtaining a transformed plant cell from step (d);
   (g) regenerating from said transformed plant cell described in step (d) above a genetically transformed male fertile plant.
   (h) producing a restorer line by permitting said genetically transformed male fertile plant to self fertilize and growing seed of such plant, over a number of generations to increase the mumbers of genetically transformed male fertile plants;

   (i) effecting a hybrid cross by pollinating said male sterile plants with pollen from said genetically transformed male fertile plants.
(V) The method of (W) wherein said pollen specific promotor consists of a DNA sequence which comprises the sequence of nucleotides, 1-595, in figure 2a or a functional fragment thereof.
(W) A method of producing a plant which carries the male sterile trait from a plant selected from those species of pollen producing plants which are capable of being genetically transformed, which method comprises the steps of:
   (a) transforming a plant cell of said plant which is capable of being regenerated into a differentiated whole plant, with a sense gene which confers on said plant resistance to achemical agent or naturally occurring or artificially induced physiological stress;
   (b) regenerating from said transformed plant cell a genetically transformed plant which is resistant to the same stress;
   (c) inserting into the genome of a plant cell of said stress resistant plant which is capable of being regenerated into a differentiated whole plant a recombinant double stranded DNA molecule comprising:
      (i) a DNA sequence that codes for RNA that is complimentary to the RNA sequence encoded by the said sense gene;
      (ii) a pollen specific promotor which functions in said plant cell to cause transcription of said DNA sequence into RNA; and preferably
      (iii) a terminator sequence which defines a termination signal during transcription of said DNA sequence.
   (d) obtaining a transformed plant cell; and
   (e) regenerating from said transformed plant cell a plant which has been genetically transformed with the genes described in step (a) and step (c) above and which can be rendered male sterile by said chemical agent or stress.
(X) A method of producing hybrid seed with restored male fertility from plants selected from those species of pollen producing plants which are capable of being genetically transformed comprising the steps of:
   (a) producing a plant which carries a male sterile trait by:
      i) inserting concomitantly or independently into the genome of a plant cell of said pollen producing plant which is capable of being regenerated into a differentiated whole plant, a sense gene which confers on said plant resistance to a chemical agent or a naturally occurring or artificially induced physiological stress and a recombinant double stranded DNA molecule comprising:
         (A') a DNA sequence that codes for RNA that is complimentary to the RNA sequence encoded by said sense gene;
         (B') a pollen specific promoter which functions in said plant cell to cause transcription of said DNA sequence into RNA; and preferably
         (C') a terminator sequence whch defines a termination signal during transcription of said DNA sequence;
      ii) obtaining a plant cell of a plant which has been transformed with the genes described in step (i) above;
      iii) regenerating from said transformed plant cell a plant which is genetically transformed with the genes described in step (i) above and can be rendered male sterile by said chemical agent or stress;
   (b) increasing the number of genetically transformed plants:
      i) growing the genetically transformed plant described in step (a)(iii) above in isolation from the same stress or chemical agent to produce a self-fertile plant;
      ii) permitting self-fertilization; and
      iii) growing seed of such self-fertile plant, over a number of generations in isolation from the same stress or chemical agent to increase the number of genetically transformed plants;
   (c) effecting a hybrid crossing growing said genetically transformed plants alongside plants of a suitable line of male fertile donors in the presence of the same stress or chemical agent during pollen formation to produce male sterile plant and to permit pollination of the male sterile plants.
(Y) The method of (D), (W), or (X), wherein said pollen specific promotor consists of DNA sequence which comprises the sequence of nucleotides, 1-595 in figure 2a, or a functional fragment thereof.
(Z) The method of any one of (D), (W), (X), or (Y) wherein said physiological stress is a herbicide.
(AA) The method of claim (D), (W), or (X), wherein said physiological stress is hygromycin and wherein said sense gene comprises:
   (i) a pollen specific promotor;
   (ii) a DNA sequence which codes for the production of hygromycin phosphotransferase; and
   (iii) a termination sequence which define a termination signal during transcription of said DNA sequence.
(AB) A method of producing hybrid seed from plants selected from those species of pollen producing plants which are capable of being genetically transformed comprising the steps of:
   (a) producing a male sterile plant by:
      (i) identifying and isolating, preferably from said pollen producing plant, a sense gene or coding sequence that is critical to pollen formation or function in said plant;
      (ii) inserting into the genome of a plant cell of said plant that is capable of being regenerated into a differentiated whole plant a recombinant double stranded DNA molecule comprising:
         (A) a DNA sequence that codes for RNA that is complimentary to the RNA sequence encoded by the said sense gene or coding sequence;
         (B) a promotor which functions in said plant cell to cause transcription of said DNA sequence into RNA at about the time of transcription of said sense gene or coding sequence; and preferably
         (C) a terminator sequence which defines a termination signal during transcription of said DNA sequence
      (iii) obtaining a transformed plant cell of said plant and
      (iv) regenerating from said transformed plant cell a genetically transformed plant which is male sterile;
   (b) producing a male sterile line by clonal propagation of said genetically transformed plant using cells derived from tissue explants of said genetically transformed plant;
   (c) growing plants of the male sterile line alongside plants of a suitable line of male fertile donors to permit pollination of the male sterile plant.
(AC) The method of any of one of (A) to (AB), wherein the selected pollen producing plant is a plant of the species Brassica napus or Brassica campestris.
(AD) The method of any one of (A) to (AB), wherein the selected pollen producing plant is of the genus Brassica.
(AE) The method of any one of (A) to (AB), wherein the selected pollen producing plant is of the family Cruciferae.
(AF) The method of any one of (A) to (AB), wherein the selected pollen producing plant is of the family Solanacae
(AG) A pollen specific promotor which consist of a DNA sequence comprising the sequence of nucleotides 1-595 in figure 2a or a functional fragment thereof.
(AH) A plant which has been transformed with a recombinant DNA molecule which comprises:
   (a) a DNA sequence that codes for RNA that is complimentary to mRNA encoded by a gene or coding sequence that is essential to cellular function or development in metabolically competent cell types;
   (b) a pollen specific promoter which functions in said plant to cause transcription of said DNA sequence into RNA in a time frame which enables said RNA to block the function of the RNA encoded by said gene or coding sequence; and preferably
   (c) a terminator sequence which defines a termination signal during transcription ofs said DNA sequence.
(AI) A plant which has been transformed with a double stranded DNA molecule comprising:
   (a) a DNA sequence that codes for RNA that is complimentary to the RNA sequence encoded by a sense gene which is critical to pollen formation or function in said plant;
   (b) a promotor which functions in said plant to cause transcription of said DNA sequence into RNA at about the time of transcription of said sense gene; and
   (c) a terminator sequence which defines a termination signal during transcription of said DNA sequence.
(AK) A plant which has been transformed with a double stranded DNA molecule comprising:
   (a) a DNA sequence that codes for RNA that is complimentary to the RNA sequence encoded by a sense gene which is critical to pollen formation or function in said plant;
   (b) an inducible promotor which functions in said plant to cause transcription of said DNA sequence into RNA during the time of transcription of said sense gene; and
   (c) a terminator sequence which defines a termination signal during transcription of said DNA sequence.
(AL) A plant which has been transformed with a double stranded DNA molecule comprising:
   (a) a DNA sequence which codes for RNA that is complimentary to an RNA sequence encoded by a gene coding for a cytotoxic molecule;
   (b) a pollen specific promotor; and
   (c) a terminator sequence which defines a termination signal during transcription of said DNA sequence.
(AM) A plant which has been transformed with:
   (a) a sense gene which confers on said plant resistance to a chemical agent or a naturally occurring or artificially induced physiological stress; and
   (b) a recombinant double stranded DNA molecule comprising:
      (i) a DNA sequence that codes for RNA that is complimentary to the RNA sequence encoded by said sense gene;
      (ii) a pollen specific promotor which functions in said plant cell to cause transcription of said DNA sequence into RNA; and
      (iii) a terminator sequence which defines a termination signal during transcription of said DNA sequence.
(AN) A plant which has been transformed with:
   (a) a gene which confers on said plant resistance to a chemical agent or physiological stress; and, linked to said gene
   (b) a gene comprising:
      (i) a DNA sequence that codes for RNA that is complimentary to the RNA sequence encoded by a sense gene which is critical to pollen formation or function in said plant;
      (ii) a promotor which functions in said plant cell to cause transcription of said DNA sequence into RNA at about the time of transcription of said sense gene; and
      (iii) a terminator sequence which defines a termination signal during transcription of said DNA sequence.
(AO) A plant as defined in any one of (AI),(AK), or (AN), wherein said DNA sequence codes for RNA that is complimentary to the RNA sequence encoded by a sense gene that is expressed exclusively in pollen tissue of said plant.
(AP) Hybrid seed containing a double stranded DNA molecule comprising:
   (a) a DNA sequence that codes for RNA that is complimentary to the RNA sequence encoded by a sense gene which is critical to pollen formation or function in a plant grown from said seed;
   (b) a promotor which functions in said plant to cause transcription of said DNA sequence into RNA at about the time of transcription of said sense gene; and
   (c) a terminator sequence which defines a termination signal during transcription of said DNA sequence.
(AQ) Hybrid seed containing a double stranded DNA molecule comprising:
   (a) a DNA sequence that codes for RNA that is complimentary to the RNA sequence encoded by a sense gene which is critical to pollen formation or function in a plant grown from said seed;
   (b) an inducible promotor which functions in said plant to cause transcription of said DNA sequence into RNA during the time of transcription of said sense gene; and
   (c) a terminator sequence which defines a termination signal during transcription of said DNA sequence.
(AR) Hybrid seed containing a recombinant DNA molecule comprising:
   (i) A DNA sequence which codes for a cytotoxic molecule;
   (ii) a pollen specific promoter which functions in said plant cell to cause transcription of said DNA sequence;
   (iii) a terminator sequence which defines a termination signal during transcription of said DNA sequence;
   and a recombinant DNA molecule comprising:
   (a) a DNA sequence which codes for RNA that is complimentary to an RNA sequence encoded by said gene coding for cytotoxic molecule;
   (b) a promoter which causes transcription of said DNA sequence into RNA at about the time of transcription of said gene coding for the cytotoxic molecule; and
   (c) a terminator sequence which defines a termination signal during transcription of said DNA sequence.
(AS) Hybrid seed containing DNA comprising:
   (a) a sense gene which confers on a plant grown from said seed resistance to a chemical agent or a naturally occurring or artificially induced physiological stress; and
   (b) a recombinant double stranded DNA molecule comprising:
      (i) a DNA sequence that codes for RNA that is complimentary to the RNA sequence encoded by said sense gene;
      (ii) a pollen specific promotor which functions in said plant cell to cause transcription of said DNA sequence into RNA; and
      (iii) a terminator sequence which defines a termination signal during transcription of said DNA sequence.
(AT) Hybrid seed containing DNA comprising:
   (a) a gene which confers on a plant grown from said seed resistance to a chemical agent or physiological stress; and, linked to said gene
   (b) a gene comprising:
      (i) a DNA sequence that codes for RNA that is complimentary to the RNA sequence encoded by a sense gene which is critical to pollen formation or function in said plant;
      (ii) a promotor which functions in said plant cell to cause transcription of said DNA sequence into RNA at about the time of transcription of said sense gene; and
      (iii) a terminator sequence which defines a termination signal during transcription of said DNA sequence.
(AW) The seed as defined in any one of (AP), (AQ), or (AT), wherein said DNA sequence codes for RNA that is complimentary to the RNA sequence encoded a sense gene that is expressed exclusively in pollen tissue of said plant.
(AV) Hybrid seed containing a recombinant DNA molecule which comprises:
   (a) a DNA sequence that codes for RNA that is complimentary to mRNA encoded by a gene or coding sequence that is essential to cellular function or development in all metabolically competent cell types;
   (b) a pollen specific promoter which functions in a plant grown from said hybrid seed to cause transcription of said DNA sequence into RNA in a time frame which enables said RNA to block the function of the RNA encoded by said gene or coding sequence; and preferably
   (c) a terminator sequence which defines a termination signal during transcription of said DNA sequence.
(AW) A plant which has been transformed with a pollen specific promotor comprising the sequence of nucleotides, 1-595 in figure 2a or a functional fragment thereof.
(AX) A plant which has been transformed with a recombinant double stranded DNA molecule comprising the sequence of nucleotides, 600-2430, in figure 2a or a functional fragment thereof.
(AY) A double stranded DNA molecule consisting of the sequence of nucleotides, 600-2430 in figure 2a ora functional fragment thereof.
(AE) A cell line produced from the genetically transformed cells of any one of (A) to (AF)
(BA) The method of (U), wherein said genetically transformed male fertile plants used in step (i) are resistant to said chemical agent or physiological stress.
(BB) A method of producing a male sterile plant or a plant carrying the male sterile trait by integrating into the genome of said plant a pollen targeted recombinant DNA molecule that selectively blocks the production of functional pollen grains or renders developing pollen grains susceptible to a chemical agent or physiological stress.
(BC) A method as defined in (BB), wherein said pollen targeted recombinant DNA molecule comprises anti-sense DNA to a gene that is critical to pollen function or formation.
(BD) A method as defined in (BB), wherein said pollen targeted recombinant DNA molecule comprises a pollen targeted promoter and anti-sense DNA to a constitutive gene where said anti-sense DNA is regulated by said promoter.
(BE) The method as defined in (BB), wherein said recombinant DNA molecule comprises a pollen targeted promoter and a gene coding for a molecule that is cytotoxic or a molecule that renders a non-toxic molecule cytotoxic.
(BF) A method as defined in (BD) or (BE), wherein said pollen targeted promoter is a pollen specific promoter.
(BG) A method of producing hybrid seed by crossing a genetically transformed female parent plant with a suitable male fertile male parent plant, said genetically transformed female parent plant containing one or more recombinant DNA sequences comprising a sense or anti-sense gene, or both, which when expressed, blocks the production of tissues critical to pollen formation or function or renders said tissues susceptible to a chemical agent or physiological stress that blocks function of said tissues, said suitable male fertile male parent plant, when required to serve as a female restorer plant, containing a recombinant DNA sequence which compensates for the gene function that has been compromised or negates the disruption caused to tissues critical to pollen formation or function in said genetically transformed female parent plant.
(BH) A method as defined in (BG), where said sense gene codes for the production of a cytotoxic molecule or a molecule which renders a substance cytotoxic.
(BI) A method as defined in (BG), where said anti-sense gene codes for the production of a molecule which blocks the function of a pollen targeted gene.
(BK) A method of producing a male sterile plant from a plant selected from those species of pollen producing plants that are capable of being genetically transformed, which method comprises:
   (a) identifying and isolating, preferably from said pollen producing plant, a sense gene that is critical to pollen formation or function in said plant;
   (b) inserting into the genome of a plant cell of said plant that is capable of being regenerated into a differentiated whole plant, a recombinant DNA molecule comprising:
      (i) a DNA sequence that codes for RNA that is complementary to the RNA sequence encoded by said sense gene;
      (ii) a promoter which functions in said plant to cause transcription of said DNA sequence into RNA at or about the time of transcription of said sense gene; and preferably
      (iii) a terminator sequence which defines a termination signal during transcription of said DNA sequence.
   (c) obtaining a transformed plant cell of said plant; and
   (d) regenerating from said transformed plant cell a genetically transformed plant which is male sterile.
(BL) A method of producing hybrid seed from plants selected from those species of pollen producing plants which are capable of being genetically transformed comprising the steps of:
   (a) producing a genetically transformed plant which is male sterile by:
      i) identifying and isolating, preferably from said pollen producing plant, a sense gene that is critical to pollen formation or function in said plant;
      ii) inserting into the genome of a plant cell of said plant that is capable of regeneration into a differentiated whole plant, a gene which confers on said plant resistance to a chemical agent or physiological stress and, linked to said gene, a gene comprising:
         (A') a DNA sequence that codes for RNA that is complementary to the RNA sequence encoded by said sense gene;
         (B') a promoter which functions in said plant to cause transcription of said DNA sequence into RNA at about the time of transcription of the RNA encoded by said sense gene; and preferably
         (C') a terminator sequence which defines a termination signal during transcription of said DNA sequence;
      iii) obtaining a transformed plant cell of the said plant; and
      iv) regenerating from said transformed plant cell a plant which is genetically transformed with the genes described in step (a) ii) above; and
   (b) increasing the number of genetically transformed plants by:
      i) clonal propagation using tissue explants thereof, or other in vitro propagation techniques; or
      ii)
         A') crossing the genetically transformed plant described in step (a) iv) above with suitable male fertile plant;
         B') using the same chemical agent or physiological stress to eliminate plants which do not contain the genes described in step (a) ii) above among plants grown from seed produced by such cross; and
         C') repeating such a cross over several generations with the plants obtained as in step (b) ii) above in the presence of said chemical agent or physiological stress to increase the numbers of male sterile plants;
   (c) effecting a hybrid cross by pollinating said male sterile plants with pollen from suitable male fertile plants.
(BM) A method of producing a male sterile plant from plants selected from those species of pollen producing plants which are capable of being genetically transformed comprising the steps of:
   (a) producing a male sterile plant by:
      (i) identifying and isolating, preferably from said pollen producing plant, a sense gene which comprises a coding sequence that is critical to pollen formation or function and a transcribed but untranslated sequence;
      (ii) inserting into the genome of a plant cell of said plant that is capable of regeneration into a differentiated whole plant, a gene which confers on said plant resistance to a chemical agent or a naturally occurring or artificially induced physiological stress and linked to this a recombinant DNA molecule comprising:
         (A') a DNA sequence that is complementary to said transcribed but untranslated sequence;
         (B') a promoter which functions in said plant to cause transcription of said DNA sequence into RNA at or about the time of the transcription of the sense gene in developing pollen; and preferably
         (C') a terminator sequence which defines a termination signal during transcription of said DNA sequence;
      (iii) obtaining a transformed plant cell of said plant; and
      (iv) regenerating from said plant cell a plant which is genetically transformed with said DNA sequences described in (a)(ii) above and is male sterile.
(BN) A method of producing hybrid seed with restored male fertility from plants selected from those species of pollen producing plants which are capable of being genetically transformed comprising the steps of:
   (a) producing a male sterile plant by:
      (i) identifying and isolating, preferably from said pollen producing plant, a sense gene which comprises a coding sequence that is critical to pollen formation or function and a transcribed but untranslated sequence;
      (ii) inserting into the genome of a plant cell of said plant that is capable of regeneration into a differentiated whole plant, a gene which confers on said plant resistance to a chemical agent or a naturally occurring or artificially induced physiological stress and linked to this a recombinant DNA molecule comprising:
         (A) a DNA sequence that is complementary to said transcribed but untranslated sequence;
         (B) a promoter which functions in said plant to cause transcription of said DNA sequence into RNA at or about the time of the transcription of the sense gene in developing pollen; and preferably
         (C) a terminator sequence which defines a termination signal during transcription of said DNA sequence;
      (iii) obtaining a transformed plant cell of said plant; and
      (iv) regenerating from said plant cell a plant which is genetically transformed with said DNA sequences described in (a)(ii) above and is male sterile, and
   (b) increasing the number of genetically transformed male sterile plants by:
      (i) clonal propagation of said genetically transformed male sterile plant described in step (a) using tissue explants thereof, or other in vitro propagation techniques; or
      (ii) crossing said genetically transformed male sterile plant with a suitable male fertile plant;
      (iii) using said chemical agent or physiological stress to eliminate plants which do not contain the DNA sequence defined in (a)(ii) amongst plants grown from seed produced by such cross; and
      (iv) repeating such cross over several generations with plants obtained in step (b)(iii) above in the presence of said chemical agent or physiological stress to increase the numbers of male sterile plants.
   (c) producing a male fertile restorer plant by:
      (i) inserting into the genome of a plant cell of a suitable male parent plant that is capable of regeneration into a differentiated whole plant a gene that confers resistance to a chemical agent or a naturally occurring or artificially induced physiological stress, linked to a recombinant DNA sequence comprising:
         (A) a recombinant DNA molecule that comprises a modified form of said sense gene that does not contain the regions complementary to said anti-sense gene;
         (B) a promoter that functions in said plant to cause transcription of said modified DNA sequence at a time which restores the function of the sense gene, preferably at or about the time of the action of the anti-sense gene; and preferably
         (C) a terminator sequence which defines a termination signal during transcription of said DNA sequence.
   (d) increasing the number of genetically transformed male fertile restorer plants in the fashion described in (b) above or preferably by selecting a plant homozygous for said restorer trait and increasing said plant by selfing in isolation.
      (e) effecting a hybrid cross by pollinating said male sterile plants with pollen from said male fertile restorer plants.
(BC) The plant cell which has been transformed with the recombinant DNA molecule defined in (K) which is capable of being regenerated into a plant which carries the male sterile trait.
(BP) The plant which has been transformed with a recombinant DNA molecule defined in (k) which carries the male sterile trait.
(BQ) Hybrid seed with restored male fertility produced by the method as defined in (BL).
(BR) A method of producing a plant that carries the male sterile trait from plants selected from those species of pollen producing plants which are capable of being genetically transformed comprising the steps of:
   (a) producing a genetically transformed plant which carries the male sterile trait by:
      i) identifying and isolating, preferably from said pollen producing plant, a sense gene that is critical to pollen formation or function in said plant;
      ii) inserting into the genome of a plant cell of said plant that is capable of regeneration into a differentiated whole plant, a recombinant DNA molecule comprising:
         (A) an anti-sense gene that codes for RNA that is complementary in whole or in part to the RNA sequence encoded by said sense gene;
         (B) an inducible promoter which can function in said plant cell to cause transcription of said DNA sequence into RNA at or about the time the time of transcription of said sense gene or coding sequence; and preferably
         (C) a terminator sequence which defines a termination signal during transcription of said DNA sequence
      iii) obtaining a transformed plant cell of said plant; and
      iv) regenerating from said transformed plant cell a plant which is genetically transformed with said DNA molecule described in (ii) above and can be rendered male sterile by said inducer.
(BS) A method of producing hybrid seed with restored fertility from plants selected from those species of pollen producing plants which are capable of being genetically transformed comprising the steps of:
   (a) producing a genetically transformed plant which carries the male sterile trait by:
      i) identifying and isolating, preferably from said pollen producing plant, a sense gene that is critical to pollen formation or function in said plant;
      ii) inserting into the genome of a plant cell of said plant that is capable of regeneration into a differentiated whole plant, a recombinant DNA molecule comprising:
         (A') an anti-sense gene that codes for RNA that is complementary in whole or in part to the RNA sequence encoded by said sense gene;
         (B') an inducible promoter which can function in said plant cell to cause transcription of said DNA sequence into RNA during the time of transcription of said sense gene or coding sequence; and preferably
         (C') a terminator sequence which defines a termination signal during transcription of said DNA sequence
      iii) obtaining a transformed plant cell of said plant; and
      iv) regenerating from said transformed plant cell a plant which is genetically transformed with said DNA molecule described in (ii) above and can be rendered male sterile by said inducer;
   (b) increasing the number of genetically transformed plants by:
      (i) clonal propagation of said genetically transformed plant described in step (a) using tissue explants thereof, or other in vitro propagation techniques; or
      (ii) selfing the genetically transformed plant carrying the inducible male sterile trait described in (a), selecting a plant homozygous for the inducible male sterile trait and increasing said plant by selfing in isolation over a number of generations in the absence of the inducer to increase the number of genetically transformed plants;
   (c) effecting a hybrid cross between said genetically transformed plants grown in the presence of said inducer and suitable male fertile plants.
(BT) A method of producing a male sterile plant from a plant selected from those species of pollen bearing plants that are capable of being genetically transformed which method comprises the steps of:
   (a) identifying and isolating, preferably from said pollen producing plant, a sense gene or coding sequence that is critical to cellular function or development and expressed in metabolically competent cell types;
   (b) inserting into the genome of a plant cell a recombinant DNA molecule comprising:
      i) a DNA sequence that codes for RNA that is complementary to the RNA encoded by said sense gene or coding sequence;
      ii) a pollen specific promoter which functions in said plant cell to cause transcription of said DNA sequence into RNA in a time frame which enables said RNA to block the function of the RNA encoded by said sense gene or coding sequence; and preferably
      iii) a terminator sequence which defines a termination signal during transcription of said DNA sequence.
   (c) obtaining a plant cell that has been genetically transformed with said DNA sequence;
   (d) regenerating from said transformed plant cell a genetically transformed plant which is male sterile.
(BN) A method of producing hybrid seed from plants selected from those species of pollen producing plants which are capable of being genetically transformed comprising the steps of:
   (a) producing a male sterile plant by:
      i) identifying and isolating, preferably from said pollen producing plant, a sense gene that is critical to cellular function or development and expressed in metabolically competent cell types.
      ii) inserting into the genome of a plant cell of said plant that is capable of regeneration into a differentiated whole plant, a gene which confers on said plant resistance to a chemical agent or physiological stress and linked to said gene, a recombinant DNA sequence comprising:
         (A') a DNA sequence that codes for RNA that is complementary to the RNA sequence encoded by said sense gene;
         (B') a pollen targeted promoter which functions in said plant to cause transcription of said DNA sequence into RNA at or about the time of transcription of the sense gene in tissues critical to pollen function or formation; and preferably
         (C') a terminator sequence which defines a termination signal during transcription of said DNA sequence
      iii) obtaining a transformed plant cell of said plant; and
      iv) regenerating from said transformed plant cell a plant which is genetically transformed with the genes described in step (a)(ii) above and which is male sterile; and
   (b) increasing the number of genetically transformed plants by:
      i) clonal propagation using tissue explants thereof, or other in vitro propagation techniques; or
      ii)
         A) crossing the genetically transformed plant described in step (a) iv) above with suitable male fertile plant;
         B) using the same chemical agent or physiological stress to eliminate plants which do not contain the genes described in step (a) ii) above among plants grown from seed produced by such cross; and
         C) repeating such a cross over several generations with the plants obtained as in step (b) ii) above in the presence of said chemical agent or physiological stress to increase the numbers of male sterile plants;
   (c) effecting a hybrid cross between said genetically transformed plants and suitable male fertile plants.
(BV) A method of producing a plant which carries a male sterile trait from a plant selected from those species of pollen producing plants which are capable of being genetically transformed, which method comprises the steps of:
   (a) transforming a plant cell of said plant which is capable of being regenerated into a differentiated whole plant with a sense gene which confers on said plant resistance to a chemical agent or a naturally occurring or artificially induced physiological or chemical stress;
   (b) regenerating from said transformed plant cell a genetically transformed plant which is resistant to the same stress;
   (c) inserting into the genome of a plant cell of the stress resistant plant which is capable of being regenerated into a differentiated whole plant a recombinant DNA molecule comprising:
      i) a DNA sequence that codes for RNA that is complementary to the RNA sequence encoded by the said sense gene;
      ii) a pollen specific promoter which functions in said plant cell to cause transcription of said DNA sequence into RNA; and preferably
      iii) a terminator sequence which defines a termination signal during transcription of said DNA sequence.
   (d) obtaining a plant cell of said stress resistant plant which has been transformed with the gene described in step (c) above; and
   (e) regenerating from said transformed plant cell a plant which has been genetically transformed with the genes described in step (a) and step (c) above and can be rendered male sterile by said chemical agent or stress.
(BW) A method of producing hybrid seed with restored fertility from plants selected from those species of pollen producing plants which are capable of being genetically transformed comprising the steps of:
   (a) producing a plant which carries a male sterile trait by:
      i) inserting concomitantly or independently into the genome of a plant cell of said pollen producing plant which is capable of being regenerated into a differentiated whole plant, a sense gene which confers on said plant resistance to a chemical agent or a naturally occurring or artificially induced physiological stress and a recombinant DNA molecule comprising:
         (A') a DNA sequence that codes for RNA that is complementary to the RNA sequence encoded by said sense gene;
         (B') a pollen specific promoter which functions in said plant cell to cause transcription of said DNA sequence into RNA; and preferably
         (C') a terminator sequence whch defines a termination signal during transcription of said DNA sequence;
      ii) obtaining a plant cell of a plant which has been transformed with the genes described in step (i) above;
      iii) regenerating from said transformed plant cell a plant which is genetically transformed with the genes described in step (i) above and can be rendered male sterile by said chemical agent or stress;
   (b) increasing the number of genetically transformed plants by:
      i) clonal propagation using tissue explants thereof, or other in vitro propagation techniques;
      ii)
         A) crossing the genetically transformed plant described in step (a) iv) above with suitable male fertile plant;
         B) using the same chemical agent or physiological stress to eliminate plants which do not contain the genes described in step (a) ii) above among plants grown from seed produced by such cross; and
         C) repeating such a cross over several generations with the plants obtained as in step (b) ii) above in the presence of said chemical agent or physiological stress to increase the numbers of male sterile plants; or preferably
      iii)
         A') selfing the genetically transformed plant described in (a) that can be rendered male sterile by said chemical agent or stress, and selecting from that selfing progeny, a plant homozygous for the male sterile trait;
         B') growing the genetically transformed plant described in step (a)(iii) above in isolation from the same stress or chemical agent to produce a self-fertile plant;
         C') permitting self-fertilization; and
         D') growing seed of such self-fertile plant, over a number of generations in isolation from the same stress or chemical agent to increase the number of genetically transformed plants;
   (c) effecting a hybrid cross between said genetically transformed plants grown in the presence of the same stress or chemical agent present during pollen formation and suitable male fertile plants.
(BX) A method to produce hybrid seed with restored male fertility from plants selected from those species of pollen producing plants which are capable of being genetically transformed comprising the steps of:
   (a) inserting into the genome of a plant cell of said pollen producing plant that is capable of being regenerated into a differentiated whole plant, a gene which confers on said plant resistance to a chemical agent or physiological stress and linked to said gene a recombinant DNA molecule comprising:
      (i) a DNA sequence which codes for a cytotoxic molecule;
      (ii) a pollen targeted promoter which functions in said plant cell to cause transcription of said DNA sequence; and
      (iii) a terminator sequence which defines a termination signal during transcription of such DNA sequence.
   (b) obtaining a transformed plant cell;
   (c) regenerating from said plant cell a genetically transformed plant which is male sterile;
   (d) increasing the number genetically transformed plants by:
      i) crossing the genetically transformed plant described in step (c) above with a suitable male fertile plant;
      ii) using a chemical agent or physiological stress to eliminate plants which do not contain the genes described in step (a) above among plants grown from seed produced by such cross; and
      iii) repeating such a cross over several generations with the plants obtained as in step (d) ii) above in the presence of said chemical agent or physiological stress to increase the numbers of male sterile plants;
   (e) inserting into a plant cell of suitable male fertile plant selected from the same species a gene which confers on said plant resistance to a chemical agent or physiological stress and linked to said gene a recombinant DNA molecule comprising:
      (i) a DNA sequence which codes for RNA that is complementary to the RNA sequence coding for said cytotoxic molecule;
      (ii) a promoter which causes transcription of the DNA sequence defined in step (e)(i) above at or about the time of transcription of the DNA sequence defined in step (a)(i);
      (iii) a terminator sequence which defines a termination signal during transcription of the DNA sequence described in step (e)(i) above;
   (f) obtaining a transformed plant cell from step (d);
   (g) regenerating from said transformed plant cell described in step (d) above a genetically transformed male fertile plant.
   (h) producing a restorer line by:
      i) selfing the genetically transformed plant described in (g) and selecting from that selfing progeny, a plant homozygous for the male restorer trait;
      ii) permitting self-fertilization of said plant homozygous for the male restorer trait; and
      iii) growing seed of said plant, over a number of generations to increase the number of genetically transformed plants;

   (i) effecting a hybrid cross by pollinating said male sterile plants with pollen from said genetically transformed male fertile plants.
(BY) A method of producing hybrid seed with restored male fertility from those species of pollen producing plants capable of being transformed and regenerated into a differentiated whole plant which method comprises:
   (a)
      (i) inserting into the genome of a plant cell of said plant that is capable of regeneration into a differentiated whole plant, a gene which confers on said plant resistance to a chemical agent or a naturally occurring or artificially induced physiological stress and linked to this a recombinant DNA molecule comprising:
         (A) a DNA sequence that when transcribed and translated codes for a cytotoxic molecule or a molecule which breaks down a substance into a cytotoxic molecule;
            (B) a pollen targeted promoter which functions in said plant to cause transcription of said DNA sequence into RNA at or about the time of the transcription of the sense gene in developing pollen; and preferably
            (C) a terminator sequence which defines a termination signal during transcription of said DNA sequence:
         (iii) obtaining a transformed plant cell of said plant; and
         (iv) regenerating from said plant cell a plant which is genetically transformed with said DNA sequences described in (a)(ii) above and is male sterile, and
   (b) increasing the number of genetically transformed male sterile plants by:
      (i) clonal propagation of said genetically transformed male sterile plant described in step (a) using tissue explants thereof, or other in vitro propagation techniques; or
      (ii)
         A') crossing the genetically transformed male sterile plant described in (a) with a isogeneic male fertile plant;
         B') using the chemical agent or physiological stress defined in (a)(ii) above to eliminate plants which do not contain the DNA sequence defined in (a)(ii) amongst plants grown from seed produced by such cross; and
         C') repeating such cross over several generations with plants obtained in step (b)(iii) above in the presence of said chemical agent or physiological stress to increase the numbers of male sterile plants.
   (c) producing a male fertile restorer plant by:
      (i) inserting into the genome of a plant cell of a suitable male parent plant that is capable of regeneration into a differentiated whole plant a gene that confers resistance to a chemical agent or a naturally occurring or artificially induced physiological stress, linked to a recombinant DNA sequence comprising:
         (A') a gene that codes for a molecule that negates the disruption caused to tissues critical to pollen formation or function in said genetically transformed female parent plant;
         (B') a promoter that functions in said tissues critical to pollen formation or function to cause transcription of said gene into RNA at or about the time that the sense gene described in (a)(i) is active, preferably a pollen targeted promoter; and preferably
         (C') a terminator sequence which defines a termination signal during transcription of said DNA sequence.
   (d) increasing the number of genetically transformed male fertile restorer plants by:
      (i) selfing the genetically transformed plant carrying the restorer trait described in (c), and selecting a plant homozygous for the restorer trait and increasing said plant by selfing in isolation; or
      (ii) when applicable, conducting anther or isolated microspore culture of the genetically transformed plant carrying the restorer trait described in (C) and selecting a plant homozygous for the restorer trait and increasing said plant by selfing in isolation.
   (e) effecting a hybrid cross by pollinating said male sterile plants described in (a) and increased in (b) in the presence of the chemical agent or physiological stress defined in (a)(ii),(if required), with pollen from male fertile restorer plants as described in (c) and increased in (d).
(BE) The plasmid pPAL 0420.
   - (CA): The plasmid PAL 1106.
   - (CB): The plasmid PAL 1107.
   - (CC): The plasmid PAL 1419.
   - (CD): The plasmid PAL 1420.
   - (CE): The plasmid PAL 1421.
   - (CF): The plasmid PAL 1422.
   - (CG): The plasmid PAL 1423.
   - (CH): The plasmid PAL 1920.
   - (CI): The plasmid pPAL 0101 (ATCC 68210).
   - (CK): The plasmid pPAL HP101.
   - (CL): The plasmid PAL 1954.
   - (CM): The plasmid PAL 1955.
   - (CN): The plasmid PAL 1302.
   - (CO): The plasmid pPAL 303.
   - (CP): The plasmid pPAL 306.
   - (CQ): The plasmid PAL 1121.
   - (CR): The plasmid pPAL 1901.
   - (CS): The pollen specific promoter constructs and nucleotide coding sequences as shown in the Figures and described in the disclosure herein.
   - (CT): The pollen specific coding sequence constructs and nucleotide coding sequences as shown in the Figures and described in the disclosure herein.
   - (CU): The nucleotide coding sequence of the constitutive promoter pPAL HP101 as shown in the Figures and described in the disclosure herein.
   - (CV): The methods, and plant cells, plants and hybrid seeds derived therefrom, as shown in the Figures and substantially described in the disclosure herein.
   - (CW): The nucleotide sequence 1-8585 of clone L4.
   - (CX): The promoter region of gene Bp4A of clone L4 comprising nucleotides 1-307 or a functional fragment thereof.
   - (CY): The first coding region of gene Bp4A of clone L4 comprising nucleotides 237-368 or a functional fragment thereof.
   - (CZ): The second coding region of gene Bp4A of clone L4 comprising nucleotides 1133-1427 or a functional fragment thereof.
   - (DA): The intron region of gene Bp4A of clone L4 comprising nucleotides 3691-1132 or a functional fragment thereof.
   - (DB): The transcribed region of gene Bp4A of clone L4 comprising nucleotides 237-1427 or a functional fragment thereof.
   - (DC): The promoter region of gene Bp4C of clone L4 comprising nucleotides 4565-6268 or a functional fragment thereof.
   - (DD).: The first coding region of gene Bp4C of clone L4 comprising nucleotides 6297-6429 or a functional fragment thereof.
   - (DE): The second coding region of gene Bp4C of clone L4 comprising nucleotides 7195-7490 or a functional fragment thereof.
   - (DF): The intron region of gene Bp4C of clone L4 comprising nucleotides 6430-7194 or a functional fragment thereof.
   - (DG): The transcribed region of gene Bp4C of clone L4 comprising nucleotides 6297-7490 or a functional fragment thereof.
   - (DH): The nucleotide sequence of clone L10 comprising nucleotides -789-2852.
   - (DI): The promoter region of clone L10 comprising nucleotides -789-64 or a functional fragment thereof.
   - (DK): The first coding region of clone L10 comprising nucleotides 1-315 or a functional fragment thereof.
   - (DL): The second coding region of clone L10 comprising nucleotides 475-1135 or a functional fragment thereof.
   - (DM): The third coding region of clone L10 comprising nucleotides 1672-2011 or a functional fragment thereof.
   - (DN): The first intron region of clone L10 comprising nucleotides 316-474 or a functional fragment thereof.
   - (DO): The second intron region of clone L10 comprising nucleotides 1136-1671 or a functional fragment thereof.
   - (DP): The transcribed region of clone L10 comprising nucleotides 1-2011 or a functional fragment thereof.
   - (DQ): The nucleotide sequence of clone L19 comprising nucleotides -2022-2955.
   - (DR): The promoter region of clone L19 comprising nucleotides -2022-135 or a functional fragment thereof.
   - (DS): The first coding region of clone L19 comprising nucleotides 1-1201 or a functional fragment thereof.
   - (DF): The second coding region of clone L19 comprising nucleotides 1339-2072 or a functional fragment thereof.
   - (DU): The intron region of clone L19 comprising nucleotides 1202-1338 or a functional fragment thereof.
   - (DV): The transcribed region of clone L19 comprising nucleotides 1-2072 or a functional fragment thereof.
   - (DW): The promoter constructs, including chimeric promoter constructs included in the vectors described in claims 75-92.
   - (DX): The nucleotide sequence of cDNA clone cBp401 comprising nucleotides 1-384, or a functional fragment thereof.
   - (DY): The nucleotide sequence of cDNA clone cBp405 comprising nucleotides 1-364, or a functional fragment thereof.
   - (DZ) The: nucleotide sequence of cDNA clone cBp408 comprising nucleotides 1-298, or a functional fragment thereof.

## Claims

1. A DNA sequence comprising a promoter sequence selected from sequences of one or more pollen specific genes from Brassica napus and from one or more of the promoter regions of the pollen targeted genes in clones L4 as shown in Fig. 2a or 3a, L10 as shown in Fig. 2b or 3b, L16 as shown in Fig. 2c or 3c or L19 as shown in Fig. 2d or 3d or of portions thereof which function as a pollen specific promoter, or a DNA sequence which hybridizes with the said DNA sequence and has the function of a pollen specific promoter.

2. A DNA sequence comprising a pollen targeted promoter, or such promoter construct, optionally a chimeric promoter construct, as present in:
PAL 0420 as obtainable by the scheme shown in Fig 7c;
PAL 1106 as obtainable by the scheme shown in Fig 7b;
PAL 1107 as obtainable by the scheme shown in Fig 7a;
PAL 1419 as obtainable by the scheme shown in Fig 7c;
PAL 1421 as obtainable by the scheme shown in Fig 7d;
PAL 1422 as obtainable by the scheme shown in Fig 7d;
PAL 1423 as obtainable by the scheme shown in Fig 7d;
PAL 1920 as obtainable by the scheme shown in Fig 9;
PAL 1954 as obtainable by the scheme shown in Fig 12;
PAL 1955 as obtainable by the scheme shown in Fig 12;
PAL 1121 as obtainable by the scheme shown in Fig 8;
or a DNA sequence which hybridizes with the said DNA sequence and has the function of a pollen specific promoter.

3. The nucleotide sequence of clone L4 as shown in Fig. 3a comprising nucleotides 1-8585, or a portion thereof which comprises the promoter region of gene Bp4A of clone L4 comprising nucleotides 1-307 and/or the promoter region of gene Bp4C of clone L4 comprising nucleotides 4565-6268, or a fragment of any of said sequences which functions as pollen specific promoter, or a DNA sequence which hybridizes with the said nucleotide sequence or the fragment thereof and functions as a pollen targeted promoter.

4. The nucleotide sequence of clone L10 as shown in Fig. 3b comprising nucleotides -789-2852, or a portion thereof which comprises the promoter region of clone L10 comprising nucleotides -789-64, or a fragment of said sequence which functions as a pollen specific promoter, or a DNA sequence which hybridizes with the said nucleotide sequence or the fragment thereof and functions as a pollen targeted promoter.

5. The nucleotide sequence of clone L19 as shown in Fig. 3d comprising nucleotides -2022-2955, or a portion thereof which comprises the promoter region of clone L19 comprising nucleotides -2022-135, or a fragment of said sequence which functions as a pollen specific promoter, or a DNA sequence which hybridizes with the said nucleotide sequence or the fragment thereof and functions as a pollen targeted promoter.
